# EUROPEAN PATENT APPLICATION

(11) **EP 0 082 133 A2**
(43) Date of publication of application: **22.06.1983**
(21) Application number: 83100022.9
(22) Date of filing: 18.04.1980
(51) Int. Cl.: C07D 205/08, C07D 487/04, C07D 498/04, C07F 7/10, C07C 149/437, C07C 69/96

(54) **A process for preparing intermediates useful for preparing 2-substituted-6-substituted-1-carbadethiapen-2-em-3-carboxylic acids**

(30) Priority: 19.04.1979 US 31694
(62) Divisional of application: 80102076.9
(71) Applicant: Merck & Co., Inc., Rahway New Jersey 07065-0900 (US)
(72) Inventor: Christensen, Burton G., Scotch Plains New Jersey 07060 (US); Johnston, David B.R., Warren New Jersey 07060 (US); Schmitt, Susan M., Scotch Plains New Jersey 07076 (US)
(74) Representative: Abitz, Walter

(57) **Abstract**

Disclosed is a process for preparing intermediates having the structure 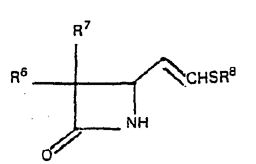 wherein R⁶, R⁷ and R⁸ are, inter alia independently selected from the group consisting of hydrogen, alkyl, alkenyl, aryl and aralkyl. Such compounds are useful for preparing 2-substituted-6-substituted-1 -carbadethiapen-2-em-3-carboxylic acid antibiotics as well as their pharmaceutically acceptable salt, ester and amide derivatives.

## Description

This invention relates to the preparation of intermediates which are useful for the preparation of 2- and 6-substituted-1-carbadethiapen-2-em-3-carboxylic acids (I) and the pharmaceutically acceptable salt, ester and amide derivatives thereof which are useful as antibiotics.

The intermediates have the formula 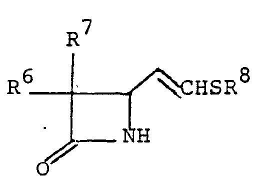 wherein R⁶, R⁷, and R⁸ are independently wherein R⁶ , R⁷ , and are independently selected from the group consisting of hydrogen, substituted and unsubstituted: alkyl, alkenyl, and elkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the aliphatic portion has 1-6 carbon atoms; heteroaryl, heteroaralkyl, heterc- cyclyl and heterocyclylalkyl; wherein the substituent or substituents relative to the above-named radicals are selected from the croup consisting of:
-X° halo (chloro, bromo, fluoro)
-OH hydroxy
-OR¹ alkoxy, aryloxy 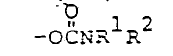 carbamoyloxy 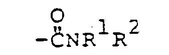 carbamoyl
-NR¹R² amino 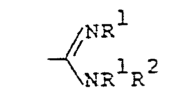 amidino
-SO₂NR¹R² sulfonamido 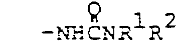 ureido 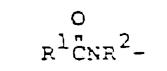 amido
-CO₂H carboxy
-OSO₂R¹ sulphate
-N0₂nitro tri-substituted amino (R¹ group independentlv chosen) 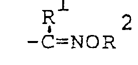 - - - - - - - - - -- - - - oximino
-CO₂R¹ carboxylate 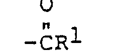 acyl 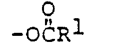 acyloxy
-SH mercanto 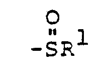 alkyl and aryl sulfinyl 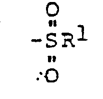 alkyl and aryl sulfonyl
-CN cyano
-^{N}₃ azido
-SR¹ alkyl- and arylthio

wherein, relative to the above listed substituents on R⁶, R⁷, and R⁸, the groups R¹ and R² are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the aliphatic portion has 1-6 carbon atoms; heteroalkyl, heteroaralkyl, heterocyclyl and heterocyclylalkyl and wherein the hetero atom or atoms in the above-named heterocyclic moieties are selected from the group consisting of 1-4 oxygen, nitrogen or sulphur atoms and wherein the alkyl moieties associated with said heterocyclic moieties have 1―6carbon atoms.

Further, relative to R radicals which carry an amino group (―NH₂) or an N-substituted amino group (-NR¹H), and which can be represented conveniently as: -R⁸-NH₂, and -R⁸-NR¹H, respectively, there as: -R⁸ -NH 2' and -R⁸- NR¹H, respectively , there exists the following groups classed under previously 8 defined R⁸ : 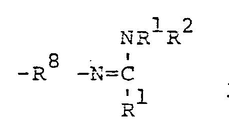 amidino 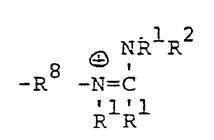 amidino 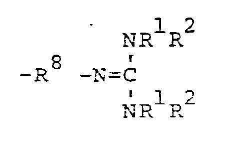 guanidino 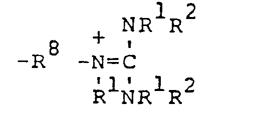 guanidino wherein: R¹ and _{R}² are as defined above.

The intermediates prepared according to the invention can be used for preparing the carboxyl derivatives which are antibiotics and which may be represented by the following generic structure (I): 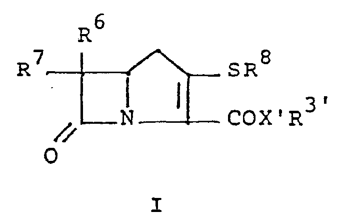 wherein X' is oxygen, sulphur or NR' (R' = H or lowei alkyl having 1-6 carbon atoms); and R is, inter alia, representatively selected from the group consisting of hydrogen, conventional blocking groups such as trialkylsilyl, acyl and the pharmaceutically acceptable salt, ester and amide moieties known in bicyclic β-lactam antibiotic art; the definition of R is given in greater detail below.

There is a continuing need for new antibiotics. For unfortunately, there is no static effectiveness of any given antibiotic because continued wide scale usage selectively gives rise to resistant strains of pathogens. In addition, the known antibiotics suffer from the disadvantage of being effective only against certain types of microorganisms. Accordingly, the search for new antibiotics continues.

Thus, it is an object of the present invention to provide intermediates for a novel class of antibiotics which are useful in animal and human therapy and in inaminate systems. These antibiotics are active against a broad range of pathogens which representatively include both gram positive bacteria such as S. aureus, Strep. pyogenes, and B. subtilis, and gram negative bacteria such as E. coli, Pseudomonas, Proteus morganii, Serratia, and Klebsiella.

The compounds prepared according to the present invention (14) are conveniently converted into antibiotics by the following scheme, which is including a further possibility to prepare such compounds 14:

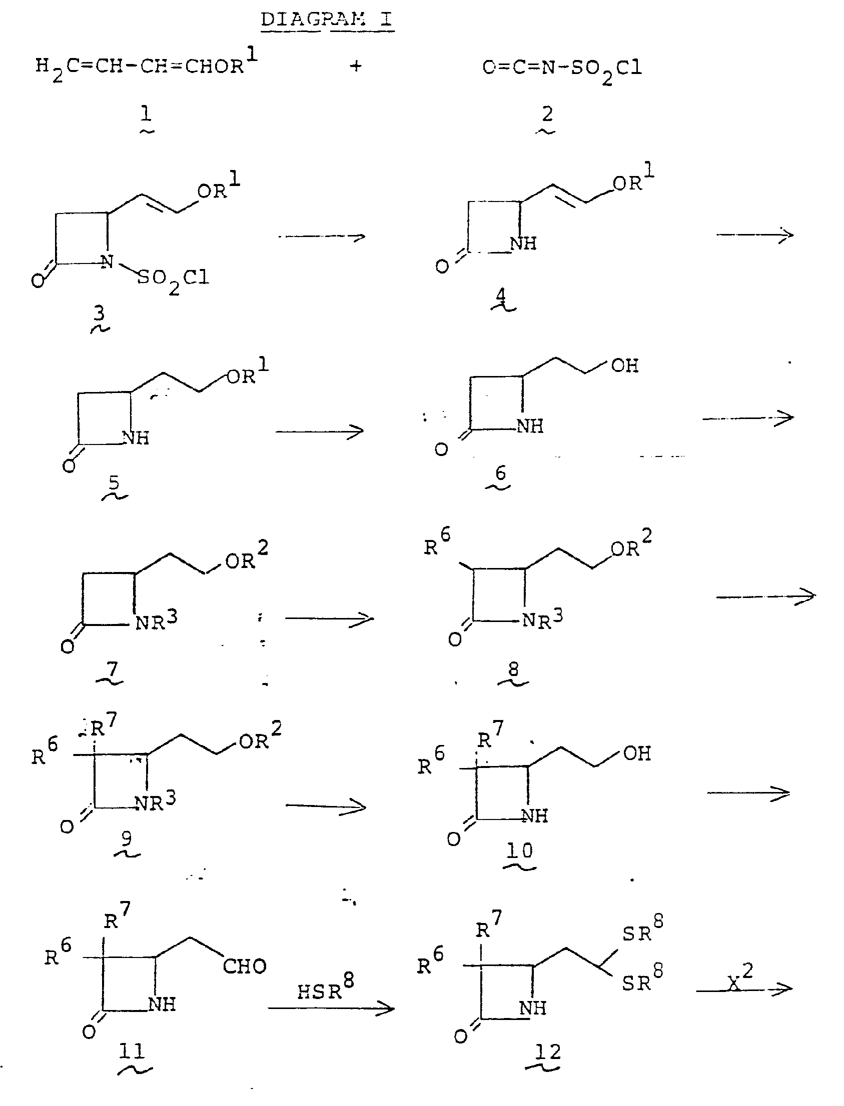

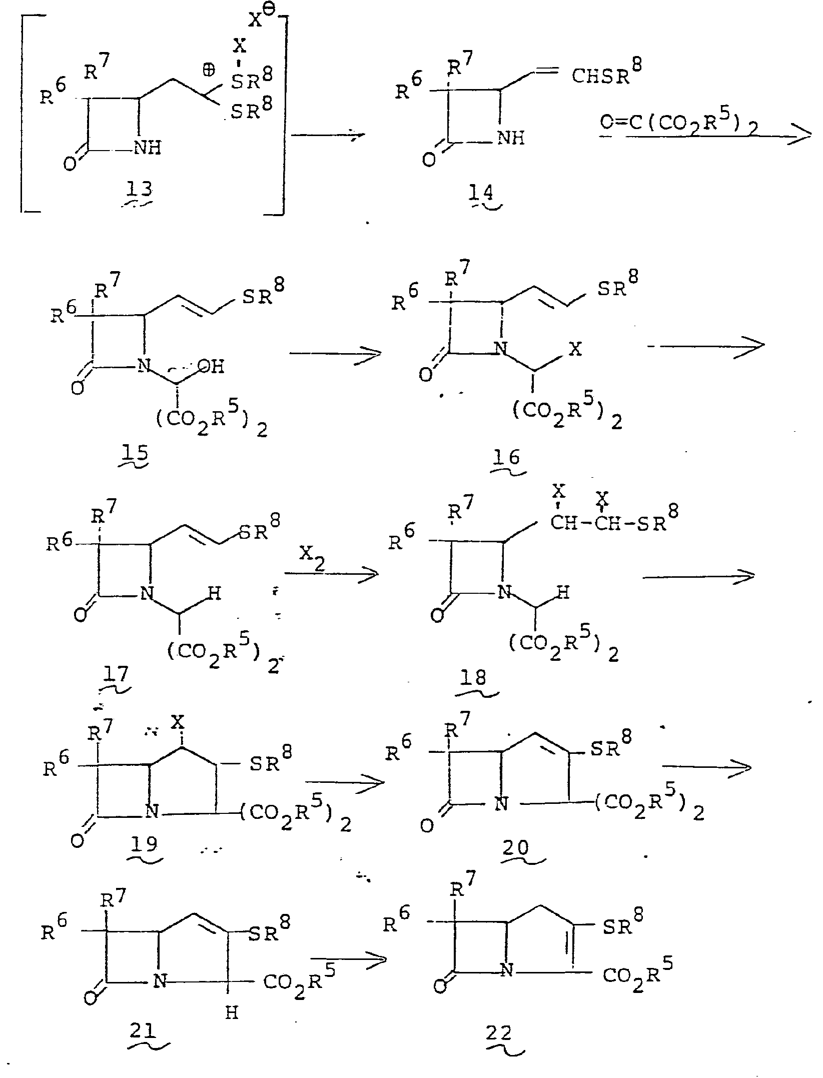

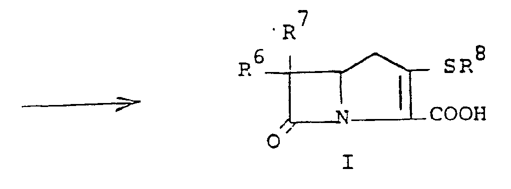

In words relative to the above diagram, the 4-(2-substituted-vinvl)azetidine-2-one, 4, starting material is prepared by reacting an R¹-oxybutadiene, 1, with chlorosulfonylisocyanate 2. The reaction is conducted without solvent or may be run in solvent such as diethyl ether, ethyl acetate, chloroform, methylene 'chloride, or the like, at a temperature of from -78°C to 25°C for from a few minutes to 1 hour to provide 3. The radical R¹ is an easily removable acyl blocking group such as alkanoyl or aralkanoyl which bears no functional group or groups which might interfere with the desired course of reaction (1 + 2↦ Intermediate species 3 is converted to the sulfinamide by reduction which is then hydrolyzed to 4 at pH 6-8. Typically the reaction solution comprising 3 is contacted (5-30 minutes) with an aqueous solution (at 0-25°C) of a reducing agent such as sodium sulfite, thiophenol, or the like, at pH 6-8 to provide

The reaction 4→5 is a reduction, and is preferably achieved by hydrogenation in a solvent such as ethyl acetate, ether, dioxane, tetrahydrofuran (THF), ethanol or the like at 0 to 25°C for from 5 minutes to 2 hours under 1 . to 10 atmospheres of hydrogen in the presence of a hydrogenation catalyst such as a platinum metal or oxide thereof such as 10% Pd/C or the like.

The de-blocking reaction 5→6 is usually desirable when R is acyl to permit the later alkylation, 7→8. The preferred de-blocking procedure is by alcoholysis wherein the solvent is a lower alkanol such as methanol, ethanol or the like in the presence of the corresponding alkali metal alkoxide, such as sodium methoxide. Typically, the reaction is conducted for from 5 minutes to 1 hour at a temperature of from -10° to 25°C.

Blocking groups R³ and R² are established (E→7) to provide a suitably protected species for alkylation (7→8→9) . There is no criticality in the choice of blocking groups, provided only that they do not interfere with the intended alkylation. R³ may be hydrogen, a triorganosilyl group such as trimethylsilyl or the like, or a cyclic ether such as 2-tetrahydropyranyl. R² may also be a cyclic ether such as 2-tetrahydropyranyl; alternatively R³ and R² may joined together to form protected species such as 7a: 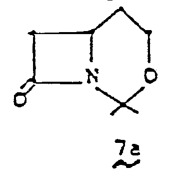 For example, species such as 7a are conveniently prepared by treating 6 with 2,2-dimethoxypropane in the presence of a catalyst such as boron trifluoride etherate, toluene sulphonic acid, or the like in a solvent such as methylene chloride, ether, chloroform, dioxane or the like at a temperature of from -10°C to 35°C for from a few minutes to 1 hour. Species 7 can be mono- or dialkylated at ring position 6. Alkylation of 7 provides 8. Typically, is treated with a strong base such as lithium diisopropyl amide, sodium hydride, phenyl lithium or butyl lithium and the like in a solvent such as tetrahydrofuran (THF), ether, dimethoxyethane and the like at a temperature of from -80°C to 0°C., whereupon the alkylating agent of choice, R⁶X, is added (R⁶ is as described above and X is chloro, iodo or bromo; alternatively the alkylating agent may be R⁶-tosylate, R6-mesylate- or an aldehyde or ketone such as acetaldehyde and the like) to provide monoalkylated species S. When desired, dialkylated species 9 may be obtained from 8 by repeating the alkylating procedure, 7→38.

The de-blocking reaction 9→10 is typically con- ducted by acid hydrolysis such as aqueous acetic acid at a temperature of from 25°C to 75°C for from 5 minutes to 3 hours.

The aldehyde intermediate 11 is prepared by treating 10 with an oxidizing agent such as CrO₃.2 (pyridine) in CH₃CN, 1:1 mixture of dimethylsulfoxide and acetic anhydride, cyclohexylcarbodiimide in DMS0 or the like at a temperature of from 0-25°C for from 5-minutes to 1 hour. The resulting species 11 in a solvent such acetonitrile, methylene chloride, chloroform or the like at a temperature of from -10 to 25°C is treated with an excess of the reagent HSR⁸ in the presence of an acid catalyst such as boron trifluoride etherate, toluene sulphonic acid or the like to provide 12. Typically, the reaction requires from 1 to 60 minutes.

The vinyl sulphide 14 is obtained via intermediate 13 by treating 12 with a halogen such as chlorine or bromine(X=C1 or Br) in a solvent such as ether, methylene chloride, tetrahydrofuran, glyme or the like at a temperature of from -78° to 30°C for from 1 to 30 minutes, followed immediately by treating with an olefin such as cyclohexene, isobutylene, or the like in the presence of base such as triethylamine, DBU, sodium hydride, or the like in a solvent such as DMF, glyme, THF, HMPA. The solution is held at -20° to 25°C for from 1 to 8 hours to yield 14.

The vinyl sulphide species 14 is reacted with a diester of oxomalonic acid (or its monohyarate) to provide 15. There is nC criticality, as to the identity of the ester moiety, R⁵, of the oxomalonic acid. R⁵ may be a conventional, easily removable blocking group or it may be a pharmaceutically acceptable ester moiety. Suitable ester radicals R are p-nitrobenzyl, benzyl, o-nitrobenzyl, t-butyl, 2, 2, 2-trichloroethyl. The reaction 14→15 is typically conducted in a high boiling organic solvent such as benzene, toluene, cyclohexane, halo aromatic or the like at a temperature of from about 50°C to reflux for from 0.5 to 6 hours.

The halogenation reaction 15→16 is typically conducted in a solvent such as THP, glyme, ether, methylene chloride, chloroform or the like in the presence of a halogenating agent such as thionyl chloride, phosphorous pentachloride or the like in the presence of base such as pyridine at a temperature of from -20° to 25°C for from 5 minutes to 3 hours. The selective reduction of 15→17 via 16 is completed by treating 16 with tributylphosphine, triphenylphosphine or the like in aqueous DMF or similar aqueous systems involving dioxane, THF, glyme, DMSO, or acetone at a temperature of from about 0-50°C for from 10 minutes to 5 hours.

Species 17 is halogenated by the previous procedure (12→13) but omitting the addition of the cyclohexene or other olefin, to provide the dihalo species 18. Species 18 is treated with a base such as triethylamine, sodium hydride or potassium hydride in a solvent such as DMF, acetonitrile, methylene chloride,_{'} chloroform, glyme or the like at a temperature of from about -78° to 25°C for 1 to 5 hours to provide 19. Species 19 is converted to 20 on treatment with a strong base such as 1,5-diazabicyclo 15.4.0]-under-5-ene(DBU),1, 5-diazabicyclo[3.4-0]non-5- ene(DBN), or the like in a solvent such as DMSO, acetone, chloroform, DM_{F}, _{T}HF, glyme or the like or on treatment with AgF in pyridine at a temperature of from 0-40°C for from 1/4 to 24 hours. The reaction 20 → 21 is conducted by treating 20 with an aromatic base such as pyridine, aqueous dimethylsulfoxide, 20→ lidine or lutidine, in the presence of a displacing agent such as lithium iodide, sodium chloride, lithium bromide, sodium bromide, or the like at a temperature of from about 80-150°C for from 15 minutes to 2 hours. An aqueous work up of the resulting reaction mixture provides 21. Isomerization of the double bond 21→22 is accomplished by treating 21 in a solvent such as DMF, DMSO, ethyl ether, THF, glyme, methylene chloride with a strong base such as diisopropylamine, DBU, DBN, or the like at a temperature of from 0° to about 25°C for from a few minutes to 2 hours or until equilibrium has been established as determined by examination of sample aliquots by ultraviolet absorption or by thin layer chromatography. The final reaction 22→I (hydrogenolysis of the blocking group) is accomplished by treating 22 in a solvent such as dioxane, ethanol, THF or the like or an aqueous mixture thereof in the presence of a Platinum metal catalyst such as Pd/C under a hydrogen pressure of from 1-4 atmospheres for from 0.5 to 8 hours at a temperature of from about 0-25°C.

The process of the present invention including the preparation of starting materials can be shown by the following Diagram II. It may advantageously start with 4-vinyl azetidinone[(23), below; E.J. Moriconi, W.C. Mayer, J. Org. Chem., 36, 2841(1971)7. This synthesis has the advantage of conveniently imparting stereoselectivity to the total synthesis of the antibiotics at an early stage.

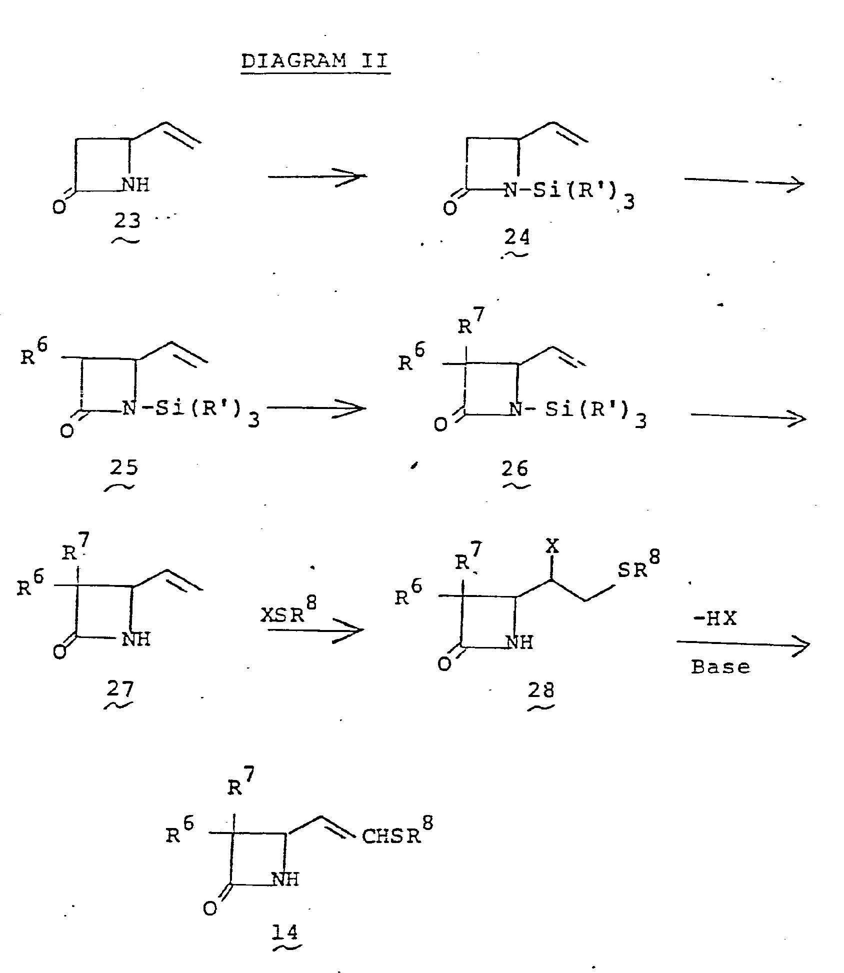

In words relative to the above reaction diagram 4-vinyl azetidinone 23 is silylated to provide the N-silyl species 24. The groups R' on the silyl radical are lower alkyl having from 1-6 carbon atoms especially preferred triorganosilyl groups are trimethylsilyl and t-butyl-dimethylsilyl. Typically, the silylation (23→ 24) is achieved by treating 23 in a solvent such as DMF, DMSO, HMPA or the like with the silylating agent of choice, dimethyl t-butylsilyl chloride, and a base such as Et₃N, pyridine, N,N-dimethylaniline and the like at a temperature of from -10° to 30°C for from 1 to 8 hours. Species 24 is alkylated to form 25 or 26 and this alkylation is conducted exactly as described above for the alkylation 7→8→9.It should be noted here that the reactions (24→25) and (25→26) represent convenient opportunities to separate species 25 and 26 into their racemic diastereoisomers if desired. The removal of the N-triorganosilyl group is accomplished intreaction 26→27 by mild acid catalyzed solvolysis. The halo sulfide species 28 is obtained from 27 by treating 27 in a solvent such as methylene chloride, THF, ^{g}lyme, or the like with the reagent XSR⁸ wherein _{R}⁸ has previously been defined and X is halogen such as chloro or bromo at a temperature of from -50° to 50°C for from 1 to 16 hours. The vinyl sulfide intermediate 14, which is common to the above illustrated scheme of total synthesis is obtained from 28 by elimination of HX on treatment of 28 with a base such as 1,5-diazabicyclo[5.4.0]undec-5-ene (DBU), 1,5-diazabicyclo[4·3·0] non-5-ene, (DBN), 1,4-diazabicyclo[2·2·2]octane, (_{D}ABCO), or silver fluoride in a solvent such as DMSO, pyridine, DMF, HMPA or the like at a temperature of from -20° to 50°C for from 1/4 to 16 hours.

### HSR⁸ and XSR REAGENTS

Relative to the foregoing description of the invention, suitable reagents HSR⁸ utilized in the transformation (11→12) and XSR⁸ (27→28) are listed below. The list'is arranged according to structural and functional characteristics of the thia side chain -SR⁸; annotation is provided where necessary. [It should be noted that only HSR⁸ reagents are expressly shown. The reagents XSR⁸ (X = Cl or Br) are shown implicitly for each entry on permissible substitution of Cl or Br for "H" in _{HSR}⁸.] The thia side chain of choice is derived from the corresponding mercaptan reagent HSR⁸. When the mercaptan contains a functional group which might interfere with the intended course of reaction, the offending group is covered. For example, when a basic nitrogen group is encountered (-NHR or -NH₂, for example) it is usually protected by acylation (e.g., -CO₂PNB) and when a carboxyl group (-CO₂H) is present, it is usually protected by esterification (e.g., PNB ester). Such protection also facilitates in the purification of products by chromatographic means. (PNB is p-nitrobenzyl). It should be noted that the processes incorporated by reference below.[1.) Process for the Preparation of 1-Carbapenems and Intermediates via 4-Allylazetidinone; 2.) Process for the Preparation of 1-Carbapenems and Intermediates via Trithioorthoacetates; and 3.) Process for the Preparation of 1-Carbapenems and Intermediates via Silyl-Substituted Dithioacetals] are preferred when R is unsaturated.
1.) Aliphatic Mercaptans: HSR⁸ wherein R⁸is 1-10 carbon alkyl, cycloalkyl, alkenyl, cycloalkenyl, or alkynyl; R⁸ may be branched or unbranched, Examples
   HSCH₃
   HSCH₂CH₃
   HSCH₂CH₂CH₃
   HSCH(CH₃)₂
   HS(CH₂)₃CH₃

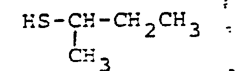

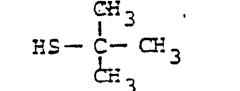

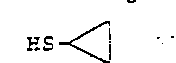

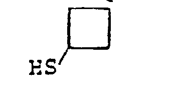

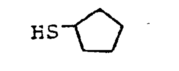

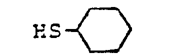

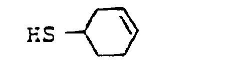

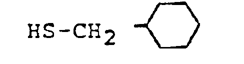

   HS-CH₂-CH=CH₂
   HS-CH₂-CH=C(CH₃)₂
   HS-CH₂-C≡CH
   HS-CH₂-C≡C-CH₃
2.} Substituted Aliphatic Mercaptans: HSR⁸ wherein R⁸ is a 1-10 carbon branched or unbranched alkyl, cycloalkyl, alkenyl, cycloalkenyl, or alkynyl group substituted by one or more halo, OH, OR¹, O 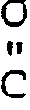 R¹, O 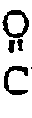 NH₂, O 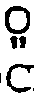 NHR¹R², NH₂, NR¹R², 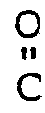 R¹, CO₂H, CO₂R¹, CONH₂, CONHR¹, CONHR¹R², CN, SR¹, 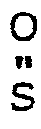 R¹, SO₂R¹, SO₂NH₂, SO₂NHR¹, SO₂NR¹R², NH 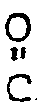 R¹ NH 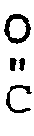 NH₂, NH 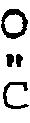 NHR¹, NH 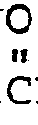 NR¹R² , NH 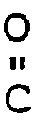 OR¹ , 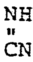 H₂, 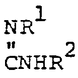 , wherein R¹ and R² are as previously defined relative. wherein R¹ and R² are as previously defined relative. to substituents on R⁸. Preferred substituents are basic nitrogen-containing groups.

### EXAMPLES

HS(CH₂)ₙOR¹ n = 2-4, R¹= H, 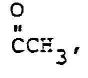 CH₃ 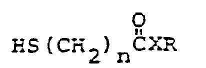 n=1-3, X=O, NH, NR¹; R, R¹=H, CH₃ HS(CH₂)ₙNH₂ n=2-4 HS (CH₂)ₙNHR¹ n=2-4, R¹CH₃, CH₂CH₃, CH₂CH₂CH_{3'} 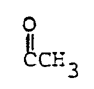 HS(CH₂)NR¹R² n=2-4, R¹/R²=CH₃ CH₂CH₃ 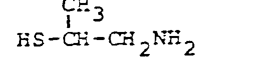 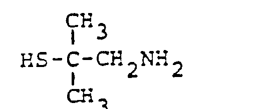 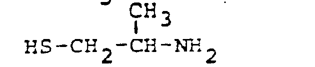 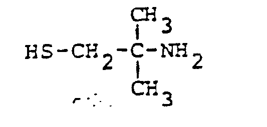 HS-CH₂CH₂SCH₃
HS-CH₂CH₂NHC(CH₃)₃ 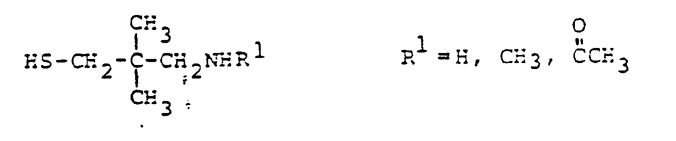 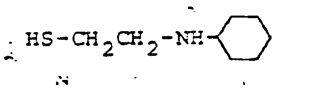 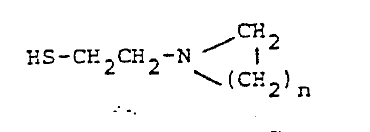 n=3-5 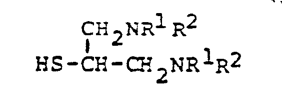 R¹=H, CH₃; R2=H, CH₃ 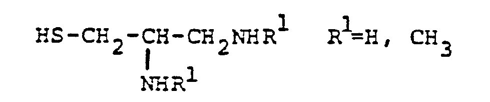 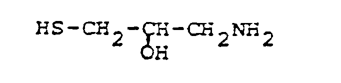 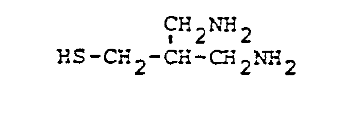 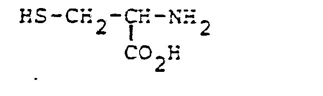 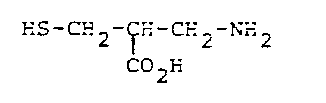 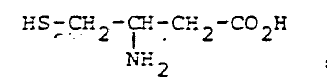 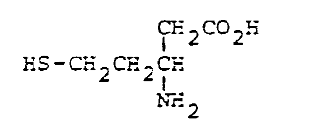 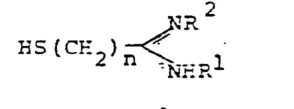 n=1-3, R²=H, CH₃, R¹=H, CH₃ 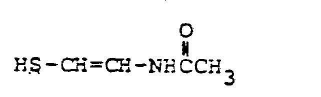 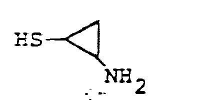 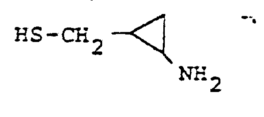 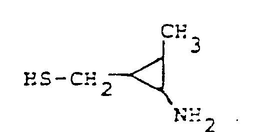 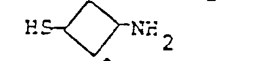 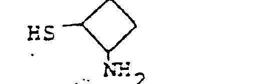 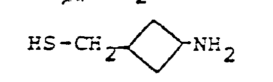 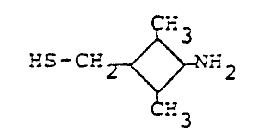 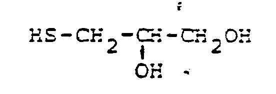 5-thio-D-glucose 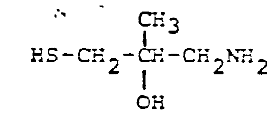 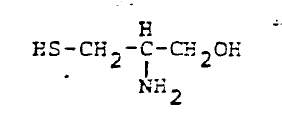 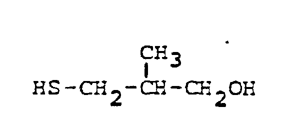 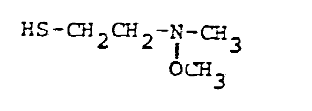 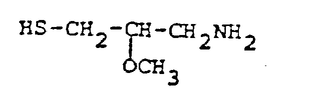 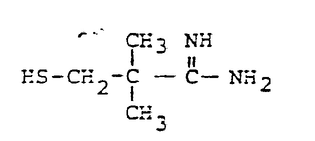 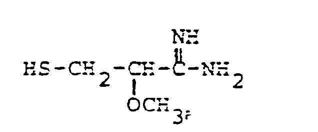 HS-CH₂-CH=CH₂-CH₂NH₂

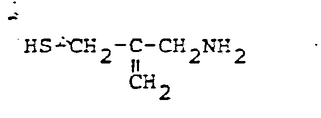

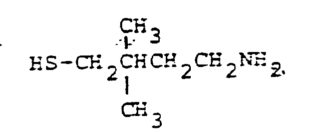

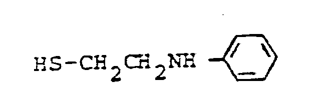

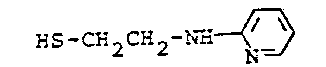

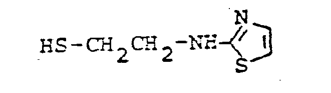

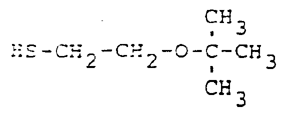

3. ) Aryl Mercaptans: HSR⁸ wherein R⁸ is phenyl or substituted phenyl. The substituents are independently selected from those previously defined for _{R}⁸. Especially preferred substituents include alkyl, halo, hydroxy, alkoxy, acyloxy, acyl, carboxy, mercapto, sulfinyl, sulfonyl, amino, substituted amino aminoalkyl, substituted amino- alkyl, amido, and ureido. 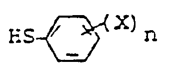
n= 1, 2 or 3,
X= F, Cl, Br, OH, OR, 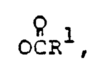 NH₂, NHR¹, NR¹R² , CH₂NH₂ , CH₂NR¹R² , CO₂H, CO₂R¹, COR¹, CONH₂, CONR¹R², R¹CONH, R¹NHCONH, SR¹, 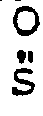 SR¹, SO₂R¹, CH₃, CF₃; R¹ and R² are as previously defined under R⁸.

### Examples

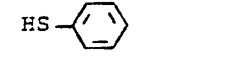

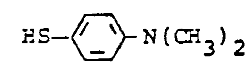

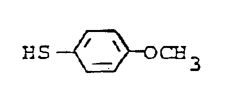

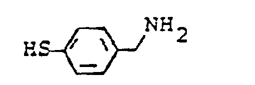

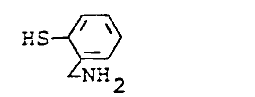

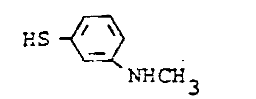

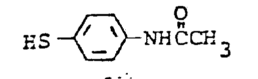

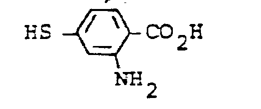

4.) Heteroaryl Mercaptans: HSR⁸ wherein R⁸ is a substituted or unsubstituted heteroaryl group containing 1-4 O; N or S atoms. Typical substituents include those mentioned above under "Aryl Mercaptans".

### Examples

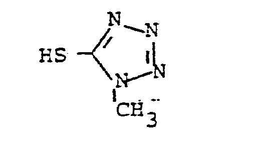

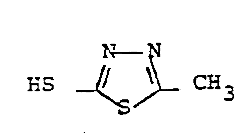

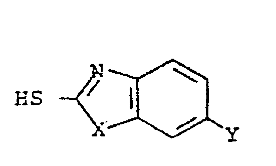

X=N,O Y=H
X=S Y=H, Cl, OCH₂CH₃ 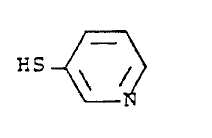 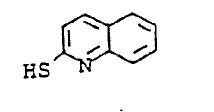 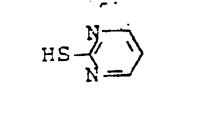 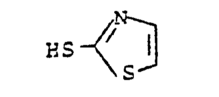 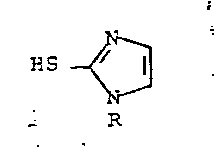
R=H, CH₃ 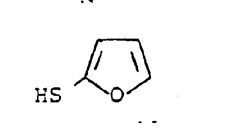 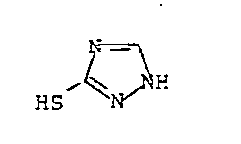 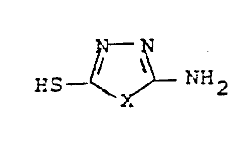
X=NH, S

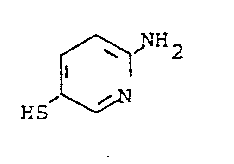

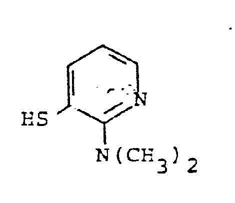

5.) Arylaliphatic Mercaptans: HSR⁸ where R⁸ is a 1-6 carbon branched or unbranched alkyl, cycloalkyl, alkenyl, or alkynyl group substituted by a phenyl or substituted-phenyl group. Typical phenyl substituents include those mentioned under "Aryl Mercaptans".

### Examples

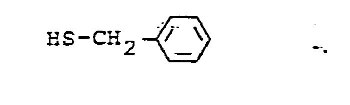

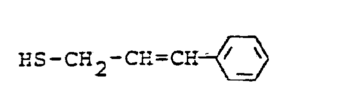

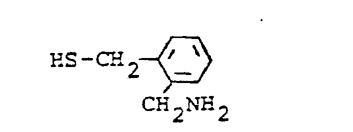

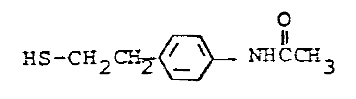

### 6.) Heteroarylaliphatic and Heterocyclylaliphatic Mercaptans

HSR⁸ wherein R⁸ is a 1-6 carbon branched or unbranched alkyl, cycloalkyl, alkenyl, or alkynyl croup substituted by a heteroaryl or heterocyclyl group containing 1-4, O, N, or S atoms. The heteroaryl or heterocyclic group is unsubstituted or substituted by those substituents mentioned under "Aryl Mercaptans", (No. 3 above).

### Examples

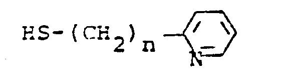

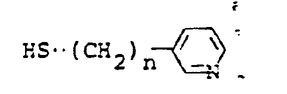

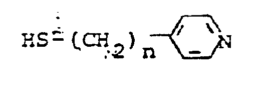

n=1,2 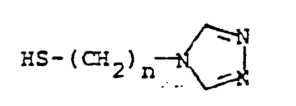 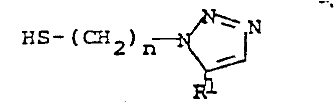
R¹= = OCH₂CH₃ 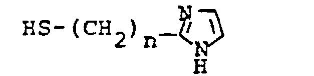 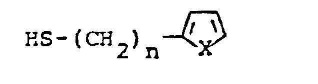 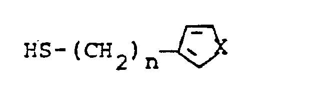
X=O, S , NH 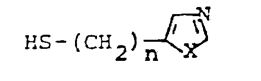
X = O, S, NH 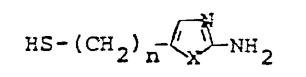
X = O, S, NH 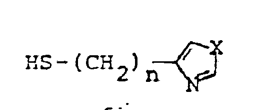
X= O, S, NH 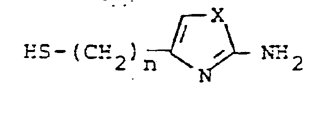
X= O, S, NH 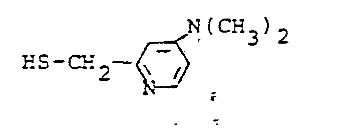 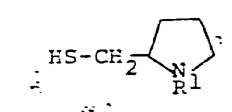
R¹ = H, CH₃ 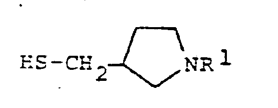 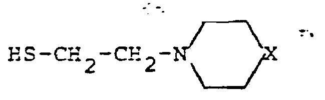
X= O, NH, NCH₃ 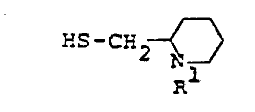
R¹ = H, CH₃ 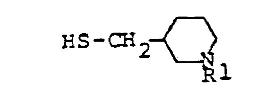 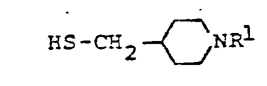
R¹=H, CH₃ 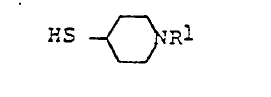
R¹ = H, CH₃ 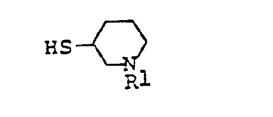
R¹=H, CH₃ 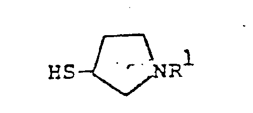
R¹=H , CH₃ 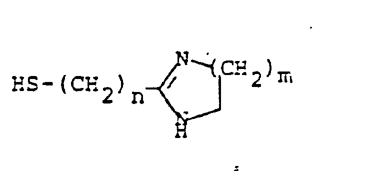
n=1-3, m=1-3 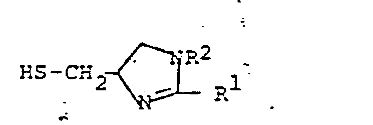
R²=H, CH₃, R¹=H, CH₃, NH₂ 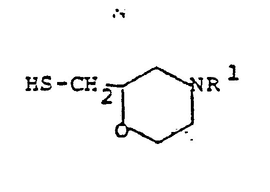
R¹=H, CH₃

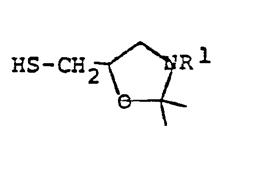

7.) Alkyl-Heteroetoatom-Alkyl Mercaptans, HSR⁸ Wherein R⁸ is
-(CH₂)ₙX(CH₂)ₘR⁹

wherein n = 2 to 4, m = 2 to 4; X is NR', O or S; and wherein R° is H, CH₃, CH₂CH₃, CH₂CH₂OH, or CH₂CH₂NH₂ and R⁹ is OH, NH₂, NHCH₃ N(CH₃)₂, OCCH₃, NHC_{O}CH₃.

Note, in the above representation, the methylene carbons may be branched; for example: 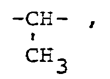 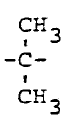 and the like.

The following HSR⁸ are representative of this class:

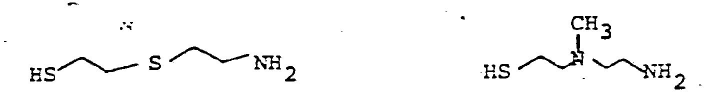

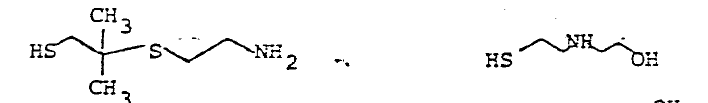

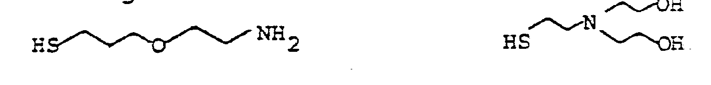

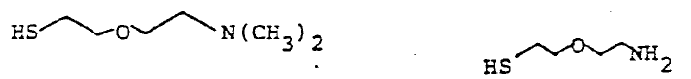

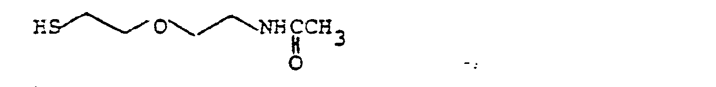

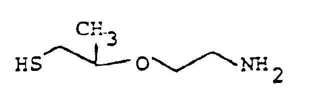

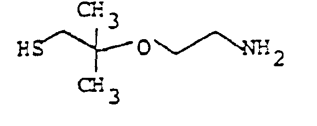

### ALKYLATING AND ACYLATING REAGENTS FOR ESTABLISHING R⁶ AND R⁷

Relative to Diagrams I and II above, the establishment of R⁶ and R⁷ by alkylation has been shown (7→8→9, Diagram I; and analogously 24→25→26, Diagram II). There is yet a third scheme for establishing R⁶ and R⁷. It involves direct acylation followed by reduction. The schemes are conveniently compared below (Diagram III) and, there following, is a representative list of suitable alkylating and acylating reagents for establishing R⁶ and R⁷. 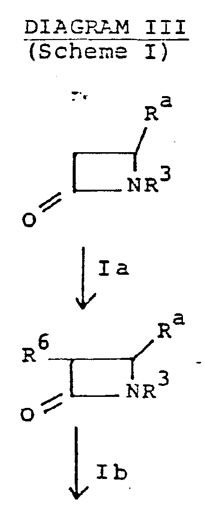 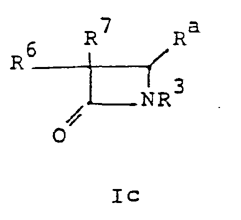 wherein :
R^{a} is CH₂CH₂CH₂ or CH=CH₂ ;
R³ and R² are as defined above.

In words relative to the above reaction diagram, and as described above, starting material Ia can be. mono-, or dialkylated at ring position 3. Alkylation of Ia provides Ic. Typically, Ia is treated with a strong Lase such as lithium diisopropylamide, lithium 2,2,6,6-tetramethylpiperidide, potassium hydride, lithium hexamethyldisilazane, phenyllithium or-the like in a solvent such as tetrahydrofuran (THF), hexamethylphosphoramide, ether, dimethoxyethane, and the like at a temperature of from -80°C to 0°C whereupon the alkylating agent of choice, R⁶X° is added (X° is chloro, iodo or bromo); alternatively the alkylating agent may be R -tosylate, _{R}⁶⁻mesylate or an aldehyde or ketone such as acetaldehyde to provide monoalkylated species Ib. When desired, dialkylated species Ic may be obtained from Ib by repeating the alkylating procedures Ia→Ib.

The eventual 6-substituents (nomenclature relative to final, bicyclic structure) can also be established by direct acylation using an acylating agent such as N-acyl imidazole or the like. Such N-acyl imidazole acylating reagents are listed below. Also given below is a detailed description of this second approach for establishing, R⁶ and _{R}⁷.

The following list is representative of useful alkylating agents for establishing R⁶ and _{R}⁷, according to the above scheme 7→→9; and 2→→26 (this will be referred to as Scheme I, to be distinguished from Scheme II, below, which involves acylation) :

### Alkylating Agents

CH₃CHO
ØCH₂CHO ⌀ = phenyl
ØCH₂CH₂CHO
CH₂O
CH₃I
ØCH₂Br
CH₃COCH₃

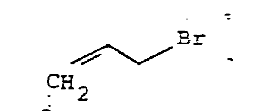

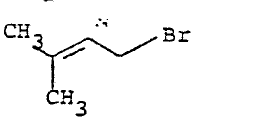

CH₃OCH₂CHO
CH₃CH₂I
(CH₃)₂CHI
N₃CH₂CHO
(CH₃) 2NCH2C-HO
R0₂CCH₂Br R = CH₃, benzyl, p-nitrobenzyl
CF₃CF₂CHO
RO₂CCH₂CHO R = CH₃, benzyl, p-nitrobenzyl
CH₃CH(CH₃)CHO,
CH₃ (CH₃) CHCH₂CHO,
CH₃CH₂CHO, 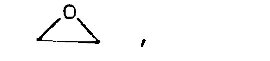
CFCHO, 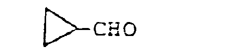 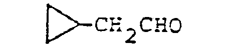 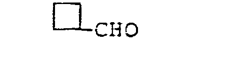 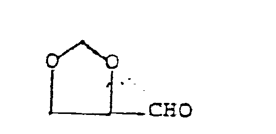 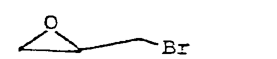 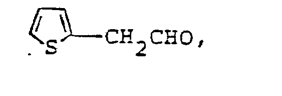 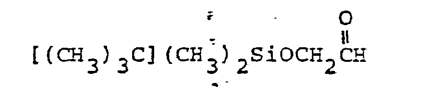 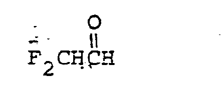 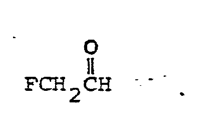 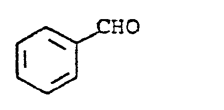 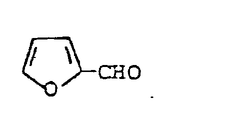 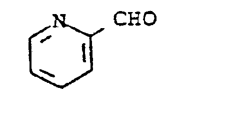 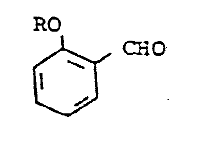 R = protecting group 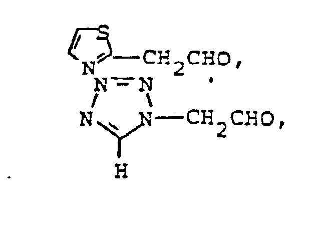 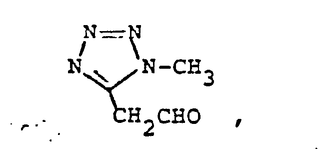 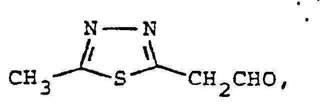 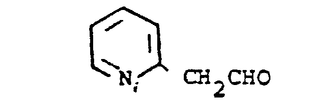 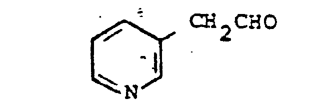 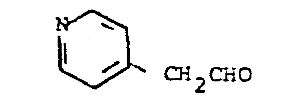 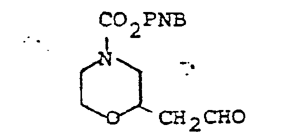 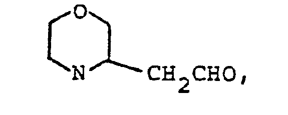 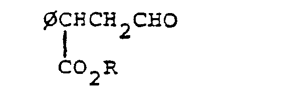
R is removable carboxyl protecting group, such as benzyl.

As mentioned above, the 6-substituents may also be established by acylation. Utilization of such acylating agents may be demonstrated in the following manner with regard to a preferred starting material Ib or Ic? 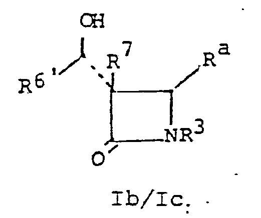 wherein R⁷, R and R³ arc as defined above. R^{6'} is defined relative to the definition of R⁶ and in that sense is the balance of the previously identified group R^{6.} In other words, for purposes of this definition R^{6'}CH(OH)- = R⁶. An especially preferred material Ibis when R⁷ is hydrogen and R⁶' is methyl. Basically,-such 1'-hydroxy R^{6'} species Ib are prepared according to the following scheme:

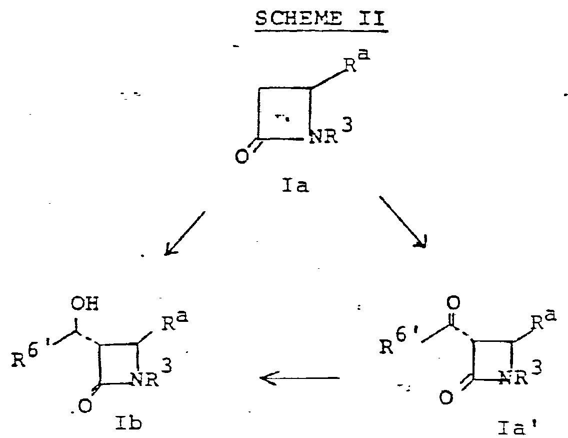

The alkylation Ia ― Ib Scheme II, is accomplished as previously described, by treating Ia in a solvent such as tetrahydrofuran, dimethoxyethane, diethylether, hexamethylphosphoramide, at a temperature of from -100° to -20°C with a strong base such as lithium diisopropylamide, lithium hexamethyldisilazide, lithium 2,2,6,6-tetramethylpiperidide, potassium hydride or the like followed by the addition of an equivalent to 10 fold excess of an aldehyde. This reaction gives a mixture of isomers from which the , desired trans-R form Ib can be conveniently separated by chromatography or crystallization.

Intermediate Ia may proceed directly to Ib as indicated above, or it may take the circuitous path via Ia'. The direct acylaticn, to Ia ' is acomplished by treating Ia with two or more equivalents f a base such as lithium diisopropylamide, lithium 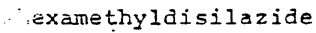 , lithium 2,2,6,6-tetramethyl- piperidide in a solvent such as tetrahydrofuran, diethylether, or dimethoxyethane, for example, at a temperature of from -100 to -20°C with an acylating agent such as N-acyl imidazole or the like. Addition of the Ia plus base mixture to the acylating agent is preferred.

Representative acylating agents for this scheme Ia→Ia'→Ib are listed below. 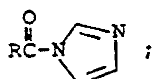 R=CH₃, ClCH₂. CH₃CH₂, N₃CH₂. CH₃OCH₂,

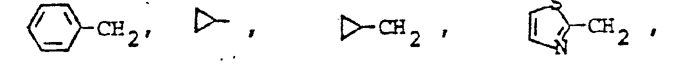

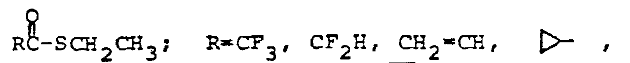

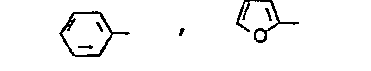

Further with respect tc Scheme II, the reduction, Ia' →Ib is accomplished by contacting the ketone with a reducing agent such as potassium tri (sec-butyl) borohydride, lithium tri(sec-butyl)borohydride, sodium borohydride, sodium tris (methoxyethoxy) aluminum hydride, lithium aluminum hydride or the like in a solvent such as diethylether, tetrahydrofuran, toluene, i-propanol or the like at a temperature of from -78° to 25°C. The reaction can conveniently be conducted in the presence of an added complexing salt such as potassium iodide, magnesium bromide or the like.

In a similar manner, urresolved Ib (cis and trans) may be oxidized toIa' for reduction to Ib as indicated above:

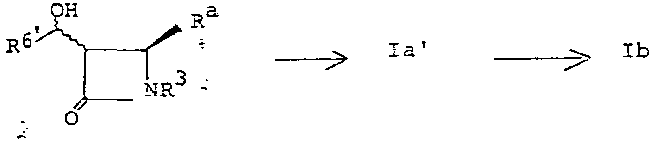

The oxidation is accomplished with an oxidizing agent such as dipyridine chromium (VI) oxide, trifluoroacetic anhydride-dimethylsulfoxide-triethylamine, pyridinium dichromate, acetic anhydride-dimethylsulfoxide in a solvent such as methylene chloride, acetonitrile, or the like at a temperature of from -78 to 25°C for from 5 minutes to 5 hours.

In the foregoing word description of the above schematic reaction diagram for the total synthesis of the defined carbapenem antibiotics, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents. Further, it is to be understood that the presentation of the synthetic scheme as comprising distinct steps in a given sequence is more in the nature of a descriptive convenience than as a necessary requirement; for one will recognize that the mechanically dissected scheme represents a unified scheme of synthesis and that certain steps, in actual practice, are capable of being merged, conducted simultaneously, or effected in a reverse sequence without materially altering the progress of synthesis.

The following examples recite a precise scheme of total synthesis. It is to be understood that the purpose of this recitation is to further illustrate the total synthesis and not to impose any limitation.

### Incorporation by Reference

The compounds of the present invention may also used for preparing the compounds disclosed and claimed in the three (3) following, co-pending, commonly assigned, concurrently filed U.S. Patent Applications of Christensen, Ratcliffe and Salzmann. To the extent that these applications define R⁶ , R⁷ , and R⁸
they are hereby incorporated by reference 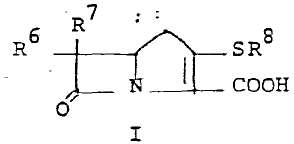
1.) Process for the Preparation of 1-Carbapenems and Intermediates via 4-Allylazetidinone; U.S. Patent Application Serial Number , filed [Merck & Co., Inc. Attorney's Docket Case 16479].
2.) Process for the Preparation of 1-Carbapenems and Intermediates via Trithioorthoacetates; U.S. Patent Application Serial Number , filed [Merck & Co., Inc. Attorney's Docket Case 16485].
3.) Process for the Preparation of 1-Carbapenems and Intermediates via Silyl-Substituted Dithioacetals; U.S. Patent Application Serial Number , filed [Merck & Co., Inc. Attorney's Docket Case 16478].

Also incorporated by reference is Belgian Parent 848,545 (which corresponds to co-pending, commonly assigned U.S. Serial Number 852,425 filed 11-17-77, now U.S. Patent 4,194,047 issued 3-18-80). This patent discloses and claims processes for converting the natural product thienamycin to certain amino derivatives:

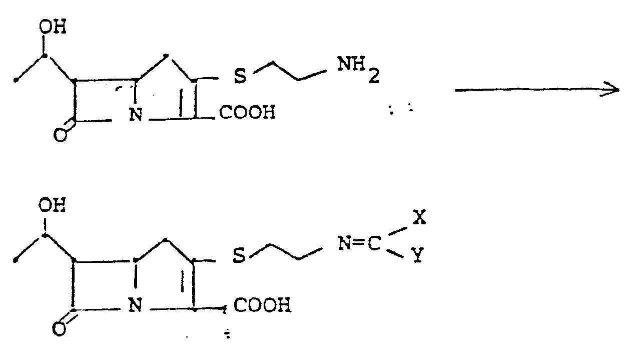

The process disclosed in the cited Belgian Patent is also suitable to prepare preferred, antibiotic embodiments of the present invention (Structure I', see below). The applicability of the process arises from the presence of an amino group on previously defined side chain -SR⁸ of the compounds of the present invention, Structure I, thus: 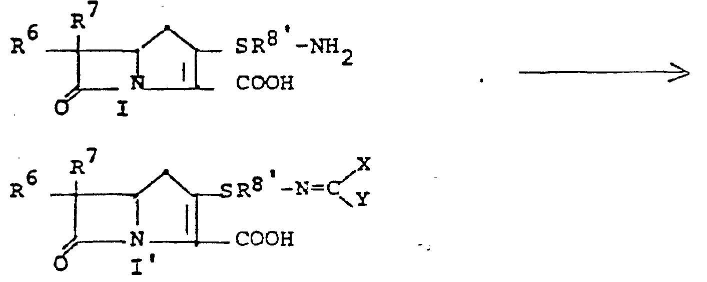 wherein:-SR^{8'} -NH₂ = -SR⁸; that is, the symbol -SR^{8'} -NH₂ indicates, and is specific to -SR⁸, above-defined, bearing an amino substituent; preferred values for X and Y include: X=NH₂; Y=H, CH₃, NH₂.

Thus; to the extent that the Belgian Patent describes the amino derivatization process, the generic definition of X and Y and the preferred, above-indicated, amidine and guanidine embodiments, it is hereby incorporated by reference.

Also incorporated by reference is published European Patent Application 0007614 (Application Number 79102615.6, filed 24 July 1979). This application discloses certain dipeptidase inhibitors which, on co-administration to mammalian subjects, enhance the efficacy of certain 1-carbadethiapenem antibiotics. Thus, to the extent that the cited application: l.) defines the manner by which susceptible carbadethiapenems substrates of the present invention may be identified; and 2.) discloses suitable inhibitors, compositions, and methods of treatment, it is incorporated herein by reference. A particularly preferred inhibitor is 6-(L-2-Amino-2-carboxyethylthio) -2-(2,2-DCC) -2-hexenoic acid.

### EXAMPLE 1

### Step A

### Preparation of [1-(t-butyldimethylsilyl)-4-vinyl-2- azetidinone]

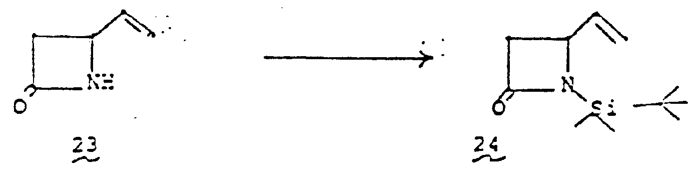

A solution of 23 [4-vinyl-2-azetidinone] (1.153 g, 11.89 mmoles) and triethylamine (1.82 ml, 13.08 mmoles) in anhydrous N,N-dinethylformamide is placed under a nitrogen atmosphere, cooled to 0°C and treated with t-butyldimethylchlorosilane (1.885 g., 12.48 mmoles) resulting in the immediate appearance of a heavy white precipitate. This mixture is stirred for one hour while gradually warming to room temperature. The mixture is partitioned between 30 ml methylene chloride and 90 ml cold 1M potassium dihydrogen phosphate. The aqueous phase is extracted with 20 ml methylene chloride. The combined organic phases are washed four times with 30 ml portions of water and finally with 50 ml brine. The methylene chloride solution is dried over anhydrous magnesium sulfate and filtered. The filtrate is evaporated under reduced pressure to give 2.25 g of 24 (1-(t-butyldimethylsilyl)-4-vinyl-2- azetidinone] as a colorless liquid.
NMR (CDCl₃) δ: 6.23 - 5.10, m, CH=CH₂
4.07, 7-line m, J=8,6 and 3Hz, C-4H
3.35, dd, J=15 and 6Hz, C-3H cis to C-4H
2.73, dd, J=15 and 3Hz, C-3H trans to C-4 H
.98, s, (CH₃)₃ C Si
.23, s
.18, s (CH₃)₂ .Si

Following the above procedure, but making the indicated substitution, the other isomer is obtained.

### Step A'

### Preparation of 3-methyl-(t-butyldimethylsilyl)-4-vinyl-2-azetidinone

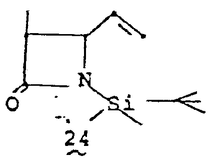

Following the procedure of the preparation of 8-oxo-2, 2,7-trimethyl-3-oxa-1-azabicyclo[4-2·0]octane (Example 4b, above), except that an equivalent amount of 1-(t-butyldimethylsilyl)-4-vinyl-2-azetidinone is substituted for the 8-oxo-2,2-dimethyl-3-oxa-1-azabicyclo[4.2.0] octane of Example 4b, the title compound is obtained.

### Step'B

### Preparation of 3-methyl-[1-(t-butyldimethylsilyl)-3-(hydroxymethyl)-4-vinyl-2-azetidinone)

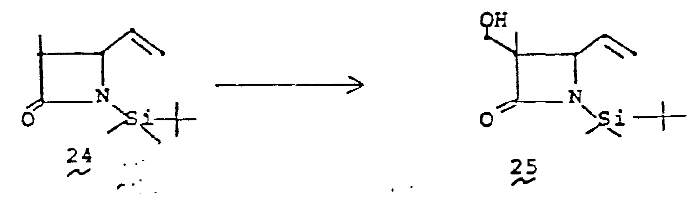

To a solution of freshly prepared lithium diisopropylamide (7.82 mmoles) in 36 ml anhydrous tetrahydrofuran under a nitrogen atmosphere at -75°C is added a solution of 3-methyl-[1-(t-butyldimethylsilyl)-4-vinyl-2-azetidinone, 24, (1.60 g, 7.11 mmoles) in 10 ml anhydrous THF. The resulting yellow solution of the lithium enolate is, after 16 minutes, treated with excess formaldehyde In 10 minutes, the reaction is quenched by adding 30 ml of a saturated aqueous ammonium chloride solution. This mixture is extracted with 50 ml and 25 ml portions of ethyl acetate. The combined ethyl acetate solutions are washed with 50 ml of brine and dried over anhydrous magnesium sulfate. The drying agent is removed by filtration and the filtrate is evaporated in vacuo to give the crude product as a yellow oil. Purification by chromatography on silica qel eluting with 10% ethyl acetate/ chloroform gives 3-methyl-[l-(t-butyldimethylsilyl)-3-hydroxymethyl)-4-vinyl-2-azetidinone, 25.

### Step C

### Preparation of 3-methyl-1-(t-butyldimethylsilyl)-3-(1-p-nitrobenzylcarbonyldioxymethyl)-4-vinyl-2- azetidinone

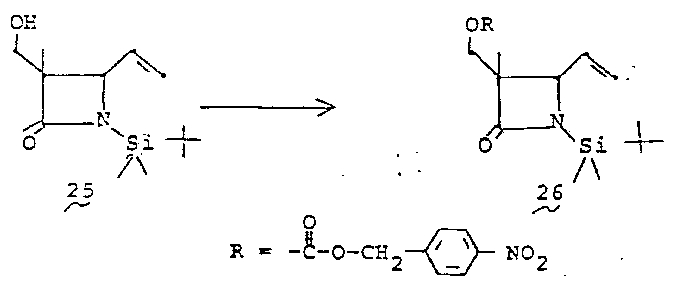

Under nitrogen at -78°C a solution of 25 (56 mg, .220 mmole) in 1 ml of anhydrous tetrahydrofuran is treated with 2.4M n-butyllithium in hexane (101 µl, .242 mmole). To this solution is added, in five minutes, a solution of p-nitrobenzyl chloroformate (52 mg, .242 mmole) in anhydrous tetrahydrofuran. After stirring at -78°C for a period of 55 minutes, 10 ml of a saturated aqueous ammonium chloride solution is added and the product extracted into ethyl acetate. The combined ethyl acetate solutions are washed with brine and dried over anhydrous magnesium sulfate. The drying agent is removed by filtration, and the filtrate is evaporated in vacuo to give crude 26. Purification by preparative thick-layer chromatography on silica gel developing with 5% ethyl acetate/chloroform gives 26.

### Step D

### Desilylation of 26 to provide 27 [3-methyl-3- (p-nitrobenzylcarbonyldioxymethyl)-4-vinyl-2-azetidinone]

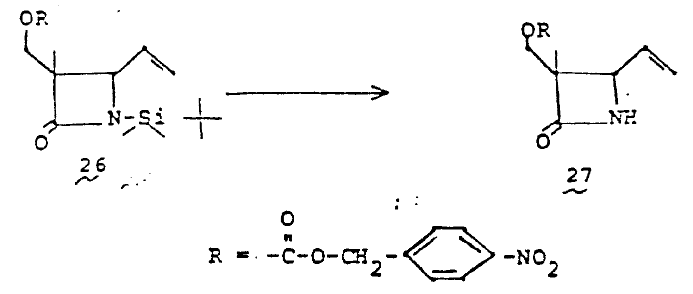

A solution of 26 [1-(t-butyldimethylsilyl)-3-methyl-3-p-nitrobenzylcarbonyldioxymethyl)-4-vinyl-2-azetidinone] (61 mg, .141 mmole), in 2 ml of .5N HCl/MeOH is stirred at room temperature (25°C) for a period of 3 hours. The solution is then cooled to 0°C and neutralized by the addition of 5 ml of.5% aqueous sodium bicarbonate. The . product is extracted into ethyl acetate (10 ml, 2x5 ml). The combined ethyl acetate solutions are washed with water (2 x 5 ml) and 10 ml brine and then dried over anhydrous magnesium sulfate. The drying agent is removed by filtration, and the filtrate is evaporated in vacuo to give an oil. Preparative thick-layer chromatography of this material on silica gel developing with 10% ethyl acetate/chloroform gives 3-methyl-3-(p-nitrobenzyl- carbonyldioxymethyl)-4-vinyl-2-azetidinone, 27.

### Step E

### a

### Preparation of 14 via 28 by sulfenyl halide addition and dehydrohalogenation

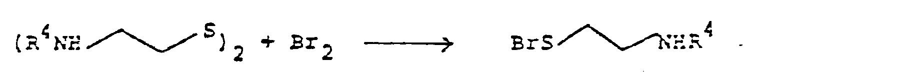

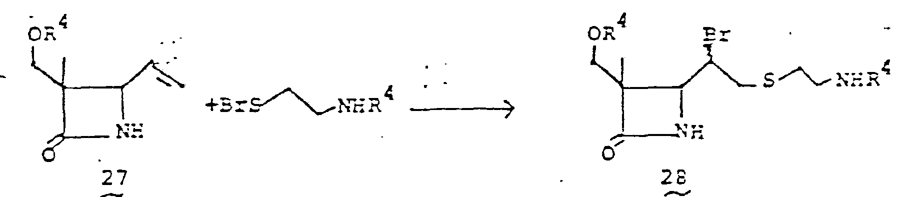

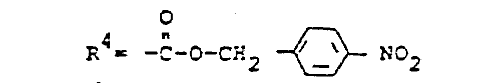

A solution of the N-p-nitroCBZ cysteamine disulfide, 96 mg (0.19 mmoles) in 1.5 ml THF (freshly distilled from LiAlH₄) is cooled to -25°C and treated dropwise with stirring with 0.5 ml of a solution of 135 mg Br₂ in sieve dried CC1₄ (2.2 ml final volume; portion added is equivalent to 0.19 mmoles of Br₂) . The resultant orange solution is stirred at -20°C for 5 min. then treated with 54.0 mg of the vinyl azetidinone, 27, in 0.5 ml sieve dried CH₂Cl₂. The color lightens to yellow. The mixture is allowed to come to 0°C over 5-10 minutes. Examination by t1c (silica 5% MeOH in CH₂C1₂ or 20% EtOAc in CH₂Cl₂) shows a main spot with _{Rf} and Ce^{IV+}/H⁺/heat characteristics different from either disulfide or starting 4-vinyl-2- azetidinone. The reaction mixture is concentrated to 0.5 ml under N₂, streaked directly on two 8" x 8" 1000µ silica GF plates, and developed with 20% EtOAc in CH₂C1₂. The main band under U.V., is scraped off, and extracted with EtOAc to aive 28.

### Step F

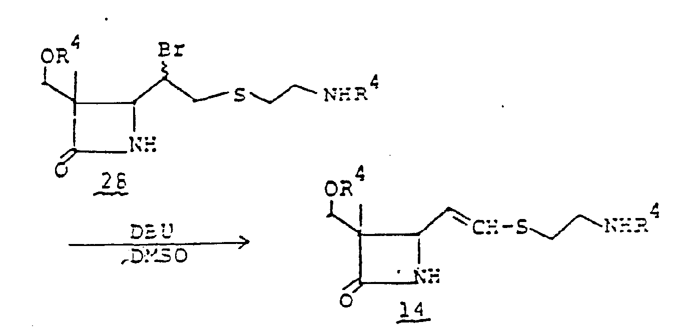

The bromosulfide, 28, 77.0 mg (0.162 mmole) is dissolved in 1.0 ml sieve stored DMSO (distilled from - CaH₂) , and stirred under nitrogen while 25λDBU (0.19 mmole) is added. After 3 hours, the mixture is poured into water/KH₂PO₄ and extracted repeatedly with EtOAc. The combined extracts are washed twice with water, dried with anhydrous MgSO₄ and evaporated under nitrogen. The crude product, is streaked on an 8 x 8" 1000µ silica GF plate and developed with 20% EtOAc in CH₂Cₗ2 to give 14.

### EXAMPLE 2

### Preparation of Bis (p-Nitrobenzyloxycarbonylaminoethyl)-disulfide

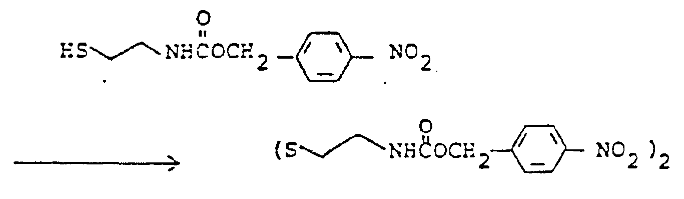

Under nitrogen at -20°C, bromine (1.21 ml, .022 mmole) is added to a solution of p-nitrobenzyloxycarbonyl- aminoethanathiol (11.28g, .044 mole) in 100 ml of an- hydrous tetrahydrofuran. The cooling bath is removed, and the cold solution is stirred for 15 minutes. The solution is then diluted with 400 ml ethyl acetate and washed with 200 ml 1M pH 7 phosphate buffer, 200 ml 1M dibasic potassium phosphate, water (2 x 200 ml, 100 ml) and 200 ml brine. It is dried over anhydrous magnesium sulfate and filtered. The filtrate is evaporated in vacuo giving a yellow solid residue. This material is chromatographed on silica gel eluting with 5% ethyl acetate/chloroform to give 10.5 g of crystalline bis (p-nitrobenzyloxycarbonylaminoethyl)disulfide:
IR (CH₂Cl₂) µ : 3.04NH
   5.96 carbonyl
   6.22, 6.61 nitro
NMR (CDCl₃)δ: 8.24
   d, J=8.5Hz, ArH
   7.54
   5.37, broad s, NH.
   5.26, 5, ArCH₂O
   3.60, q, J=6Hz and 6Hz, NHCH₂CH₂
   2.86, t, J=6Hz, NHCH₂CH₂S

### EXAMPLE 3

### Preparation of 3-methyl-3-(p-nitrobenzylcarbonyldioxy- methyl)-4β-[1-bromo-2-(2-p-nitrobenzyloxycarbonylamino) 1,1-dimethylethylthio) ethyl]-2-azetidinone

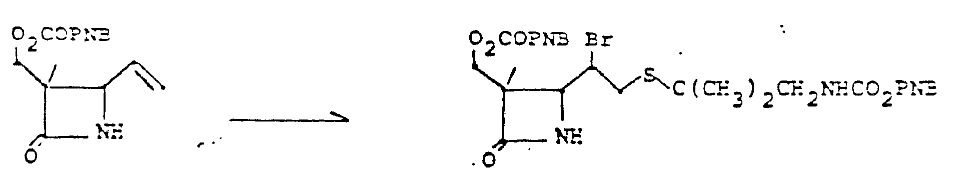

If in Example 1 Step E, an equivalent solution of 2-(p-nitrobenzyloxycarbonylamino)-1,1-dimethylethylsulfenyl bromide, prepared by cleavage of bis(2-(p-nitrobenzyloxycarbonylamino)-1,1-dimethylethylthio)mercury with bromine in THF/ether at 0°C., is substituted for the solution of 2-(p-nitrobenzyloxycarbonylamino)ethylsulfenyl bromide, the title compound is obtained.

### EXAMPLE 4

### Preparation of 3-(2-amino-1,1-dimethylethylthio)-6-methyl-6-(1-hydroxymethyl)-7-oxo-1-azabicyclo[3·2·0] bept-2-ene-2-carboxylic acid

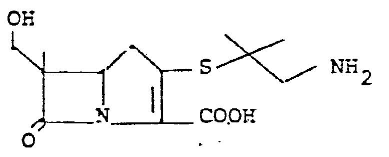

Following the procedure of Example 1 Step F, except substituting for the indicated azetidinone the azetidinone of Example 3 followed by the reactions corresponding to those of Example ₄a D-K, the title compound is obtained.

### EXAMPLE 4a

### Preparation of 3-(2-aminoethylthio)-6-methyl-6-(hydroxymethyl)-7-oxo-1-azabicyclo[3.2.0]hept-2-ene-2-carboxylic acid

### Step

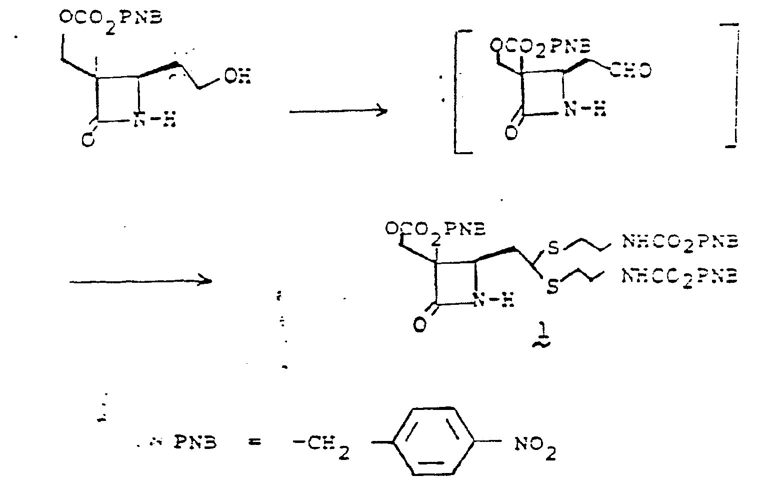

To 6.75 ml anhydrous pyridine (mw=79:ρ =0.982; 83.9 mmole) in 350 ml anhydrous acetonitrile is added 4.05 g anhydrous powdered'chromium trioxide (mw=100; 40.5 mmole). After stirring at room temperature (25°C) for 30 minutes, 9.6g dried Supercell is added and stirring is continued for 5 additional minutes. A solution of 3.21g 3-methyl-3-(p-nitrobenzylcarbonyl- dioxymethyl)-4-(2-hydroxyethyl)-2-azetidinone (mw = 338; 9.5 mmole) in 30 ml anhydrous acetonitrile is added all at once. The reaction mixture is stirred under anhydrous conditions at room temperature (25°C) for one hour. Addition of 9.6g NaHSO₃ is followed by 5 minutes of stirring after which the reaction mixture is filtered through a mixed packed bed of 40g silica gel and 40g anhydrous magnesium sulfate. The bed is washed repeatedly with acetonitrile (total volume of filtrate~600 ml). The filtrate is concentrated under a N₂ stream to 130 ml total volume. To this solution containing crude aldehyde at 0 °C under N₂ is added 9.64c p-nitrobenzyloxycarbonylaminoethanethiol (mw = 256; 37.7 mmole) as prepared below (Example 12, Step B). To the stirred reaction mixture is added 8.0 ml boron ) trifluoride etherate (mw = 142; ρ =1.125; 63. 4 mmole) . After 1.5 hours at 0°C, the reaction mixture is poured into a stirred ice-cold mixture of 69c K₂HPO₄ - 500 ml H₂O and 700 ml ethyl acetate (EA). The layers are separated, and the aqueous one is saturated with NaCl and re-extracted with additional EA. The combined organic layers are washed twice with brine, dried over anhydrous MgSO₄ and filtered. The filtrate is concentrated under a N₂ stream and then pumped on high vacuum to give crude 1.

The material is chromatographed on 450g silica ^{g}el (column height = 48 cm; diameter = 5.5 cm) packed and applied in CHCl₃ and eluted with increasing percentages of MeOH in CHCl₃ (0-4% MeOH/CHCl₃). Those fractions containing the desired product are combined, concentrated under a N₂ stream; and pumped on high vacuum to give 1.

### Step B

### Preparation of p-Nitrobenzyloxycarbonylaminoethanethiol

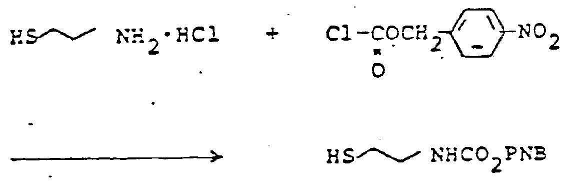

To 600 ml diethyl ether (Et₂O) - 75 ml H₂O in an ice bath with stirring is added 3.2g cysteamine hydrochloride (mw = 114; 28.1 mmole). A solution of 7.14g NaHCO₃ (mw = 84; 85 mmole) in 75 ml H₂0 is added. The ice bath is removed, and at room temperature a solution of 6.75g p-nitrobenzylchloroformate (mw = 216; 31.3 mmole) in 270 ml Et₂0 is added dropwise over a period of one hour. After 10 additional minutes, the layers are separated. The ether layer is extracted with 150 ml 0.25 N HCl, and then with 200 ml brine. Each acueous layer is then backwashed successively with 100 ml Et₂O. The combined Et₂O layers are dried over an- hydrous MgSO₄, filtered, and concentrated under a N₂ stream. The crystalline residue is slurried in a small amount of ether, filtered, and the pale yellow crystals are dried under high vacuum to give 4.7g p-nitrobenzyloxycarbonylaminoethanethiol (65% yield).
NMR (CDCl₃), 8.18 (d, J=8Hz, aromatic protons ortho to nitro), 7.47 (d, J=8Hz, aromatic protons meta to nitro), 5.27 (-NH-) . 5.20 (s, -CH₂- Ø-pNO₂), 3.40 (m, -CH₂-NH-), 2.67 (m , -CH₂-SH) , 1.35 (t, J=8.5Hz, -SH) in ppm downfield from TMS.
IR (CHCl₃ solution) carbonyl-∼ 1725 cm⁻¹
M.S. - molecular ion-256, (M-47) at 209, (M-136) at 120, + CH₂øpNO₂ at 136.

### STEP C

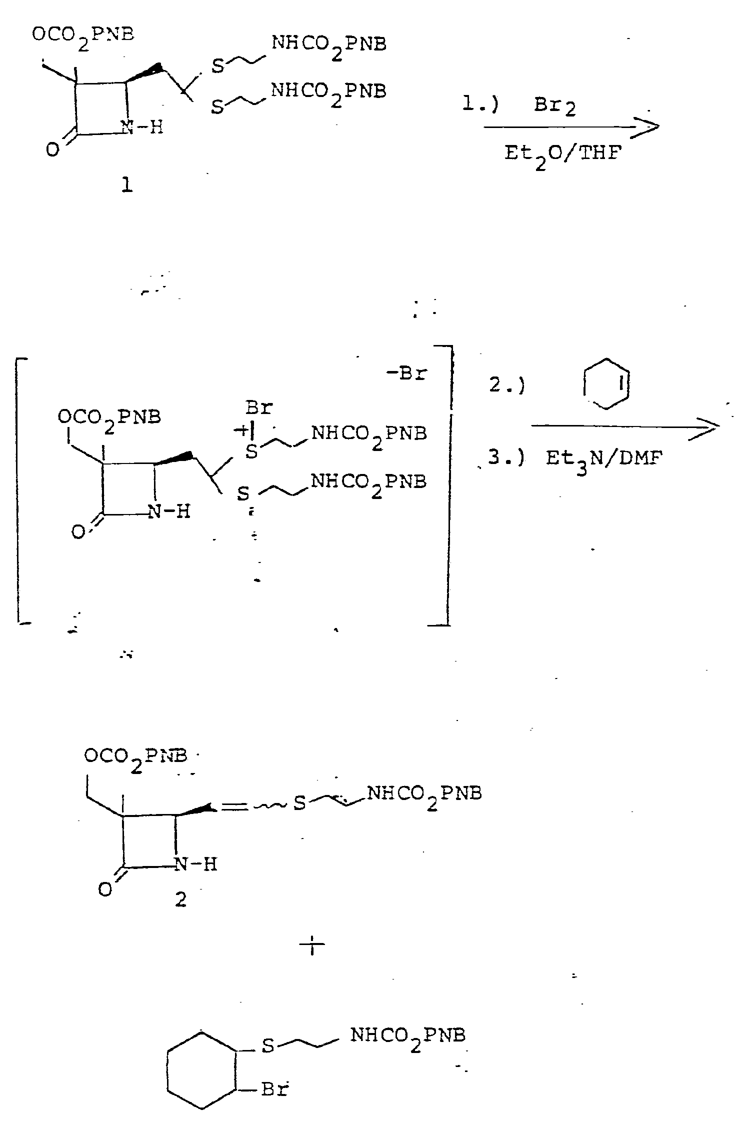

To 14.2 pentane (dried over 4A Linde molecular sieves) is added 0.5 ml Br₂ (mw = 160; 9.75 mmole). To 5g of 1 (mw = 830; 6.02 mmole) in 58 ml tetrahydrofuran (THF) (freshly distilled from lithium aluminum hydride) (LAH) and 65 ml Et₂0 (dried over 3A 1/16" Linde molecular sieves) at 0°C under N₂ with stirring is added dropwise 10 ml of the above 0.66M Br₂ solution (6.6 mmole). After 10 minutes at 0°C, 0.67 ml cyclohexene (mw = 82; ρ =0.81; 6.6 mmole) is added. After 5 minutes at 0°C, 1.7 ml triethylamine (MW=101;ρ =0.729; 12.3 mmole) is added immediately followed by 40 ml ice-cold dimethy Iforrmamide (DMF) (distilled from anhydrous CaSO₄ at 40 mm and stored over 4A Linde molecular sieves). The ice bath is removed, and stirring is continued for 2 1/4 hours at room temperature. The action mixture is'poured into a stirred ice-cold mix- wire of 12.6 ml 1MKH₂PO₄ 160 ml E₂O - 500 ml (EA). After separation of the layers, the aqueous one is saturated with sodium chloride and re-extracted with EA. The combined organic layers are extracted once with brine, dried over anhydrous MgSO4_{'} filtered and concentrated under a N₂ stream followed by pumping under high vacuum to provide crude 2.

The material is chromatographed on 250g silica gel (height = 45 cm; diameter = 4.5 cm) packed and applied in CHCl₃ and eluted with increasing percentages of MeOH in CHCl₃ (0-3% MeOH/CHCl₃) . Those fractions containing clean product are combined, concentrated under a N₂ stream, and pumped on high vacuum to give 2 Contaminated fractions are rechromatographed on silica gel using increasing percentages of EA in CHCl₃ (0-25% EA/CHCl₃) to give additional 2.

### STEP 3

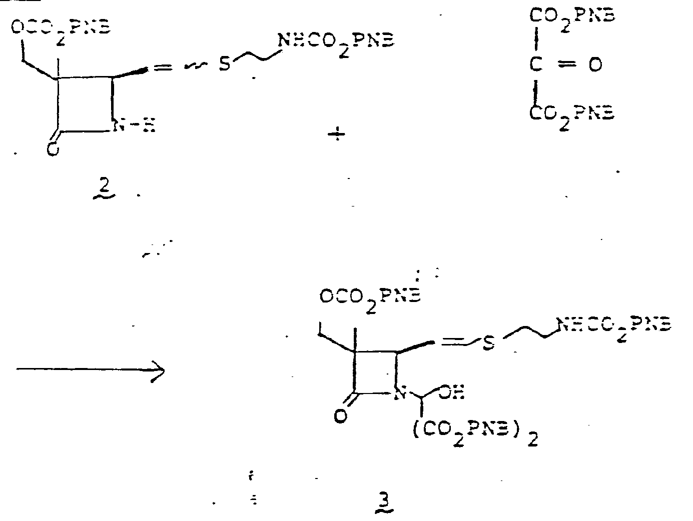

To a stirred solution of 2.48g di(p-nitrobenzyl) ketomalonate (from Example 12, Step E) (mw = 388; 6.39 mmole) in 400 ml hot anhydrous toluene is added a-solution of 2.52g of 2 (mw = '574; 4.39 mmole) in 20 ml THE (distilled from LAH) and 40 ml anhydrous toluene. After some of the solvent is boiled off, additional anhydrous toluene is added, and the azeodrying process is repeated three times. The solution is then refluxed under N₂ for 30 minutes. Additional toluene is then. allowed to boil off yet the volume is not allowed to diminish so much that precipitation occurs. Total heating time is approximately 2 1/2 hours. The clear yellow reaction mixture is removed from the oil bath and placed under a stream of N₂ which instantaneously causes clouding. After concentration to a yellow oil, the residue is dissolved in CH₂Cl_{2'} dried over anhydrous MgSO₄, filtered, and concentrated under a N₂ stream to cive crude 3.

The material is chromatographed on 250c silica çel packed and applied in CHCl₃ (height = 43 cm; diameter = 4.5 cm). Elution with 500 ml 0.5% MeOH/ CHCL₃ Is followed by continued elution with 1% MeOH/ CHCL₃ for the remainder of the chromatography. After the emergence of excess reagent, those fractions containing pure 3 are combined, concentrated under a N₂ stream and then on high vacuum to give 3.

Later fractions containing 3 and the corresponding cis thioenol-ether are re-chromatographed on silica gel to give additional 3.

### Step E

### Preparation of di-p-hitrobenzwl Ketomalonate

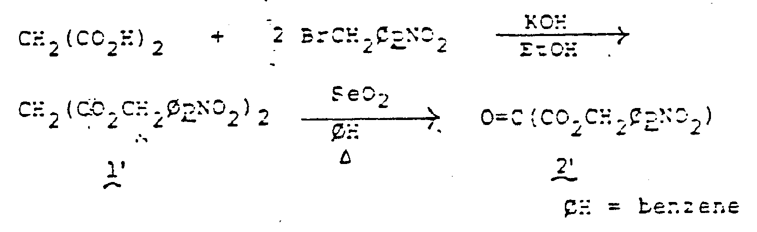

A mixture of 100 c p-nitrobenzyl broimide (0.46 mole), 28.6g malonic acid (0.275 mole) and 750 ml ethanol (EtOH) is stirred and warmed on the steam bath until solution is achieved. A solution of 33g KOH (>85% purity;~0.6 mole) in 200 ml of water is added carefully with swirling. An additional 200 ml of water is added, and the two-phase system is refluxed for 1.8 hours. The lighter color homogeneous solution is cooled in ice for 1 hour and the crude product isolated by filtration, washed twice with a minimum of cold EtOH, and dried by pulling dry N₂ through the cake; 33.7g of solid is obtained. If, during the refluxing stage the reaction mixture is slowly concentrating to ca. half volume by allowing refluxing solvent to distill off, the crude product yield rises to 77g. The material is recrystallized from methanol to give pure di-p-nitrobenzyl malonate 1'.

A mixture of 23.4 of 1', 10g Se0₂, and 30-40 ml of xylene is stirred in a flask immersed in an oil bath. The bath temperature is raised over 1 hour to 130-135°. A gradual darkening of the reaction mixture is noted, ar after a total of 4 hours at 130-135°, most of the insoluble residue is black Se°. The mixture is cooled, MgS0₄ is added to'remove the water, and Celite is added to aid in filtration. The mixture is filtered through Celite and the cake washed with xylene and a small por- tion of EtOAc. Final volume: 60 ml. A 100g column of Baker Silica Gel is prepared in benzene and 10 ml of ..iltrate applied, then eluted with increasing amounts of EtOAc in benzene, 500 ml fractions being collected. After one 2% ethyl acetate (EtOAc)/ØH, and two 10% EtOAc/ØH fractions, the third 10% and first 20% EtOAc/ ØH provide the bulk of the product (~1.6g from 10 ml filtrate) as judged by tlc (20% EtOAC/CHCl₃; silica gel GF). Recrystallization from benzene, (lg in ca. 50 ml concentrated to~1/3 volume and "spiked" with 1 ml of H₂0 saturated benzene): provides 0.24g 2'; mp(117) 121-122°.

### STEP F

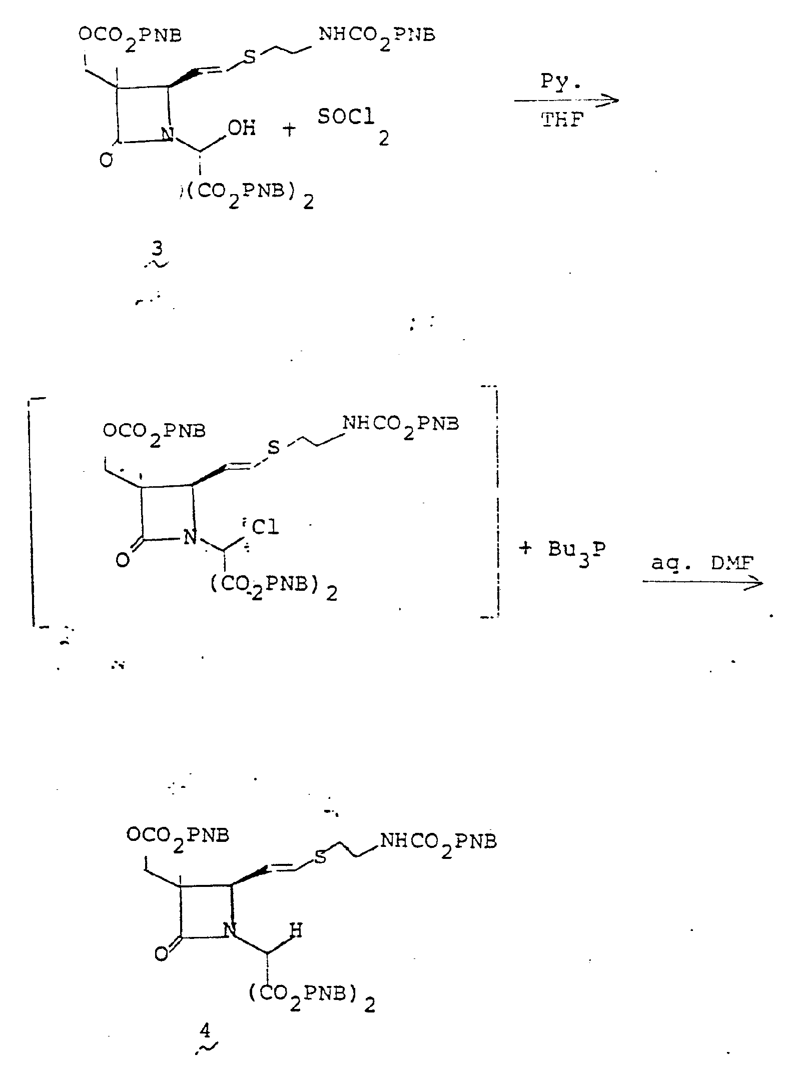

A solution of 1.468g of 3 (mw = 962; 1.53 mmole) in CH₂Cl₂ is dried over anhydrous MgS0₄, filtered, concentrated under a N₂ stream, and dried further under high vacuum just prior to the following reaction. To a solution of 3 in 24 ml THF (freshly distilled from LAH) at -20°C is added 0.206 ml anhydrous pyridine (mw = 79; ρ = .982; 2.56 mmole). With stirring under N₂, 294 mg of freshly distilled thionyl chloride (mw = 119; 2.47 mmole) in 5 ml THF is added dropwise. The reaction mixture is stirred for 10 minutes at -20°C., then 1/2 hour at 0°C and finally 1 hour at 25°C. The pyridine hydrochloride is filtered under N₂ and washed with 20 ml _{TH}F. The filtrate is concentrated under a N₂ stream followed by pumping on high vacuum. The resulting yellow foam is swirled in 25 ml anhydrous THF, and a small amount pf orange-red insoluble material is filtered off under N₂. The filtrate is reconcentrated as above to a yellow foam.

To this freshly prepared chloro compound is added with stirring a freshly shaken suspension of 678 mg tributylphosphine (mw = 202; 3.36 mmole) in 36.5 ml 9:1 DMF - H₂0 followed by 294 mg K₂HP0₄ (mw = 174; 1.69 mmole). The reaction mixture is stirred at 25°C., for 35 minutes. After dilution with 120 ml EA and 60 ml brine, the layers are separated, and the aqueous one is extracted two times with EA. The combined organic layers are washed one time with brine, dried over anhydrous MgS0₄, filtered and concentrated under a N₂ stream followed by pumping on high vacuum to give crude 4.

The material is chromatographed on lOOg silica gel (height = 28.5 cm; d = 4 cm) packed and applied in CHCl₃ and eluted with 0.5% MeOH in CHC1₃. Those fractions containing clean product are combined, concentrated under a N₂ stream and then on high vacuum to give 4. Contaminated fractions are re-chromatographed on silica gel thin layer plates (eluant = 50% acetone/ hexane; extraction of desired u.v. band with CHCl₃ and EA to provide additional 4.

### Step G

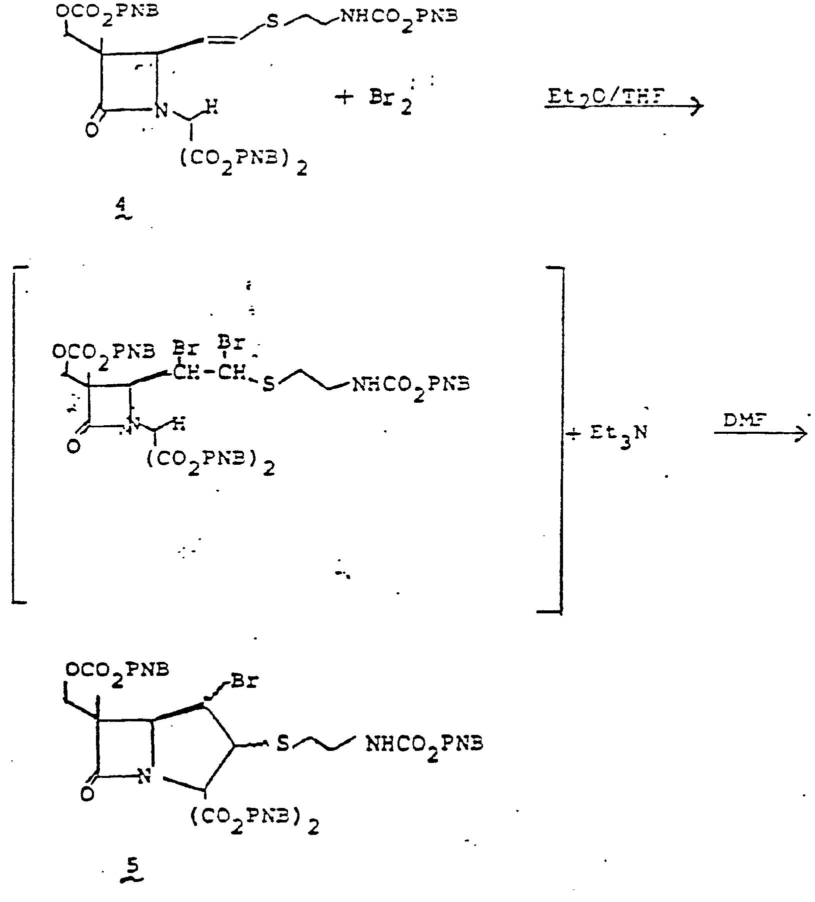

To 8.5 ml pentane (dried over 4A Linde molecular sieves) is added 0.2 ml Br₂ (mw = 160; 3.9 mmole). To 0.706g of 4 (mw = 946; 0.746 mmole) in 18 ml THF (freshly distilled from LAH) and 5.7 ml Et₂0 (dried over 3A 1/16" Linde molecular sieves) at 0°C under N₂ with stirring is added dropwise 1.8 ml of the above 0.45M Br2 solution (0.81 mmole). After 15 minutes at 0°C., 0.42 ml triethyl amine (mw = 101; ρ = 0.729; 3.03 mmole) is added immediately followed by 10.5 ml ice-cold DMF (distilled from CaS0₄ at 40 mm and stored over 4A Linde molecular sieves). The ice-bath is removed, and stirring at room temperature is continued for 2 hours. The reaction mixture is poured into a stirred ice-cold mixture of 3.1 ml 1M KH₂P0₄ - 70 ml H₂0 - 100 ml EA. The layers are separated, and the aqueous one is saturated with NaCl and re-extracted with EA. The combined organic layers are washed once with brine, dried over anhydrous MgS0₄, and filtered. The filtrate is concentrated under a N₂ stream and then pumped on high vacuum to give crude 5.

The material is chromatographed on 60g silica gel (diameter = 2.8 cm) packed and applied in CHC1₃ and is eluted with 100 ml-2% EA/CHCl₃; 100 ml-4% Ea/CHCl₃ and then 5% EA/CHC1₃ for the remainder of the chromato- ^{g}raphy. The fractions containing pure 5 are combined, concentrated under a N₂ stream, and pumped on high vacuum to give 5.

### Step H

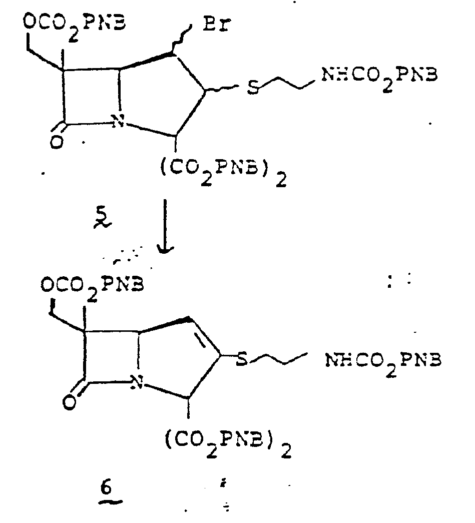

To 29 mg anhydrous silver fluoride (mw = 127; 0.23 mmole) is added a solution of 146 mg of 5 (mw = 1024; 0.14 mmole) in 3.5 ml anhydrous pyridine. The stoppered reaction mixture is stirred at room temperature in the dark for one hour and then poured into 20 ml cold water - 30 ml EA. After separation of the layers, the aqueous one is extracted two times with EA and one time with CHCl₃. Each organic layer is extracted one time with H₂0 and one time with brine. The combined organic layers are dried over anhydrous MgS0₄, filtered, and concentrated under a N₂ stream followed by pumping on high vacuum to give crude 6.

Preparative thin layer chromatography (eluant = 40% acetone/hexane; repeated extraction of desired u.v. band with a large volume of CHC1₃) yields slightly contaminated 6. Re-chromatographing on silica using EA in CHC13 as an eluting system gives 6.

### step I

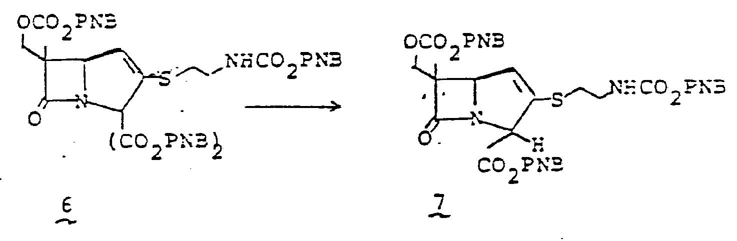

A solution of 77 mg of 6 (mw = 944; 0.082 mmole) in 0.9 ml S-collidine (distilled from powdered KOH ~ 30 mm pressure) is added to 13.4 mg anhydrous Lil (dried for few hours at 100°C over P₂0₅ under vacuum) (mw = 134; δ.1 mmole). With stirring under N₂, the reaction mixture is heated in an oil bath at 120°C. After a total of 30 minutes, the reaction mixture is cooled to 25°C.; diluted with CH₂Cl₂, and transferred to a round bottom flask for concentration under a N₂ stream and then on high vacuum. Partitioning the residue between EA-H₂0 and 1 ml 1M KH₂P0₄ is followed by extraction of the aqueous layer two additional times with EA and one time with CHCl₃. Each organic layer is then backwashed with brine. The combined organic layers are dried over anhydrous MgS0₄, filtered, concentrated under a N₂ stream and then on high vacuum to give crude 7.

Preparative thin layer chromatography on silica gel (plate is eluted two times with 40% acetone/ hexane; repeated extraction of the appropriate u.v. hands with large volume of CHCl₃) yields recovered starting material and 7.

### Step J

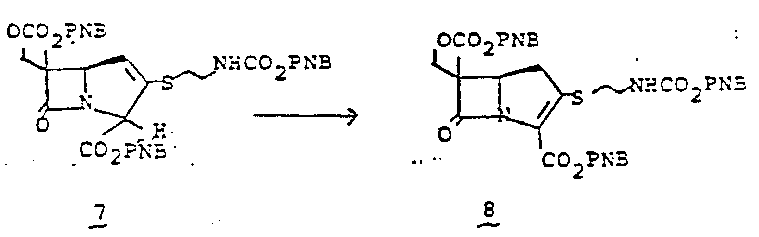

To 49 mg of 7 (mw = 765; 0.064 mmole) in 0.7 ml DMSO (distilled from CaH₂ at 8mm and stored over 4A Linde molecular sieves) is added 100 µl diisopropylamine (distilled from NaH under N₂ and stored over 4A Linde molecular sieves) (mw = 101; ρ =0.722; 0.71 mmole). The stoppered reaction mixture is stirred for a few minutes and then allowed to stand for 2 hours. 'he amine and most of the DMSO are then concentrated off under high vacuum with no external heating. The residue is passed quickly through a column of silica gel (packed, applied, and eluted with EA) to remove residual DMSO. After concentration under a N₂ stream of all fractions having u.v. absorbance, the material is chromatographed on a thin layer silica gel plate (eluant = 50% EA/CHCl₃; repeated extraction of desired u.v. bands with a large volume of chloroform). Recovered starting material is re-submitted to the reaction conditions and isolation procedure two more times to yield additional 8.

### Step K

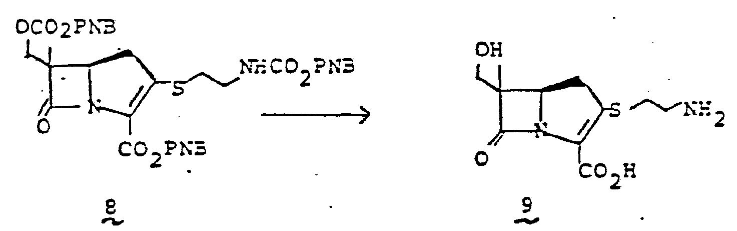

To 5.2 mg 8 is added 0.60 ml dioxane, 0.05 ml ethanol, 0.35 ml deionized water and 0.01 ml of 1.OM K₂HP0₄. To the resultant clear solution is added 5 mg of 10% Pd/C. The suspension is flushed with N₂, then 5-6 times alternately with 50 psi H₂ and vacuum. Finally, it is shaken under a 50 psi H₂ atmosphere for 30-40 min. After centrifugation, the Pd/C is washed and centrigufed 2-3X with 0.5 ml portions of deionized water. The combined centrifugates are extracted 5xl - 2 ml ether. Residual ether is removed under vacuum and the aqueous solution applied to an X_{A}D-2 column (20 x 140 mm). Fractions of 100 drops (6-7 ml) are collected, with continuous UV monitoring, by elution with deionized water. Emergence of strongly UV absorbing material begins around fractions 3-5 and is usually complete by fractions 25-30. Early fractions are examined by UV to exclude those few deemed too strongly absorbing in the 270-280 mµ. region. The remaining fractions are combined and lyophilized. The residue is evaluated by dissolving in 10.0 ml of deionized water and measuring the UVabsorption at 298 mµ indicating a 10-30% yield of desired product.

The following Example specifically illustrates a . preferred stereo-selective process embodiment of the present invention. As described above in detail, the starting material is a pure optical isomer of 4-vinyl-2-azetidinone (23, above).

### EXAMPLE 5 Preparation of (±)-9,9-dimethylthienamycin

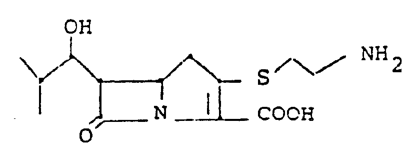

### Step A

### Preparation of 1-t-butylaimethylsilyl-3a-(1(R^{±}-)-hydroxy-2-methylpropyl)-4β-vinyl-2-azetidinone

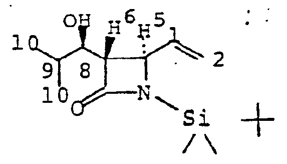

A solution of 1.226 g (5.8 m moles) of 1-t-butyldimethylsilyl-4-vinyl-2-azetidinone in 6 ml of anhydrous THF containing ~1 mg of 2,2'-dipyridyl and 0.075 ml (0.53 m moles) of diisopropyl amine is cooled under nitrogen to -78°C and treated with 2.5 ml of 2.3 M n-butyl lithium (5.75 m moles) with stirring over 5 minutes.

To the resultant orange solution is added 0.8 ml (0.63 g; 8.2 m moles)-of freshly distilled isobutyraldehyde over one minute to give a pale yellow solution. The cooling bath is removed and the mixture is allowed to stir for 15 minutes, and is then worked up by pouring into 60 ml of ice/water plus 10 ml 1 M KH₂P0₄, and extracting 4 times with 15 ml portions of ethyl acetate. The combined extracts are washed once with 0.2 M KH₂PO₄, once with saturated NaCl, dried with MgS04, and evaporated evaporated under a N₂ stream to give 1.74 g (1.67 g = 100%).of pale yellow oil. Examination by 300MHZ PMR shows three main components, identified as the 3β-(1R*) , 3z- (1S*),
and the desired 3c-(1R*) isomers in ca. 20, 60, and 20% yield respectively. If desired the product may be obtained at this point by chromatography on silica-cel using CH₂Cl₂ with low percentages of EtoAc. Because the product is crystalline, it can be isolated from enriched fractions even though not completely resolved. The 300 MHZ PMR (CDC1₃) shows (see numbering system above) 5.91 (H₁; ddd, J_{1,2 cis} =1OHz;J, _{2 trans}=l7Mz; J_{1,5}= 9Hz)., 5.21 (H_{2 cis}, d'J_{1,2 cis}= 10Hz) , 5.33 (H₂ trans' d, J_{1,2} trans 17Hz)_{'} 4.17- (H₅, dd, J_{1,5}=9HZ: J_{5,6}=3Hz) , 3.86-3.79 (H₈, m), 3.17 (H₆, dd, J_{5,6}=3 Hz; J₆,₈=3 Hz), 1.86 - 1.74 (Hg, m, J₅ ~ 6 Hz) , 0.96 (Si-t-butyl,s) , 0.81 (H_{10'}d,J_{9,10}=6 Hz), 0.25 and 0.17 (Si-CH₃.s) δ .

A preferred procedure entails oxidizing the initial aldol mix to the ketone and reducing with borohydride to give a mixture much enriched in the desired product.

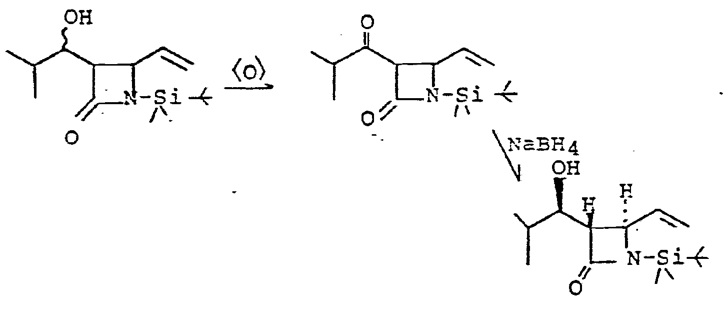

Thus the crude product above is taken up in 10 ml dry CH₂Cl₂ and cooled under N₂ in a -78°C bath. To 10 ml dry CH₂Cl₂ is added 0.86 ml (945 mg; 12.1 m mole) DMSO and the mix cooled to -65°C under N₂. After 10 minutes, 1.30 ml (1.94 g, 9.2 m moles) of trifluoroacetic anhydride is added and the mixture stirred for 15 minutes at -65°C. To this clear solution is added the chilled aldol solution by siphoning. The mixture is stirred for 1 hour .at -65°_{C}, treated with 2.7 ml (1.98 g; 19.5 m moles) of Et₃N, the cooling bath is removed and stirring is continued for 1 hour. The mix is then washed four times with water, once with saturated NaCl, and dried with MgS0₄: upon removing the solvent under a N₂ stream, 1.2 g of an orange oil is obtained. Examination by tlc (silica- ^{g}el; CH₂Cl₂). shcws the material to be overwhelmingly one component. If desired, further purification can be effected by chromatography on silica-gel, eluting with CH₂Cl₂. A 300MHz PMR spectrum (CDC1₃) shows 5.89 (H₁, ddd J_{1,2} =l0Hz; J _{2 trans}=l7Hz; J_{1,5}=9Hz) , 5.26 (H_{2cis}, d, J_{1,2cis} (H_{2 trans'} d,J_{1,2}= l7Hz) , 4.41 (H₅, dd, J_{1,5}=9Hz; J_{5,6}=2Hz) , 4.18 (H₆, d, J_{5,6}=3Hz) , 2.75-2.90 (H₉, m, J₉,₁₀=7Hz), 1.16 (H₁₀, d, J_{9,10}= 7Hz) 0.97 (Si-t-butyl, s), 0.17 and 0.25 (Si-CH₃, s)δ . More commonly, the reduction is carried out on crude ketone which has been freed of the most contaminants by filtration through silica-gel with CH₂Cl₂.

Thus, 63.85 g crude ketone is passed through 76 g Baker silica-^{g}el and washed on through with a total--of 2 liters of CH₂Cl₂ to give, upon evaporation 61.64 g (219 m mole) material which is taken up in 480 ml isopropyl alcohol and cooled with stirring in an ice/saturated NaCl bath for 30 minutes. To this is added 10.2 g NaBH4 (268 m moles) and the cloudy suspension stirred for 2 hours with continued cooling. It is then (cautiously) poured, with continued swirling, into 2 liters of ice/water containing 150 g KH₂P0₄ (1.08 moles). The mix is extracted five times with 100 ml CH₂Cl₂, the combined extracts are washed once with water, once with saturated NaCl, dried with MgSO₄ and evaporated to an oil. Examination by PMR shows no significant 3S-isomer; the 3a-(1S*) and 3a-(1R*) isomers are present-in 45-40% and 55-60% yield re_{'}spectively. By concentrating in petroleum ether to ~ 200 ml and seeding, 13 g of crystalline product is obtained directly; a second crop of 10 g is obtained upon further concentration. The mother liquors may be further processed using a preparative HPLC apparatus using 6% EtOAc/CH₂Cl₂.

When product can no longer be isolated, the combined mother liquors may be recycled through the oxidation- reduction sequence to generate more.

### Step B

### Preparation of 1-t--butydimethylsilyl-3z-(1 (R*)-o-nitrobenzylcarbonyldioxy-2-methylpropyl)-46-vinyl-2-azetidinone

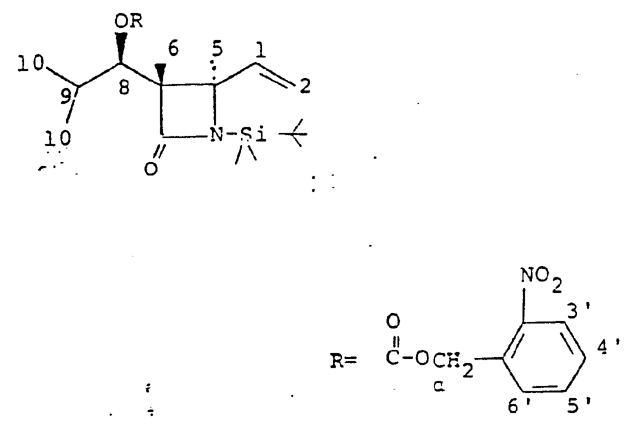

To a mixture of 1.7 g of 1-t-butyldimethylsilyl-3a-('l(R*)-hydroxy-2-methylpropyl)-4β-vinyl-2-azetidinone (6.0 mmoles) and 1.6 g of 4-dimethylaminopyridine (13.1 mmole) in 20 ml of sieve dried CH₂Cl_{2'} stirring in an ice bath under .a nitrogen atmospheres is added 2.9 g of o-nitrobenzyloxycarbonyl chloride (13.4 mmole) in 5 ml of sieve dried CH₂Cl₂. The initially slightly milky solution turns within minutes to a thick paste. The bath is removed and the mix allowed to stir over night. The mix is then stirred with 20 ml ice/water for several minutes affording two clear phases. The organic phase is removed and washed twice with water, dried with MgS0₄; concentrated to ~5 ml and passed through 5 g of Baker Silica followed by 200 ml of CH₂Cl₂, The entire filtrate is blown down with nitrogen to yield 3.1 g gum. Chromatography was carried out with a Waters Prep 500 Preparative HPLC apparatus, using one 500 ml pack and eluting with 3% EtOAc in CH₂Cl₂. After two column volumes, product emerges in ca. three column volumes; after another five column volumes, starting aldol begins to emerge and is more quickly eluted by changing to 7.5% EtOAc in CH₂Cl₂ and finally straight EtOAc. One obtains 0.97 g recovered starting material and 1.5 g of product which exhibits a 300 MHz PMR(CDCl₃) with peaks at 8.34 (H₃, d, J_{3,4},=8Hz) ,7.74-7.62 (H₅,₋₆,M) 7.52(H_{4'} dd J_{3',4'}=8Hz, J_{4',5},=8Hz), 5.88 (H₁, ddd,J_{1,2cis} =9Hz; J_{1,2trans}=l7Hz; J_{1,5}=9Hz), 5.62(H_{c},s)r 5.29 (H₂ₜᵣₐₙₛ,d,J_{1,2trans}= l7Hz), 5.21(H_{2cis'}d_{'}J_{1,2cis}=9Hz),5.07(H₈,dd,J_{6,8}=6Hz; J_{8,9}= 5Hz), 4.13(H₅,dd,J_{1,5} =9Hz; J_{5,6}=3HZ), 3.28 (H₆,dd,J_{5,6}= ^{3Hz;J}6, 8=5Hz) Z.18-Z.0Z(H9' ₘ), 0.95-0.85 (H₁₀ and Si-t-butyl), 0.24 and 0.15 (Si-CH_{3'}s)δ On tlc(silica gel GF, 3%BtOAC/CH₂Cl₂) product R_{f} was ~0.4, starting material ~ 0.15.

### Step C

### Preparation of 3a-(1(R*)-o-nitrobenzylcarbonyl- dioxy-2-methylpropyl)-4β-vinyl-2-azetidinone

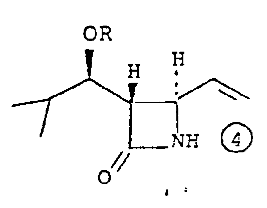

To 50.3 g of 1-t-butyldimethylsilyl-3a-(1 (R*) -o-nitrobenzylcarbonyldioxy-2-methylpropyl)-4β-vinyl-2-azetidinone (109 mmoles) in 480 ml of methanol is added 4 ml of concentrated HCl (48 mmoles) and the mix is stirred for four hours, poured into 4 liters of water plus 50 ml of 1M K₂HPO₄ and extracted with EtOAc several times. An insoluble interface solid, isolated by filtration, proves to be 9.8 g of product. The combined extracts are washed once with water, once with saturated Na_{c}l solution, dried and evaporated to give 24.7 g of solids. This is combined with the interface solid and recrystallized from acetone/ether to give 29.7 g product in three crops. Pure material has mp. 137.5-139°C. The 60 MHz PMR (CDCl3) shows 8.15(H₃,d,J₃, _{,4},=7Hz) , 7.75-7.35(H₄,₋₆, _{,}m) , 6.25-5.00 (H_{1-2,8,}complex) , 6.18 (H₄,bs), 5.30 (Hₐ , s) , 4.21 (H₅,dd,J_{1,5}=7Hz; J_{5,6}=2Hz), 3.22(H₆,dd,J_{5,6} =2Hz;J_{6,8 =} 6_{H}z), 1.7-2.5 (H₉,m) , 0.9 & 1.0(H_{10,s}) , δ .

### Step D

### Preparation of 3α-(1(R*)-o-nitrobenzylcarbonyl- dioxy-3-methylpropyl)-4B-[1-bromo-2-(2-p-nitro- benzyloxycarbonylaminoethylthio)ethyl]-2-azetidinone

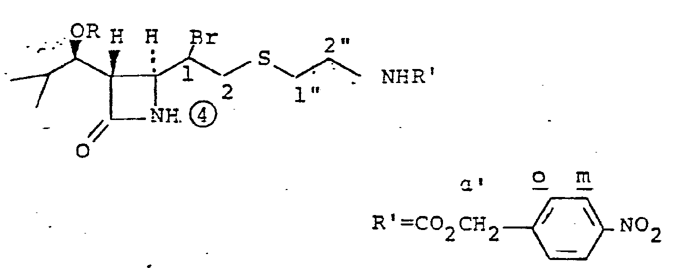

A solution of 254 mg of p-nitrobenzyloxycarbamido- ethyldisulfide (0.5 mmole) in 3 ml of THF (freshly distilled from LiAlH₄) is cooled under a nitrogen atmosphere to -40°C. and treated dropwise with 80 mg of Br₂ (0.5 mmole) in 0.5 ml of CC1₄. After stirring for ca. 10 minutes, a solution of 349 mg (1.0 mmole) of 3c-(1 (R*) -o-nitrobenzylcarbonyldi- oxy-2-methylpropyl)-4β-vinyl-2-azetidinone in ~3 ml of methanol free CHCl₃ (incompletely soluble) is added and rinsed in with additional dry THF. The color of the solution lightens considerably, and it is allowed to stir 2 hours without maintaining the cooling bath. At this point the mix has lightened further and the temperature is ca. -10°C. It is poured into ~ 50 ml ice/water, shaken vigorously and the phases separated. The aqueous phase is then extracted three more times with CHCl₃ the combined extracts washed once with water, once with saturated NaCl solution, dried with MgSO₄, and evaporated under a nitrogen stream to give 0.71 g of gum. Examination by tlc (silica; 20%EtOAc/ CH₂Cl₂) shows some unreacted starting materials plus two slower major product spots. The material is separated using the Waters Autoprep 500 with one 500 ml pack and eluting with 20% EtOAc in CH₂Cl₂. The starting materials emerge with the 3rd and 4th column volumes, the faster bromo-product(105 mg) emerges in the 5th and 6th column volumes and the slower bromo-product (282 mg) emerges in the 8th and 9th column volumes.

The faster bromo isomer has a 300 MHz PMR spectrum (CDCl₃) showing 8.25 (Hₘ,d,J_{o,m}=8Hz) , 8.18 (H₃' , d, J_{3',4'}=8Hz) , 7.8-7.5(H_{4'-6',} m) , 7.55(H₂,d,J_{o,m}=8Hz) , 6.26(H₄,s), 5.70&5.62(H_{α}, pair of doublets, J_{ac}=14 Hz), 5.23(H_{α,}s), 5.06(H₈,dd,J_{8,9}=4Hz ; J_{6,8}=8Hz) , 4.17 (H₁ , ddd , J₁, ₂ₓ=7Hz ; J_{1,2y}=7Hz ; J_{1,5}=7Hz), 3.54-3.30(H_{2"},m), 3.10-2.94(H₂,m), 2.76(H_{1"},t, J_{1" ,2"}=6Hz),2.32-2.14 (H₉,m ), 1.0 and 0.98(H₁₀, pair of doublets, J₉,₁₀=6Hz) , δ .

The slower bromo isomer has a 300 MHz PMR spectrum (CDCl₃) showing 8.25(Hₘ,d,J_{o,m} ⁼ 8Hz), 8.17 (H₃, _{,}d,J₃,₄,=8Hz) , 7.8-7.5(H₄,₋₆, _{,m}) , 7.55 (Hₒ,d,J_{o,m}=8Hz) 6.58 (H₄,s), 5.57(H_{c},s), 5.19 (H_{α}, s), 5.05(H₈,dd,J_{8,9}=5Hz; J_{6 ,8}=8,5Hz), 4.12(H₁,ddd,J_{1,2} =5Hz; J_{1,2y}=7.5Hz; J_{1,5}=7Hz), 3.54-3.34 (J_{2",}m), 3.18(H_{2,} dd,J_{2,2y}=14Hz; J_{1,2}=5Hz), 3.04 (H_{2y},dd,J_{1,2y}=7,5Hz; J_{2,2y}=14Hz). 2.74 (H_{1",} t,J_{1",2"}=6Hz) 2.32-2.18 (H₉,m) , 1.4 & 0.98(H₁₀, bs) δ.

In most cases the reaction is more complete than in this example. The ratios of fast to slow isomer appear variable and may even change on handling or standing; however, in the following reaction, the yields and ratios of cis to trans olefins obtained are substantially the same, regardless of which isomer is used.

### Step E

### Preparation of 3α-(1(R*)-o-nitrobenzylcarbonyldioxy-2-methylpropyl)-4β-[2-(2-p-nitrobenzyloxycarbonyl- aminoethylthio)vinyl]-2-azetidinone

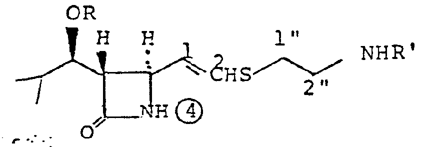

The fast bromo isomer from Step D, 105 mg. (0.154 mmole) in 1 ml. KOH dried pyridine was treated with 49 mg. AgF (0.39 mmole) and stirred for 2 hours under N₂, the mix poured into 10 ml. ice/water plus 1-2 ml. EtOAc, shaken vigorously and centrifuged. The organic phase is removed and the aqueous phase extracted three more times with 1-2 ml. EtOAc. The combined extracts are washed twice'with water, once with saturated NaCl solution, dried with MgSO₄ and evaporated under a nitrogen stream. The residue is flushed twice by evaporating from CHCl3 solution under high vacuum to give 58 mg. dark gum. Preparative tlc on silica with 20% EtOAc/CH₂Cl₂ gives 27 mg. of trans olefin and 14 mg. of cis olefin. (In larger runs, using somewhat lower ratios of AgF to substrate, the ratio of trans to cis appears somewhat higher.) The pure trans isomer has a 300 MHz PMR spectrum (CDC1₃) which exhibits peaks at 8.²² (Hₘ, d, J_{o,m} ⁼ 8Hz), 8.19 (H₃ , d, J_{3',4'} = ⁸H^{z}), 7.80-7.77 (H₃,_{-5'} ,m) 7.56 (J_{o,m} = 8Hz), 6.31 (H_{2'} d,J_{1,2} =14Hz) , ^{5.94} (H_{4,} s) , 5,71 (H₁, dd, J_{1,2} = 14 Hz, J_{1,5} = ^{8Hz}), ^{5.58} and 5.66 (H_{α}, two doublets, J_{αα} = 14) , 5.23 (H_{α}' , s) , 5.10 (H₈ , dd, J_{6,} 8=6Hz ; J_{8,9} ^{= 5}Hz), ⁴.3⁰ (H₅, ^{dd,} J_{1,5} = 8Hz ; J_{5,6} = ^{2Hz}), ^{3.47} (H_{2"}, dd, J_{1",2"} = 6Hz; J_{2" ,NH} = 14Hz), 3.28 (H₆, ^{dd,} J_{5,6} = 2Hz; J_{6,8} = ⁶H^{z}), 2.90 (H_{1"} , t, J_{1",2"} = 6Hz), 2.21 - 2.03 (H₉,m), 0.97 and 0.95 (H₁₀, two doublets, J_{9,10} = 6Hz) δ. The cis isomer has a 300 MHz PMR spectrum (CDCl₃) with peaks at 8.24 (Hₘ, d, J _{o,m} ^{= 8Hz}), 8.17 (H₃, , d, J₃ , _{,4'} = 8Hz), 7.76 - 7.49 (H_{4'-6'}m), ^{7.53} (Hₒ d,J_{o,m} = 8Hz), ^{6.}19 (H₂, d, J_{1,2} ⁼ 10Hz), 5.9⁵ (H₄, s) , 5.74 - 5.54 (H₁ and H_{α}, complex), 5.20 (H_{α}, s) , 5.11 (H₈, dd, J_{6,8} = 6Hz ; J_{8,9}=6Hz), 4.⁵⁷ (H₅,dd, J_{1,5}= ^{9Hz,} J_{5,6} = 2Hz), 3.^{49 -} 3.37 (H_{2" ,}m), 3.33 (H₆, ^{dd,} J_{5,6} = 2Hz ; J_{6,8} = 6Hz), 2.89 - 2.81 -(H_{1",}m), 2.3 - 2.1 (H₉, m) , ~1.0 (H₁₀,bs) δ.

### Step F

### Preparation of 1-bis carbo-p-nitrobenzyloxy- hydroxymethyl-3α(1-(R*)-o-nitrobenzylcarbonyl- dioxy-2-methylpropyl)-4β-[2-(2-p-nitrobenzyloxy- carbonylaminoethylthio)vinyl]-2-azetidinone

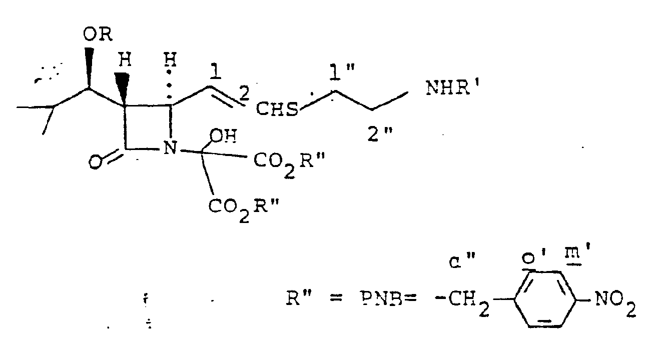

The procedure used in Example 31a, Step F, is followed except that 588 mg. (1.31 mmole) of ketomalonate, 25 ml. toluene, 605 mg. (1.0 mmoles) of 3α-(1-(R*)-o-nitrobenzylcarbonyldioxy-2-methylpropyl)-43-[2-(2-p-nitrobenzyloxycarbonylamino- ethylthio)vinyl]-2-azetidinone in 4 ml. THF, distillation of 18 ml. toluene and 1.5 hours of refluxing are employed. The analytical tlc's are developed three times in 1:2 acetone/hexane for optimum resolution. Chromatography on 30 g. silica gel packed in 1:2 acetone/hexane and collecting 25 ml. fractions after a 150 ml. forerun fraction gives 265 mg. ketomalonic acid (fractions 4-8), 177 mo. starting azetidinone (fractions 9-11), and 706 mg. of product (fractions 12-20). The 60 MHz PMR (CDCl₃) shows peaks at 8.24 - 7.95 (H_{3' ,m,m',}m) , 7.68 - ^{7.38} (H_{o,o' ,4'-6',} m) , 6.26 (H₁, d,J_{1,2} = 15 Hz), 5.65 (H₂, dd,J_{1,2} ^{= 15} Hz; J_{1,5} =8Hz) , 5.48 (H_{α},S) , 5.3⁰ (H_{α"},s) , 5.12 (H_{α'}, s) ~5.12 - 4.9 (H₈, partially buried under α'), ^{4.7} (H₅, dd, J_{1,5} ⁼ 8Hz,:J_{5,6} = 2Hz), 3.58 - 3.12 (H_{2" ,6,} m), 3.0 - 2.55 (H_{1" ,} m), 2.3 - 1.7 (H₉, m) , 0.95 and 0.83 (H₁₀, two bs) δ .

### Step G

### Preparation of 1-biscarbo-p-nitrobenzyloxymethyl-3α-(1-(R*)-o-nitrobenzylcarbonyldioxy-2-methylpropyl)-4β-[2-(2-p-nitrobenzyloxycarbonylaminoethylthio)vinyl]-2-azetidinone

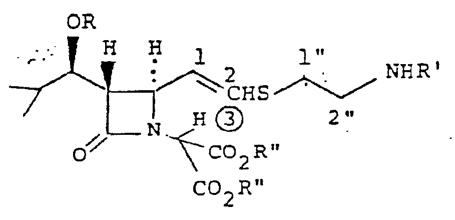

If 706 mg. (0.71 mmole) of 1-biscarbo-p-nitrobenzyloxyhydroxymethyl-3α-(1-(R*)-o-nitrobenzyl- carbonyldioxy-2-methylpropyl)-4β-[2-(2-p-nitro- benzyloxycarbonylaminoethylthio)vinyl]-2-azetidinone in 7 ml. THF containing 100λ pyridine (98 mg.; 1.24 mmole) is treated substantially as in Example ₃₂, Step G, with 86λ SOCl₂ (144 mg.-; 1.21 mmole) are subsequently with 3-53λ (n-but) ₃P (1.42 mmole) in 7.2 ml. sieve dried DMF with 167 mg. K₂HPO₄ (0.96 mmole) and worked up as above, the crude title compound is obtained. Chromatography on 50 g. silica gel packed in CH₂Cl₂ and developed with 5% EtOAc/CH₂Cl₂, collecting a liter fraction after discarding a 125 ml. forerun; elution with 10% EtOAc/CH₂Cl₂, collecting 25 ml. fractions, gives product in fractions 10-30. Further purification on preparative tlc with 10% EtOAc/CH₂Cl₂ gives a 40-50% yield of product which displays a 60 MHz PMR(CDCl₃) with peaks at ^{8.2} (H,_{3,m,m',} b_{d}, J = 7-8Hz), ^{7.75 - 7.35 (H}4'-6',o, o' ' m) , 6.3 (H₂, d, J_{1,2} ^{= 15}H^{z}), 5.6(H₁, dd, J_{1,2} ⁼ 15 Hz; J_{1,5} ⁼ 9 Hz), 5.58 (H_{α},s) , 5.44-5.18 (H_{α,α",3,} complex), 5.35 - 5.0 (H₈, m) , 4.60 (H₅, dd, J_{5,6} = 2Hz; J_{1,3} = 9 Hz), 3.6-3.2 (H_{2", 6},m) , 2.65-3.05 (H_{1"} , m) , 2.4 - 1.8 (H₉ , m) , 1.02 and 0.95 (H₁₀ , two singlets ) δ .

### Step H

### Preparation of 2,2-biscarbo-n-nitrobenzyloxy-3- (2-p-nitrobenzyloxycarbonylaminoethylthio)-4-bromo-6-exo-(1-(R*)-o-nitrobenzylcarbonyldioxy-2- methylpronyl)-1-azabicyclo[3.2.0]heptan-7-one

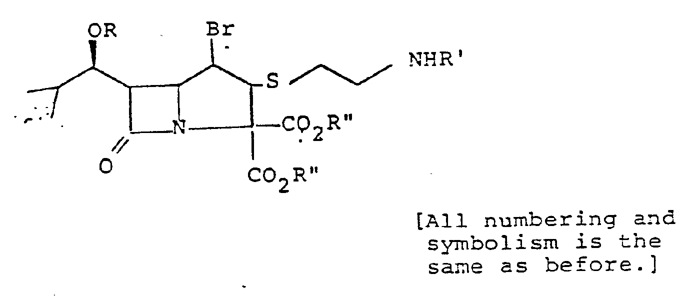

Following the procedure of Example 31a, Step H, and adding 0.20 ml of 132 mg Br/1.3 ml CCl₄ (0.125 mmoles) to 98 mg (0.10 mmoles) of 1-biscarbo-p-nitrobenzyloxymethyl-3α-(1(R*)-o-nitrobenzylcarbonyldioxy-2-methylpropyl)-4β-[2-(2-p-nitrobenzyloxycarbonylaminoethylthio)-vinyl]-2-azetidinone in 2 ml THF, and then using 40λEt₃N-(0.54 mmoles) and 1.5 ml DMF as in Example 32, Step H, there is obtained, upon work-up, 111 mg crude product. Preparative tlc using 77% EtOAc/CH₂Cl₂ affords the product, 48 mg, the slowest of the 3-4 major bands observed. The 60 MHz PMR (CDC1₃) shows peaks at 8.15 - 8.0 and 7.⁷ - ^{7.4} (H_{aromatics}; clusters of multiplets), 5.60(H_{α}, s), 5.34(H_{α"} , s) , 5.20(H_{α'} , s), 4.8 - 5.1 (H₈, complex), 4.45 - 4.15 (H₅, m), 4.70(H₁, dd,J_{1,2}=6; J_{1,5}=6) , 3.7 - 3.25 (H_{2,2"} ,m) , 2.65 - 3.00(H_{2"} , m), 2.35 - 1.6 (H₉,bm), 0.98 and 1.1 (H₁₀, two singlets) δ.

### Step I

### Preparation of 2,2-biscarbo-p-nitrobenzyloxy-3-(2-p-nitrobenzyloxycarbonylaminoethylthio)-6-exo-(1-(R*)-o-nitrobenzylcarbonyldioxy-2-methylpropyl)-1-azabicyclo[3.2.0]hept-3-en-7-one.

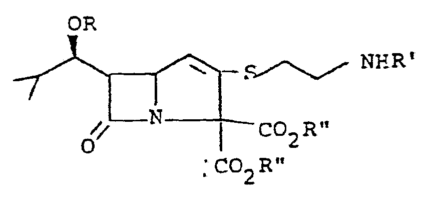

Following the procedure of Example 32, Step I, using 48 mg (0.046 mmole) of 2-biscarbo-p-nitrobenzyloxy-3-(2-p-nitrobenzyloxycarbonylaminoethylthio)-4-bromo-6-exo-(1-(R*)-o-nitrobenzylcarbonyldioxy-2-methylpropyl)-1-azabicyclo [3.2.0] heptan-7-one in 10 ml pyridine with 15 mg _{A}gF (0.12 mmole) but working up by pouring into H₂O/NaCl and extracting only with EtOAc,crude title compound is obtained. Preparative tlc with 8% EtOAc/CH₂Cl₂ affords 38 mg clean product with a 60 MHzPMR(CDCl₃) spectrum showing peaks at 8.25-8.02(H_{3',m,m',}multiplet), 7.7-7.4 (H_{4'-6',0,0,}m) , 6.23(H₁,d,J_{1,5}=1Hz) 5.80 and 5.45(H , two doublets, J_{αα}=14Hz) , 5.35 (H_{α",} s), 5.20 (H_{α',} s) , 5.3-5.0 (H₈,m,partially buried under H_{α'), 4.8}(_{HS},bm), 3.6-3.25(H₂n_{, 6 ,} m) 3.2-2.9(H₁n_{,}m) , ₂.4-1.9(H₉,bm), 1.05 and 0.95-(H_{10,} two singlets) δ .

### Step J

### Preparation of 2-carbo-p-nitrobenzyloxy-3- (2-p-nitrobenzyloxycarbonylaminoethylthio)-6-exo-(1(R^{*})-o-nitrobenzylcarbonyldioxy-2-methylpropyl)-1-azabicyclo[3.2.0]hept-3-en-7-one

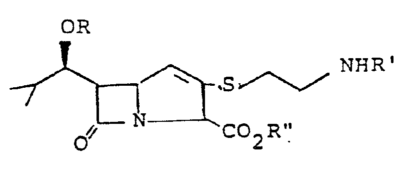

The procedure of Example 31a, Step J, is followed except: 491 mg (0.51 mmoles) of 2-bis carbo-p-n-trobenzyloxy-3-(2-p-nitrobenzyloxycar- bonylaminoethylthio)-6-exo-(1-(R*)-o-nitrobenzyl- carbonyldioxy-2-methylpropyl)-1-azabicyclo[3.2.0]-hept-3-en-7-one, 6 ml collidine, 87 mg (0.65 mmole) LiI and the crude product is purified by tlc with 40% acetone/hexane, the title compound is obtained which gives a 300 MHz PMR spectrum (CDCl₃) with peaks at 8.15 (H_{3' ,m,m',} d.J=8HZ) , 7.66 - 7.²² (H_{4'-6',2,2',}m) , 5.86(Hₗ,ₛ), 5.68 and 5.55(H two doublets, J_{αα} =14Hz), 5.13(Hα_{",} s) , 5.05(H_{α',3,} bs) , 4.96(H₈,dd,J_{8,9}=4Hz; J_{6,8}=7Hz) , 4.54 (H₅,6s) , 3.3-3.6(H_{2",}m) , 3.38 (H_{6,} dd,J_{5,6}=2Hz; J_{6,8}=7Hz), 2.90-3.11 (H_{1",} m), 1.86-2.04 (H₉,bm) , ~1.0(H_{10,} off scale) δ .

### Step K

### Preparation of 2-carbo-p-nitrobenzyloxy-3-(2-p-nitrobenzyloxycarbonylaminoethylthio)-6- exo-(l-(R*)-hydroxy-2-methylpropyl)-l-azabicyclo-[3,2,0]hept-3-en-7one

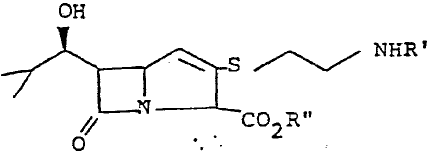

A solution of ca. 130 gm of 2-carbo-p-nitrobenzyloxy-3-(2-p-nitrobenzyloxycarbonylaminoethylthio)-6-exo-(1-(R*)-o-nitrobenzylcarbonyldioxy-2-methylpropyl)-1-azabicyclo [3,2,0] hept-3-en-7-one in 10 ml of CHCl₃ is placed in a quartz vessel (~ 1x1x10cm) equipped with a 14/20 standard taper mouth and jacketed by a larger quartz messel through which cold water may-be run.. A fine stream of nitrogen is blown through the solution for 10 minutes and the system is then closed with a nitrogen line fitted with a pressure-releasing bubbler. The water flow through the jacket is turned on and the vessel placed in a Rayonet Photolysis apparatus equipped with 3500Å sources. The photolyzer is turned on and the sample is irradiated for 3 hours. Upon evaporation of the solvent and preparative tlc, with an EtOAc/CH₂Cl₂Stytem, a 5-lQ_{%} yield of the title compound is obtained along with a 75-80% recovery of starting material which is resubjected to the photolysis. Overall, a 30-40% conversion is effected. The product has a 300 MHz PMR spectrum (CDC1₃) with peaks at 8.23 (Hₘ, m , , d, J_{o,m} = 8Hz), 7.53 and 7.51(Hₒ and Hₒ , doublets, J=8Hz), 6.01(H₁,s), 5.20 (H_{α",}s) , 5.17 (H_{α',3},bs) , 4.67 (H₅,bs) , 3.92(H_{8,} dd,J_{6,8}=6Hz ; J_{8,9}=6Hz) , 3.53-3.46 (H_{2" ,} bm) , 3.25 (H₆ ,dd,J_{5,6}=3Hz; J_{6,8}=6Hz) , 3.12-2.87 (H_{1 ",} bm), 1.94 - 1.78 (H_{9 '} bm), -0.97 (H₁₀ , bs) δ.

### 5tep L

### preparation of 2-carbo-p-nitrobenzyloxy-3-(2-p-nitrobenzyloxycarbonylaminoethylthio)-6-exo-(1-(R*)-hydroxy-2-methylpropyl)-1-azabicyclo[3.2.0]-he^{p}t-2-en -7-one

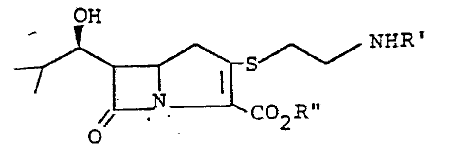

A solution of 9 mg(0.014 mmoles) of 2-carbo-p-nitro- benzyloxy-3-(2-p-nitrobenzyloxycarbonylaminoethylthio)-6-exo-(1- (R*)-hydroxy-2-methyl-propyl)-1-azabicyclo-[3.2.0]hept-3-en-7-one in 0.1 ml DMSO is treated witl 0.2-0.3λ(0.002 mmole) DBU. After standing for 1-2 hours, the solution is diluted with 1 ml of water and extracted several times with EtOAc.-The extracts are combined , washed with water, 1M KH₂PO₄,dried with MgSO₄ and concentrated. The concentrate is processed by preparative tlc using 1:1 CH₂Cl₂/EtOAc to give the starting compound and the title compound. The former may be recycled through

### Step M

### Preparation of (±)9,9-dimethylthienamycin

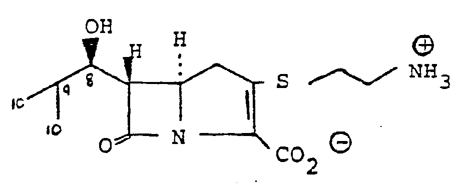

A 5-6 mg sample of 2-carbo-o-nitrobenzyloxy-3-(2-p-nitrobenzyloxycarbonylaminoethylthio)-6-exo-(1-(R*)-hydroxy-2-methylpropyl)-1-azabicyclo- [3,2,0]hept-2-en-7-one in a 13x100 mm culture tube is deblocked under precisely the conditions given in Example 12 Step K . The aqueous extract, however, is not applied to an XAD-2 column; instead it is injected in portions onto a Waters Associates

Microbondapak C₁₈ column ( 3ml void volume) and eluted with 5% THF in deionized water. The desired material appears as a major peak (254 photocell) at approximately 6 minutes with a flow rate of 0.7 ml/ minute. Collected peaks from multiple injections are combined and carefully concentrated on a rotary evaporator equipped with a Dry Ice/acetone charged cold finger condenser and attached to a high vacuum oil pump to 0.5-1.0 ml. The concentrate is shell frozen and placed in a shelf lyophilizer with T≤ -10°C overnight The web-like residue exhibits a 300 mHz PMR Spectrum (D₂0) with peaks at 4.27 (H₅,bt,J_{1,5}=8-10Hz) 3.83 (H₈,dd, J_{8,9}=5Hz ; J_{6,8}=5Hz) , 3.62(H₆,dd,J_{5,6}=3Hz ; J_{6,8}=4Hz) , 3.31-2.95(H_{1",2",} bm) 1.87-1.71 (H₉,bm) , 0.94 & 0.88 the isomerization process to increase the overall conversion to the letter. The product gives a 300 MHz PMR spectrum (CDCl₃) with peaks at 8.24 (H_{m,m',} d,J=8Hz) , 7.68(H_{o',} d,Jₒ,ₘ,=8Hz) , 7.52 (Hₒ,d,J_{o,m} =8Hz) , 5.53 and 5.25(_{H} pair of doublets J_{αα}=14Hz) , 5.20(H_{α',}s) , 4.38-4.28- (H₅m) , 3.94-3.88 (H₈,bm) , 3.51-3.42 (H_{2",} m) , 3.41-3.32(H₆, bdd), 3.20-2.94 (H_{1",1,}m) , 1.95-1.85 (H₉,bm) , ~ 0.97(H₁₀, not resolved ) δ. (H₁₀, two doublets, ^{J}9,10^{=6Hz})δ . There is no significant absortion below the HDO peak at 4.84 δ. The PMR sample has a U.V. spectrum with λmax. 299 mu, quenchable with hydroxylamine.

### EXAMPLE 5a

### Preparation of (±)-9-benzylthienamycin

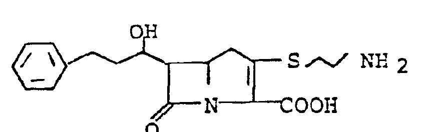

### Step A

### Preparation of 1-t-butyldimethylsilvl-3d-[(1(R*)-_ hydroxy-3-phenyl)propyl]-48-vinyl-2-azetidinone- and 1-t-butyldimethylsilyl-3c-[(1(S*)-hydroxy-3-phenyl)propyll-4∈-vinyl-2-azetidinone (1 and 2 respectively)

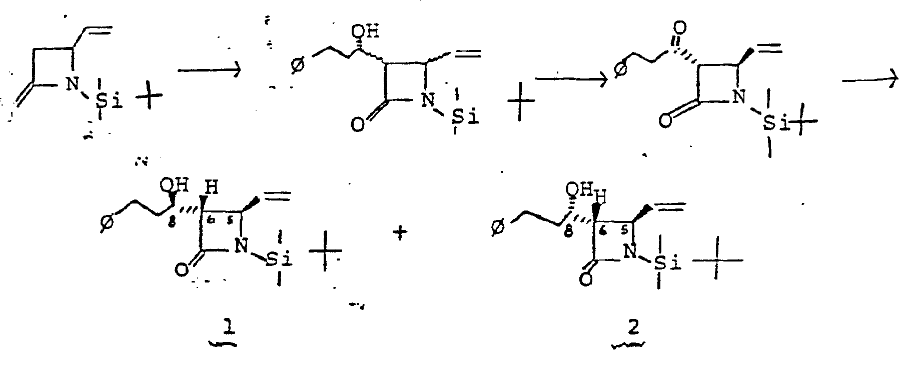

Generation of the anion of 17.8 g.of 1-t-butyldimethylsilyl-4-vinyl-2-azetidinone (mw=211; 84.4 mmole), as in Example 31, Step A, is followed by condensation with 12.6 g of hydrocinnamaldehyde (mw=₁₃₄; 94 mmole) rather than with isobutyraldehyde. Upon work-up, the crude aldol product is quickly chromatographed on silica gel (100 g). Elution with 5% EA/CH₂C1₂ provides 30° g of 1-t-butyldimethylsilyl-3-[(1-hydroxy-3-phenyl)propyl]-4-vinyl-2-azetidinone as a mixture of diastereomers.

NMR (300 MHz, d6-acetone)δ 0.14-0.22 (series of s, 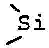 (CH₃) ₂'s) . 0.94, (s's, 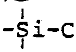 (CH₃)₃'s) , 1.76-2.10 (m, ØCH₂CH₂-), .2.59-2.89 (m, ØCH₂CH₂-), 3.00-3.06 (2dd, H₆ of (8R* & 8S*)-trans-β-lactams) 3.46 - (overlapping dd appearing as a t, J_{5,6}=J_{6,8}= 5Hz, H₆ .of cis-β-lactam) , 3.77-3.97 (m's, H₈ 's) , 4.06-4.23 (m's, H₅ 's), 5.13-5.38 & 5.92-6.29 (m's, -CH=CH₂),' 7.14-7.30(m, Ø).

A preferred procedure for isolation of the title compounds in quantity involves oxidation of the mixture of diastereomers to the all trans-ketone followed by reducing with NaBH₄ to a mixture from which thé desired (lR^{*} -hydroxy)diastereomer is more readily isolated.

Using essentially the same oxidation procedure as in Example 31, Step A, 30g of diastereomeric 1-t-butyldimethylsilyl-3-[(1-hydroxy-3-phenyl)propyl)-4-vinyl-2-azetidinone is oxidized to the corresponding trans-ketone (30 g crude material). Recrystallization from a small volume of Et₂0 gives 10.9 of crystalline product in two crops. The mother liquor is_{w} chromatographed on silica-gel (100 g; eluting with CH₂Cl₂). The fractions containing product are combined and washed with cold petroleum ether to give 2.73g additional crystalline ketone. A second recrystallization from Et ₂O gives an analytical sample: mp= 79.5-82.5°C; IR(CHCl₃)µ 5.76, 5.86; MS m/e 343 (M+), 328, 286;

NMR (300MHz, CDCl₃) δ 0.16 & 0.24 (2s, 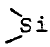 (CH₃) ₂) . 0.93 (s, 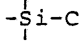 (CH₃)₃), 2.74-3.14 (m, ØCH₂CH₂) , 3.97 (d, J_{5, 6}=3Hz, H₆) , 4.44 (dd, J=3Hz & 8Hz, H5), 5.21-5.39 & 5.77-5.89 (m's, CH=CH₂), 7.19-7.31(m,Ø). Anal (C₂₀H₂₉NO₂Si)C, H, N.

With stirring, 3.4g of crystalline ketone (mw=343, 9.9 mmole) dissolved in 70 ml i-propanol is treated with 471mg NaBH₄ (mw=38; 12.4 mmole). After stirring under N₂ at room temperature for 0.5 to 2 hours the reaction mixture is poured (cautiously) into a mixture of 60 ml. 1MKH₂PO₄-ice water-CH₂Cl₂. Continuing the work-up as in Example 31, Step A, 3.5g crude product is isolated. Chromatography on silica-gel (100g, eluting with 5% EA/CH₂Cl₂) followed by chromatography on a Waters Associates Prep LC/System 500 instrument (10% acetone/hexane recycle mode) gives l.Og of 1-t-butyldimethylsilyl-3c-((1(S*) - hydroxy-3-phenyl)propyl]-4β-vinyl-2-azetidinone and l.lg of the desired 1-t-butyldimethylsilyl-3a-[(1(R*)-hydroxy-3-phenyl)propyl]-4β-vinyl-2- azetidinone diastereomer as a crystalline solid. Recrystallization from petroleum ether gives an analytical sample: mp=63-65°C; IR(CHCl₃) µ. 5.79 br; MS m/e 345 (M+), 288, 105, 91;

NMR(300MHz ,d6-acetone)δ0.14 & 0.21 (2s, 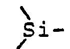 (CH₃) ₂) , 0.93 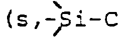 (CH3)3). 1.73-1.94(m,⌀CH2-CH2), 2.62-2.88(m,⌀CH₂CH₂), 3.02 (dd, J₅,₆=3Hz, J₆,₈=5Hz,H6) , 3.92-3.98 (m,H₈), 4.21 (dd,J=3Hz & 9Hz, H₅) . 5.14-5. 39 & 5.96-6.08 (m's, CH=CH₂), 7.14-7.30 (m,∅)

Anal (C₂₀H₃₁NO₂Si) C,H,N.

Data for 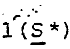 -hydroxy diastereomer: IR(CHCl₃)-5.80 br; MS m/e 345 (M+) , 288, 105, 91; NMR (300MHz, d₆-acetone) δ 0.13 & 0.20 (2s, 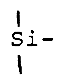 (CH₃), 0.93(s, 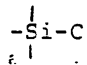 (CH₃)₃,) 1.80-2.10 (m,Ø-CH₂CH₂), 2.62-2.88 (m,ØCH₂CH2). 3.04 (^{dd,} J₅,₆= ³ Hz,-J_{6,8}=4. 5Hz,H₆) , 3. 83-3.90 (m,H₈) , 4.08 (dd, J=3Hz and 6Hz, H₅) , 5.13-5.35 & 5.92-6.06 (m's, CH=CH₂)-, 7.14-7.30 (m,Ø).

### Step B

### Preparation of 1-t-butyldimethylsilyl-3a-((1(R*)-o-nitrobenzylcarbonyldioxy-3-phenyl)propyl]-48-vinyl-2-azetidinone (3)

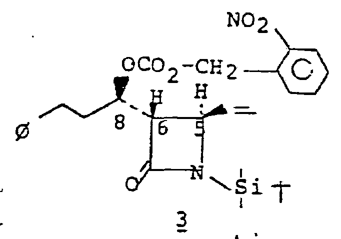

As in Example 31, Step B, 5.7 g of 1-t-butyldimethylsilyl-3=-[(1(R,*)-hydroxy-3-phenyl)propyl]-4:-vinyl-2- azetidinone (mw=345; 16.5 mmole) and 4.24 g 4-dimethylaminopyridine (mw=122, 34.8 mmole) in 100 ml CH₂Cl₂ is treated with 7.56^{f}g o-nitrobenzyloxycarbonylchloride (mw=217; 34.8 mmole) in 13 ml _{CH2C}1₂. The cooling bath is removed, and stirring is continued under N₂ for 4-hours at room temperature. The reaction mixture is poured into a mixture of 18 ml 1M K₂ HPO₄- water. Continuing the work-up as in Example 31, Step B, 11.7 g crude material is obtained. Chromatography on silica gel (350 g, eluting with 0→2% EA/CH₂Cl₂) gives 5.76 g of the title compound. Chromatography on silica gel (100 g, O→2% EA/CH₂Cl₂) of contaminated column fractions gives 1.36 g. additional product (82% total yield). IR (CHCl3); 5.75br; NMR (300MHz, CDCl₃) δ 0.15 & 0.24(2s Si- (CH3)2), 0.93(s, -Si-C(CH₃)₃), 2.06-2.14 (m,ØCH₂CH₂) , 2.60-2.78(m,ØCH₂CH₂), 3.22 (dd, J_{5,6}=3Hz,J_{6,8}=7Hz, ^{H}₆), 4.12(dd,J=3Hz & J=9Hz, H₅), 5.17-5.32 & 5.81-5,93 (m 's, H₈ & CH=CH₂), 5.60(mid-point of 2d, J=14Hz, nonequivalent-OCO₂CH₂-arom.)7.16-8.2(m,aromatics) .

### Step C

### Preparation of 3r-[(1(R*)-o-nitrobenzylcarbonyldi- oxy-3-phenyl)propyl]-4β-vinyl-2-azetidinone (4)

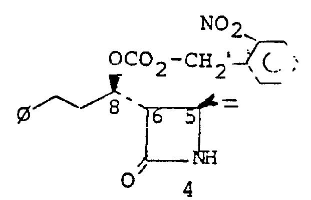

Using essentially the same procedure as in Examples 31, Step C, 150 mg of 11-t-butyldimethylsilyl-3a-((1(R,) -o-nitrobenzylcarbonyldioxy-3-phenyl)propyl]-4;-vinyl-2- azetidinone is converted to 116 m^{g} crude product. Preparative thin layer chromatography on silica qel (5% EA/ CH₂Cl₂) provides 104 mg of the title compound (87%).

IR(CHCl₃)µ 5.69 br.

NMR(300MHz, CDCl₃) δ 2.06-2.4-3 (m, ØCH₂CH2 2.62-2.81 (m, ØCH₂CH₂)_{'} 3.20(dd, J_{5,6} =2Hz, J_{6,8} =7Hz, H₆), 4.²3 (dd, _{J}=_{2Hz} & 7Hz, H₅), 5.18-5.34 & 5.85-5.97(m's, H₈ & CH=CH₂). 5.59 (s,-OCO₂CH₂-arom.), 7.17-8.19 (m, aromatics) .

Larger runs can be purified by chromatography on silica gel eluting with 0 → 10% EA/CH₂Cl₂ or 0→ 4% EA/CHC1₃.

### Step D

### Preparation of 4β-[1-bromo-2-(2-p-nitrobenzyloxycarbonylamino)ethylthio]ethyl]-3n-[(1(R*)-o-nitrobenzylcarbonyldioxy-3-phenyl)propyl]-2- azetidinone

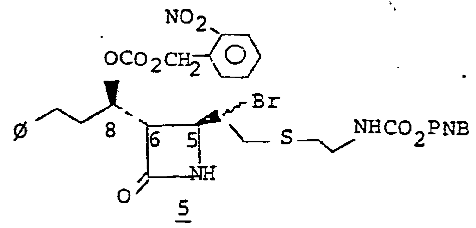

Following the procedure of Example 31, Step D, except that the reaction is allowed to stir for 1.5 hours during which time the temperature is not allowed to rise above + 10°C, 100 mg of 3a-[(1(R*)-o-nitrobenzyicarbonyldioxy-3-phenyl)propyl]-4β-vinyl-2-azetidinone is converted to 171 mg of crude product. Chromatography on silica gel- ( ~ 10 g, eluting with 0 → 20% EA/ CH₂Cl₂) provides 139 mg of the more polar product as a mixture of diastereomeric bromides (77%). IR(CHCl₃) 5.66, 5.74 sh, 5.82

NMR(300MHz, CDCl₃) δ 2. 06-2.34 (m,ØCH₂CH₂) , 2.64-2.85 (m,SCH₂CH₂N and ØCH₂CH₂), 2.95-3.09 & 3.16-3.23 (m's, CHBrCH₂S),3.32-3.50 (m,SCH₂CH₂N and H₆'s) , 3.95 (.dd,J_{5,6}=2Hz,J_{CH 5- CHBr} =7Hz, H₅ of minor diastereomer), 4.01 (dd,J_{5, 6=}2Hz, J_{CH5}=7.5Hz, H of major diaster- eomer), 4.04-4.16 (m.,-CHBr-'s), 5.16 (Midpt. of m,H₈) , 5.18 (s,NHCO₂CH₂arom.), 5. 52-5. 66 (m, -OCO₂CH₂arom.), 6.30, 6.46 & 6.65(3br s, NH'S), 7.16-8.25(m,aromatics).

### Step E

### Preparation of 3r-((1(R*)-o-nitrobenzylcarbonyldioxy-3-phenyl)fropylJ-4β-[2-(2-p-nitrobenzyloxycarbonylamino)ethylthio]vinyl]-2-azetidinone (6)

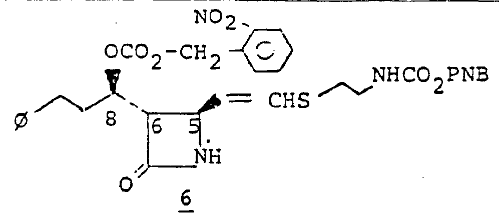

Treatment with 1.01 g. AgF (mw=127 ; 7.95 mmole) of 3.1 o. crude 4β - [ [1-bromo-2- (2-p-nitrobenzyloxycarbonylamino) ethylthiolethyl] -3a- [(1(R*)-o-nitro- benzylcarbonyldioxy-3-phenyl)propyl]*2-azetidinone (mw=744; 4.2 mmole) in 34 ml. pyridine at room temperature in the dark under N₂ for 1 hr. is followed by concentration of the entire reaction mixture under high vacuum and with a water bath at 25-30°C. to a brown-black residue. After chasing a few times with CHCl₃, the residue is slurried in silica gel CH₂-Cl₂ and run through a short column of silica gel (eluting with 2%. MeOH/CH₂Cl₂) thus removing mcst of the silver salts. The fractions containing product are combined. Two additional runs carried out as above give a total of 3.26 g. product (from a total of 9.84 mmol'e starting-material) still containing less polar impurities. Chromatography on silica ^{g}el (200 g., 1% MeOH/CH₂Cl₂) provides 0.63 g. of the desired product. Preparative thin layer chromatography on silica ^{g}el of 2.3 g. from column fractions which still contained less polar impurities (2,5% MeOH/CH₂Cl₂), provides 1.29 g. additional product total yield = 30%). Although the product is used as is for completion of the synthesis of (±)-9-benzyl- thienamycin, for complete characterization, preparative thin layer chromatography (100% Et₂O) of 35 mg. of product provides 30 mg. of the faster running trans-olefin and 5 m^{g}. of the cis-olefin.

Data for trans-olefin: IR(CHCl₃)µ 5.66, 5.77sh
_{N}MR (300 MHz, CDCl₃)S 2.02-2.22 (m, ØCH₂CH₂)_{'} 2.61-2.82.(m, ØCH₂CH₂), 2.85 (t, J=6.5Hz, SCH₂CH₂N), 3.18 (dd, J_{5,6} = 2Hz, J_{6,8} = 8Hz, H6) 3.40-3.47 (m, SCH2CH2N), 4.25 (dd, J = 2Hz φ 7.5 Hz, H5), 5.18 (s & m, H₈ & NHCO₂CH₂-arom.), 5.57 (midpt. of 2d, J = 15Hz, nonequivalent-OCO₂-CH₂-arom.)., 5.66- (dd, J = 15 Hz & 7.5 Hz, -CH=CHS), 5.92 (s, NH), 6.27 (d, J = 15 Hz, CH=CHS), 7.15 - 8.24 (m, aromatics).

Data for cis-olefin: IR(CHCl₃)u. 5.68, 5.79sh;
_{N}MR (300 MHz, CDCl3) δ 2.06-2.20 (m, ØCH₂Cl₂)_{'} 2.60-2.86 (m, ØCH₂CH₂ δSCH₂CH₂N), 3.23 (dd, _{5,6} = 2Hz,J_{6,8} = 7 Hz, H₆), 3.32 - 3.43 (m, CH₂CH₂N), 4.54 (dd, J=2Hz & 9.5 Hz, H₅) , 5.18 (s^{N}& ^{m,} H₈ & -NHCO₂CH₂-arom, 5.52 - 5.69 (m, noneauivalent-OCO₂-CH₂-arom. and -CH=CHS), 6.00 (s, NH), 6.16 (d, J=9Hz, CH=CHS), 7.14 - 8.22 (m, aromatics).

### Step F

### Preparation of a-hydroxy 3a-[(1-(R^{*})-o-nitrobenzyl- carbonyldioxy-3-phenyl)prepyl]-4β3-[2-T2-(p-n-trobenzyloxycarbonylamino)ethylthio]vinyl]-2-oxo-1-azetidirnemalonic acid, di-p-nitrobenzvl ester (7).

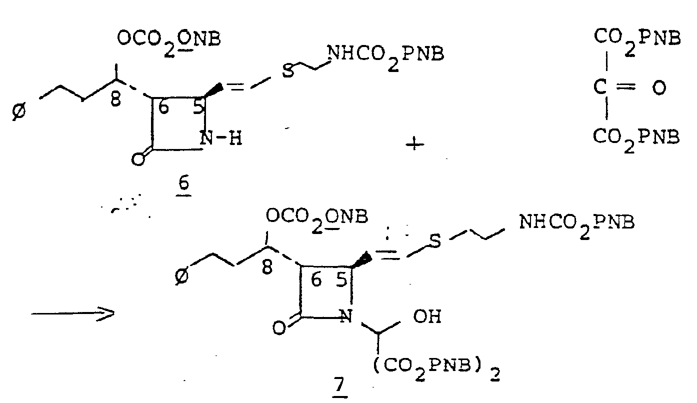

To a stirred solution of 473 mg. di(p-nitrobenzyl) ketomalonate (from Example 12, Step E) (mw = 388; 1.22 mmole) in 40 ml. hot anhydrous toluene is added a solution of 516 m^{g}. of mainly trans 6 (mw = 664; 0.78 mmole) in 5 ml. THF (distilled - from LAH) and 5 ml. anhydrous toluene. After some of the solvent is boiled off, additional anhydrous toluene is added, and the azeodrying process is repeated three times. The solution is then refluxed under N₂ for 30 minutes. Additional toluene is then allowed to boil off yet the volume is not allowed to diminish so much that precipitation occurs. Total heating time is approximately 2 1/2 hours. The clear yellow reaction mixture is removed from the oil bath and placed under a stream of N₂ which instantaneously causes clouding. After concentration to a yellow oil, the residue is dissolved in CH₂Cl₂, dried over anhydrous MgSO4, filtered, and concentrated under a N₂ stream to give crude 7.

The material is chromatographed on 120 g. silica gel packed and applied in CHC1₃. The column is eluted with CHCl₃ until the excess ketomalonate reagent emerges. Continued elution (1→)2% MeOH/CHCₗ₃) provides 563 mg. of slightly impure 7. Preparative thin layer chromatography on silica gel (30% EA/CH₂Cl₂) provides 450 mg^{.} of pure 7 (55%) . IR (CHCl₃ )u 5.67, 5.80sh

NMR (300 MHz, CDCl₃)δ 2.02 - 2.14 (m, ØCH₂CH₂), 2.58 - 2.82 (m, ØCH₂ CH₂ & SCHCH₂N), 3.22 (dd, J_{5,6} = 2Hz, J_{6,8} = 7Hz, H₆), 3.26 - 3.51 (m, SCH₂CH₂N) , 4. 66 (dd, J = 2Hz & 9Hz, H₅) , 5.15 (s & m, H8 & NHCO₂CH-arom.), 5.29 (s,-COH(CO₂CH₂-arom.) ₂) , 5.52 (midpt. of 2 d, J = 14Hz; nonequivalent - OCO₂CH-arom), 5.63 (dd, J=9Hz & 15 Hz, CH=CHS), 6.21 (d, J=15 Hz, CH=CH5) , 7.13 -'8.22 (m, aromatics). Cis olefin or mixtures of cis and trans olefin may also be carried through the following steps to yield more (±)-9-benzylthienamycin.

### Step G

### Preparation of 3α(-!(1- (P*) -o-nitrobenzylcarbonyldioxy-3-phenyl) propyl) -4β-[2- [2-p-nitrobenzyloxycarbonylamino) ethylthio]vinyl]-2-oxo-1-azetidinemalonic acid, di-p-nitrobenzylester (8).

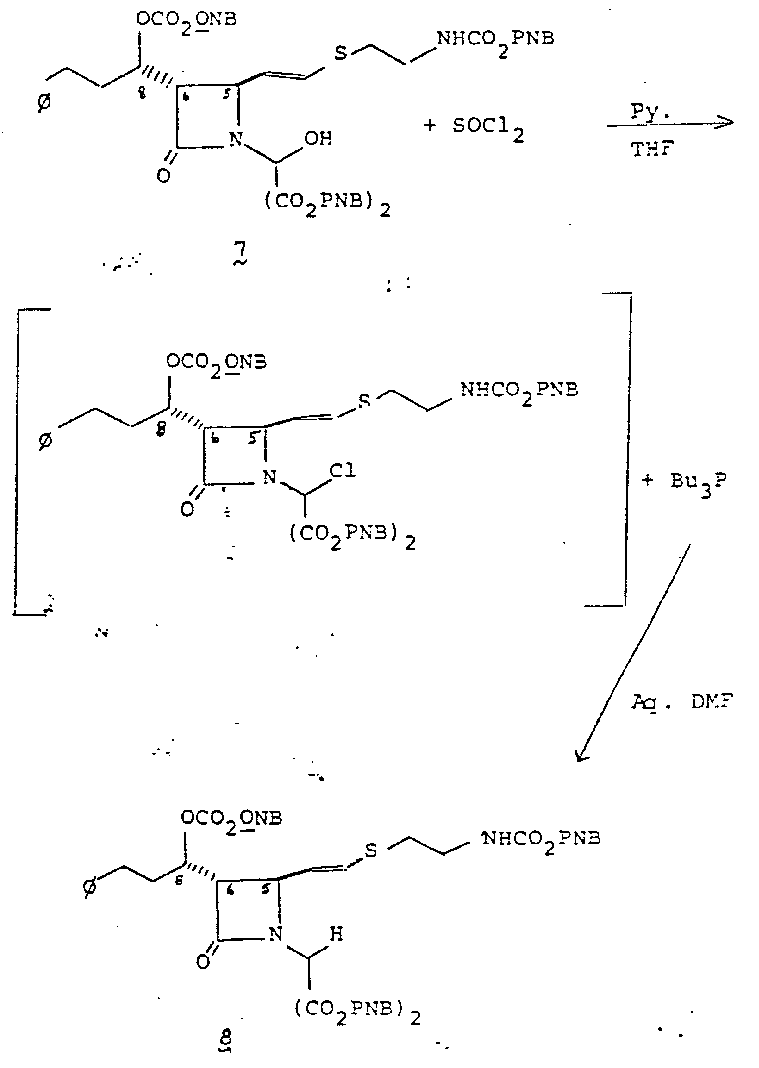

A solution of 431 mg. of 7 (mw = 1052; 0.41 mmole) in CH₂Cl₂ is dried over anhydrous MgSO₄ , filtered, concentrated under a N₂ stream, and dried further under high vacuum just prior to the following reaction. To a solution of 7 in 77 ml. THF (freshly distilled from LAH) at -20°C. is added 56 ul anhydrous pyridine (mw = 79; ρ= .982; 0.70 mmole).' With stirring under N₂, 80 mg. of freshly distilled thionyl chloride (mw = 119; 0.67 mmole) in 0.7 ml. THF is added. The reaction mixture is stirred for 10 minutes at -20°C., then 1/2 hour at 0°C. and finally 1 hour at 25°C. The pyridine hydrochloride is filtered under N₂ and washed with a few ml. THF. The filtrate and washings are concentrated under a N₂ stream followed by pumping on high vacuum. The resulting yellow foam is swirled in 7 ml. anhydrous THF, and a small amount of orange-red insoluble material is filtered off under N₂. red insoluble material is filtered off under N₂* The filtrate is reconcentrated as above to a yellow foam.

To this freshly prepared chloro compound is added with stirring a freshly shaken suspension of 181 mg. tributylphosphine (mw = 202; 0.90 mmole) in 10.4 ml. 9:1 DMF - H₂0 followed by 83 mg. K₂HPO₄ (mw = 174; 0.48 mmole). The reaction mixture is stirred at 25°C., for 35 minutes. After dilution with EA and brine, the layers are separated, and the aqueous one is extracted two times with EA. The combined organic layers are washed one-time with brine, dried over anhydrous MgSO₄, filtered and concentrated under a N₂ stream followed by pumping on high vacuum to give crude 8. The residue is slurried with a small volume of petroleum ether. The supernatant is removed, and the process repeated a few times. The petroleum ether supernatants containing some of the excess n-Bu₃P and n-Bu₃P(O) are discarded.

The residue is chromatographed on 40 g. silica gel packed, applied in, and eluted with CHCl₃. Those fractions containing product are combined, concentrated under a N₂ stream and then on high vacuum to give 286 mg, of slightly impure 8. Preparative thin layer chromatography on silica gel (40% acetone/hexane) then provides 251 mg. of pure 8 (59%).

IR(CHCl3) µ, 5.64 sh, 5.71, 5.81 sh NMR (300 MHz, CDCl₃) δ 1.99 - 2.18 (m, ØCH₂CH₂), 2.58 - 2.82 (m, ØCH₂CH₂ and SCH₂CH₂N), 3.27 (dd, J_{5,6} = ^{2.}5Hz, J₆,₈ = 7.5Hz, H₆) , 3.30 - 3.42 (m, SCH₂CH₂N) , 4.54 (dd, J = 2.5Hz and 9Hz, H₅), 5.13-5.36 (m, NHCO₂CH₂-arom. and CH(CO₂CH-arom)₂), 5.44 - 5.61 (m, -OCO₂CH₂-arom. and CH=CHS), 6.23 (d, J = 15Hz, CH=CHS), 7.13 - 8.22 (m, aromatics).

### Step H

### Preparation of 2,2-bis(carbo-p-nitrobenzyloxy)-4-bromo-6-exo-[(1-(R*)-o-nitrobenzylcarbonyldioxy-3-phenyl)propyl]-3-[(2-p-nitroben2yloxycarbonylamino) ethylthio]-1-azabicyclo[3.2.0]heptan-7-one (9).

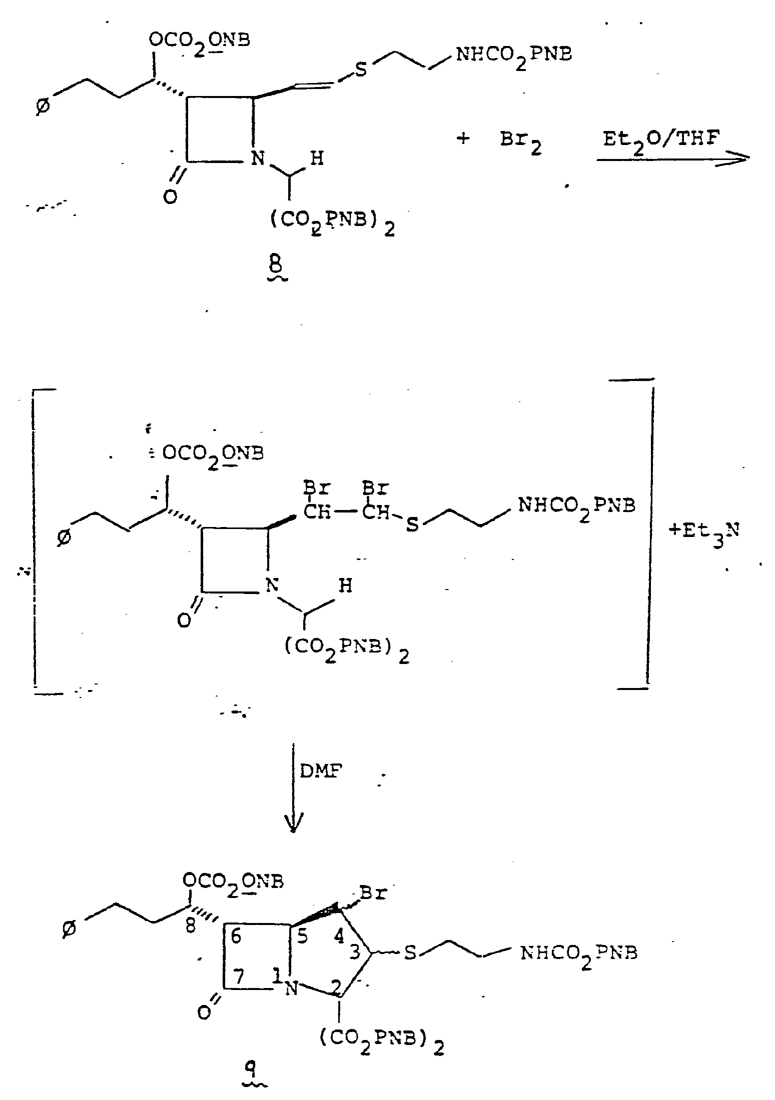

To 8.5 ml. CCl₄ (dried over 4A Linde molecular sieves) is added 0.2 ml. Br₂ (mw = 160; 3.9 mmole).

To 0.244 g. of 8 (mw = 1036; 0.236 mmole) in 6 ml. THF (freshly distilled from LAH) and 1.8 ml. Et₂0 (dried over 3A 1/16" Linde molecular sieves) at 0°C. under N₂ with stirring is added dropwise 0.6 ml. of the above 0.45M Er₂ solution (0.27 mmole). After 15 minutes at 0°C., 143µl triethyl amine (mw = 101; = 0.729; 1.03 mmole) is added immediately followed by 3.4 ml. ice-cold DMF (distilled from Caso₄ at 40 mm and stored over 4A Linde molecular sieves). The ice-bath is removed, and stirrinc at room temperature is continued for 2 hours. The reaction mixture is' poured into a stirred ice-cold mixture of 1.1 ml. 1M KH₂P0₄ - H₂0-EA. The layers are separated, and the aqueous one is saturated with NaCl and re-extracted with EA. The combined organic layers are washed once with brine, dried over anhydrous MgS0₄, and filtered. The filtrate is concentrated under a N₂ stream and then pumped on high vacuum to give crude 9.

Preparative thin layer chromatography on silica gel (40% acetone/hexane) provides 250 mg. of 9 (93%). IR (CHCl₃)µ 5.61, 5.74, 5.81 sh; NMR (300 MHz, d₆-acetone) peaks related to major diasteriomer: 2.10 (m buried under d₆-acetone, ⌀CH₂CH₂), 2.71 - 3.16 (m, ⌀CH₂CH₂ and SCH₂CH ₂N), 3.37 - 3.53 (m, SCH₂CH₂N), 3.94 (dd, J_{5,6} = 3Hz, ^{J}6,8 ⁼ 5.5Hz, H₆). 4.41 (dd,-J_{5,6} = 3HZ,J_{4,5} = 6Hz, H₅) , 4.52 (d, J_{3 ,4} = 6Hz, CHS) , 5.11 (dd appearing as a ^{t,} J4,5 = J_{3,4} = 6Hz, CHBr), 5.21 - 5.65 (m, all CH₂-arom. and H₈), 6.72 (br, NH), 7.2_ - 8.27 (m,aromatics); peaks unique to two minor diastereomers_{'} ^{4.}13 (m, H₆) . 4.24 (m, H₅) , 4.36 (m, 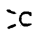 HS and 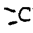 HBr of one minor diastereomer), 5.02 (d, J_{3,4} ⁼ 5_{H}z, 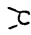 HS of 2nd minor diastereomer), 5.14 (m, 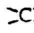 HBr of 2nd minor diastereomer.

### Step I

### Preparaticn of 2,2-bis(carbo-p-nitrobenzyloxy)-6-exo- [ (1- (R*) -o-nitrobenzylcarbonyldioxy-3-phenyl) propyl] -3- [ (2-p-nitrobenzyloxycarbonylamino) ethylthio] - 1-azabicvclo[3.2.0]hept-3-en-7-one (10)

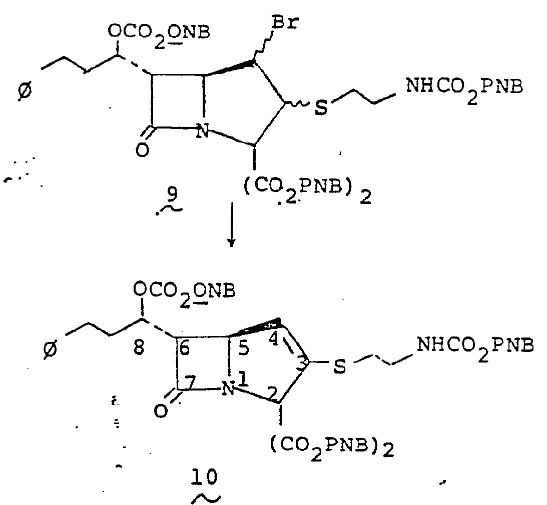

To 80 mg anhydrous silver fluoride (mw=127; 0.63 mmole) is added a solution of 503 mg of 9 (mw=1114; 0.45 mmole) in 13 ml anhydrous pyridine. Under N₂' the reaction mixture is stirred at room temperature in the dark for one hour and then poured into cold water and EA. After separation of the layers, the aqueous one is extracted two times with EA and one time with CHC1₃. Each organic layer is extracted one time with H₂0 and one time with brine. The combined organic layers are dried over anhydrous MgSO₄, filtered, and concentrated under a N2 stream followed by pumping on high vacuum to give crude 10. Preparative thin layer chromatography on silica gel (40% acetone / hexane)provides 398 mg of 10 which by thin layer chromatography (3% Et₂O/ CH₂Cl₂) still contained a slightly faster impurity. Therefore, further preparative thin layer chromatography (3%Et₂O/CH₂Cl₂) provides 255 mg of pure 10(55%).

IR(CHCl₃)µ 5.61, 5.73, 5.81 sh

NMR (300MHz, CDCl₃)δ 2.04-2.14 (m,⌀CH2CH2) 2.61-2.83 (m ⌀CH₂CH₂): 2.91-3.08(m,SCH₂CH₂N), 3.36-3.46 (m.H₆ & SCH₂CH₂N) ; 4.70(brs, H₅) , 5,16(s &m,NHC0₂^{_} i CH2-aromatics &H₈), 5.29 & 5.31(2s, 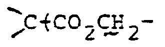 arom.)₂), 5.58(midpt. of 2d, J=15Hz, nonequivalent -OCO₂-CH₂-arom. ) , 6.12 (br . S^{H}, vinyl H₄ ) , 7 .14 - 8.22 (m, aromatics) .

60 MHz shows additional couplings not observable with 300MHz: 4.70(dd, J_{5,6}=3Hz; J_{4,5}=1.5Hz,H₅), 6.12(d,J_{4,5}=1.5Hz,vinylH₄).

### Step J

### Preparation of 2-carbo-p-nitrobenzyloxy-6-exo- . [(1-(R*)-o-nitrobenzylcarbonyldioxy-3-phenyl)propyl]- 3- [(2-p-nitrobenzyloxycarbonylamino) ethylthio] -1- azabicyclo [3.2.0] hept-3-en-7-one (11).

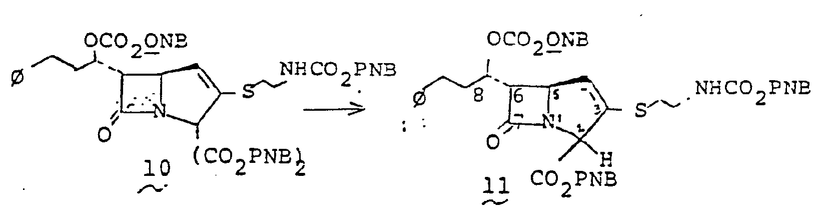

A solution of 144 mg of 10 (mw = 1034; 0. 014 mmole) in 1.5 ml S-collidine (distilled from powdered KOH^{~} 30 mm pressure)a is added to 25.1 mg anhydrous LiI (dried for few hours at 100°C over P₂O₅ under vacuum) (mw = 134; 0.19 mmole). With stirring under N₂, the reaction mixture is heated in an oil bath at 120° - 130°C. ^{N} After a total of 30 minutes, the reaction mixture is cooled to 25°C., diluted with CH₂Cl₂, and transferred to a round bottom flask for concentration under a N₂ stream and then on high vacuum. Partitioning the residue between EA-H₂O and 2.3 ml 1M KH₂PO₄ is followed by extraction of the aqueous layer two additional times with EA and one time with CHCl₃. Each organic layer is then backwashed with brine. The combined organic layers are dried over anhydrous MgS0_{4'} filtered, concentrated under a N₂ stream and then on high vacuum to give crude 11.

Preparative thin layer chromatography on silica gel (40% acetone/hexane) provides 57 mg of slightly impurell and 48 mg of slightly impure starting material, travelling just slightly slower.

Two subsequent decarbalkoxylations (starting with 146 mg and 234 mg of 10)followed by preparative thin layer chromatography as above yields a total (for the three runs) of 200 mg slightly impure 11 and 111 mg slightly impure starting material. Further preparative thin layer chromatography on silica gel (5%-Et₂ O/CH₂Cl₂ ) provides 164 mg of pure 11(38%). Similar chromatography of the recovered starting material provides 78 mg of pure 10 (15%) which may be recycled.

Data for 11:
IR(CHCl₃) 5.63, 5.72, 5.76
KMR(300MHz, CDCl₃)δ 2.07-2.15(m,⌀CH₂CH₂) 2.63- 2.85 (m,⌀CH₂CH₂), 2.89-3.09 (m, SCH₂CH₂N) 3.33 (dd,J_{5,6}=2.5Hz, J_{6,8}=7.5Hz, H₆), 3.40-3.49 (m,SCH₂CF₂N) , 4 . 66 (brs, H₅), 5.19(s & m, NHCO₂CH₂- arom., H₈ and H₂) , 5.27 (midpt. of 2d, J=15Hz, nonequivalent CO₂CH₂-arom.), 5.60(midpt. of 2d, J=14Hz, nonequivalent-OCO₂CH₂-arom.), 5.94(brs, 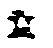 vinyl H₄), 7.15-8 . 24 (m, aromatics) .

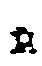 60 MHz shows additional couplings not observable with 300 MHz: 4.57-4.70(m,coupling to H₆,vinyl H₄, and homoallylic coupling to H₂ evident, H₅) , 5.94 overlapping dd appearing as a t, J_{2,4}=J_{4,5}= 1.5Hz, vinyl H₄).

### Step K

### Preparation of 2-carbo-p-nitrobenzyloxy-6-exo-[(1-(R*)-o-nitrobenzylcarbonyldioxy-3-phenyl)propyl]-3-[(2-p-nitrobenzyloxycarbonylamino)ethylthio]-1-azabicyclo[3.2.0]hept-2-en-7-one (12).

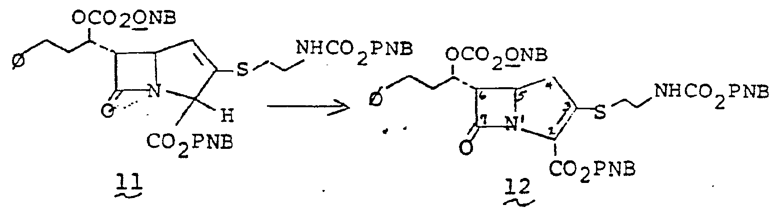

To 52 mg of 11 (mw=855; 0.061 mmole) in 0.75 ml DMSO (distilled from CaH₂ at 8 mm and stored over 4A _{L}inde Molecular Sieves) is added 102 µl diisopropylamine (distilled from NaH under N₂ and stored over 4Ar Linde molecular sieves) (mw=101;f= 0.722; 0..73 mnole). The stoppered reaction mixture is stirred for a few minutes and then allowed' to stand for 2.5 hours. The amine and most of the DMSO are then concentrated off under high vacuum with no external heating. The residue is chased two times with EA and two times with CHCl₃ to give a yellow foam essentially free of DMSO. Preparative thin layer_{.}chromatography on silica gel (15% EA/CHCl₃) provides 10 mg of the more polar product 12. Extraction of the starting material band yields 39 mg recovered 11. Resub- mittingll to the reaction conditions and isolation procedure-three more times gives a total of 24 mg of 12. A final preparative thin layer chromatography of 12 on silica gel (50% EA/hexane ) removes two very minor less polar impurities and provides 22 mg of pure 12(42_{%}). A similar final purification of recovered starting material (40% EA/hexane) yields 11 mg of recoveredll (21%).

Data for 12:
IR(CHCl₃)µ 5.63, 5.74 sh, 5.81
NMR(300MHz, CDCl₃)δ 2.10-2.22 (m,⌀CH₂CH₂) , 2.64-2. 88 (m,⌀CH₂CH₂) , 2.92-3.12 (m,SCH₂CH₂N,- & dd, J_{4,5}=8.5Hz & J_{4,4},=18Hz,H₄), 3.36(dd,J_{4',5}= 10Hz,J _{4',4} =18Hz,H₄,), 3.40-3.50(m,SCH₂CH₂N and H₆), 4.25-4 .32 (m,H₅) , 5.18(midpt. of m,H₈) , 5.19 ( s,-NHCO₂CH₂-arom.), 5.36(midpt. of 2 widely spaced d, J=14Hz, nonequivalent -CO₂CH₂-arom. ) , 5.58 (midpt. of 2 widely spaced d,J=14Hz, nonequivalent, -OCO₂CH₂-arom.), 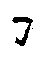 .18-8.24(m,aromatics).

When spectrum is run in d₆-acetone, H₆ is no longer buried under SCH₂CH₂N: NMR(300MHz,d₆-acetone) δ 3.82(dd,J_{5,6}= 3Hz, J_{6,8}=6Hz,H₆).

### Step L

### Preparation of (±)-9-benzylthienamycin (13).

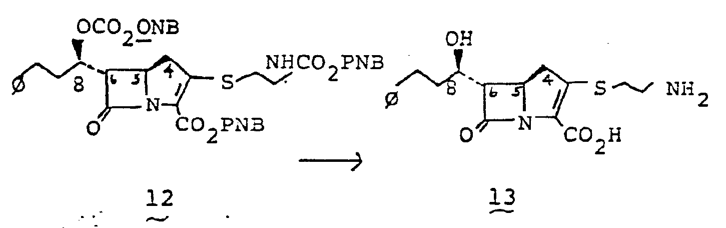

To 5.3 mg 12(mw=855; 0.0062 mmole) is added 350 µl dioxane, 350 µl ethanol, 175 pl deionized water, and 11 µl 1M K₂HPO₄- To the resultant clear solution is added 5.3 mg of 10_{%} Pd/C and a glass bead for efficient mixing. The suspension is flushed with N₂, then 5-6 times alternately with 50 psi H₂ and vacuum. `Finally, it is shaken under a 50 psi H₂-atmosphere for 50 minutes. After cooling in an ice bath followed by centrifugation, the supernatant is removed and filtered through a small plug of cotton. The Pd/C is washed and centrifuged 5x with small volumes of cold, deionized water, and the supernatants are filtered as above. The combined, cold fil.trates are extracted 3x0.5ml Et₂O, 2x0.5 ml EA, and then 2x0.5 ml Et₂0. The aqueous layer is then pumped briefly on a water aspirator to remove any residual organic solvents. The remaining solution (ca0.7 ml in volume) is chromatographed (portionwise) by reverse phase HPLC on a Waters analytical µC₁₈-column (eluting with 10% _{T}HF in water). The peak having a UV_{Max.} at 299m-is collected and is estimated to contain 280-300 ug of 13(13% yield based upon hydroxylamine quenchable UV). The solution is concentrated on a rotary evaporator under high vacuum and with a water bath no warmer than 25-30°C to a final volume of,ca 1 ml. The concentrate is then shell-frozen in a lyophilizing vial and lyophilized in a shelf lyophilizer wherein the shelf may be held at -20°C. The product exhibits a 300MHz NMR spectrum in D₂0 with. peaks at: δ (no internal standard is used) 1.92-2.04 (m, ⌀CH₂CH₂), 2.70-3.28 (m, ⌀CH₂ CH₂, SCH₂-CH₂N, and H₄'s), 3.66 (dd, J_{5,6}=3HZ, J_{6,8}=6Hz,H₆), 4.10-4.17 and 4.24-4.30 (m's, H₅&H₈) , 4.90 (HDO), 7. 36-7.50 (m,⌀)

### EXAMPLE 6

Following the procedure of the foregoing Examples and text, the following endproducts can be prepared from the compounds of the present invention wherein _{R}⁶, _{R}⁷ and _{R}⁸ are defined accordingly.

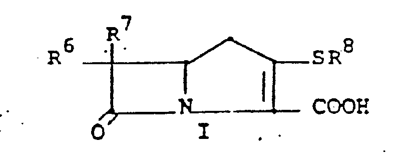

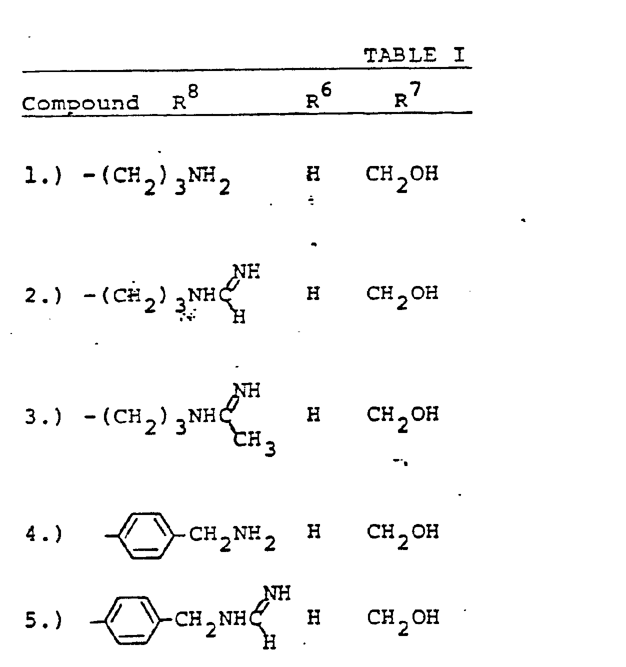

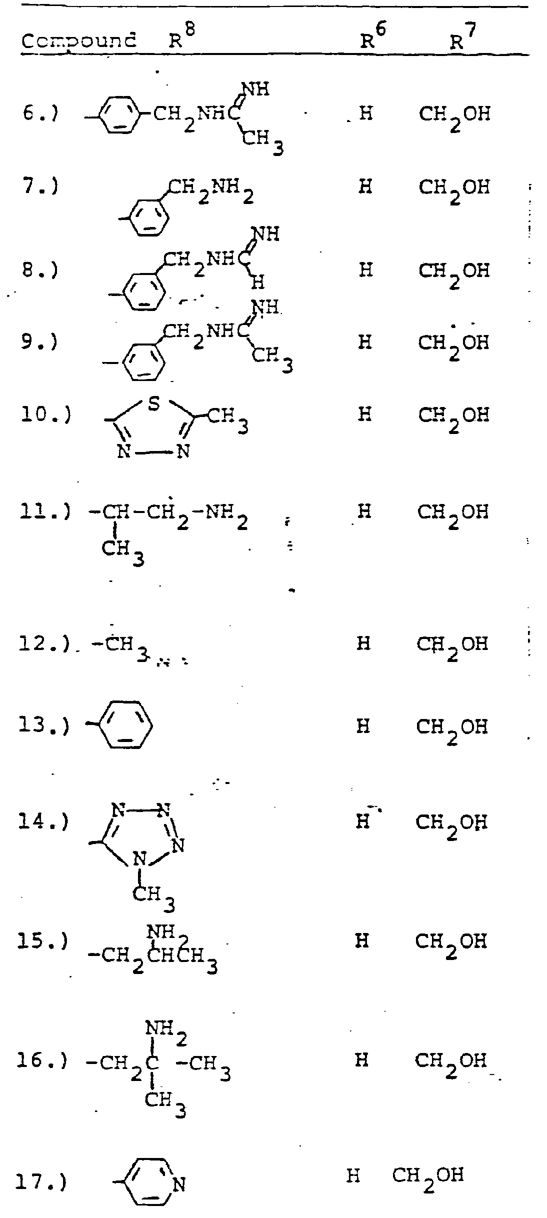

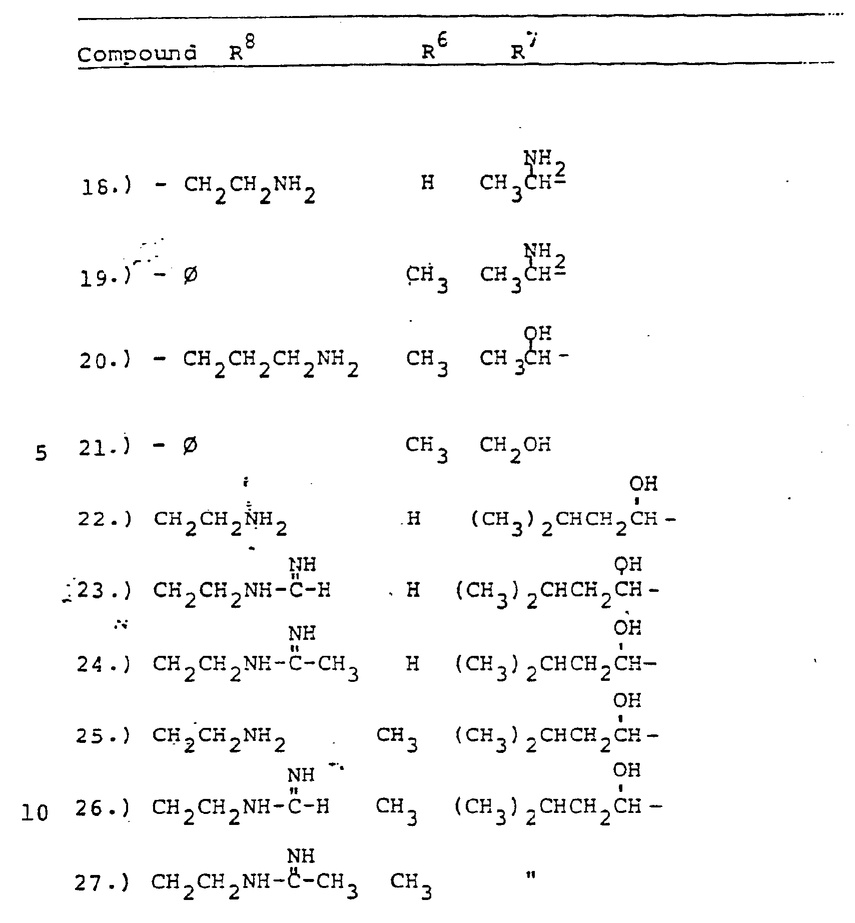

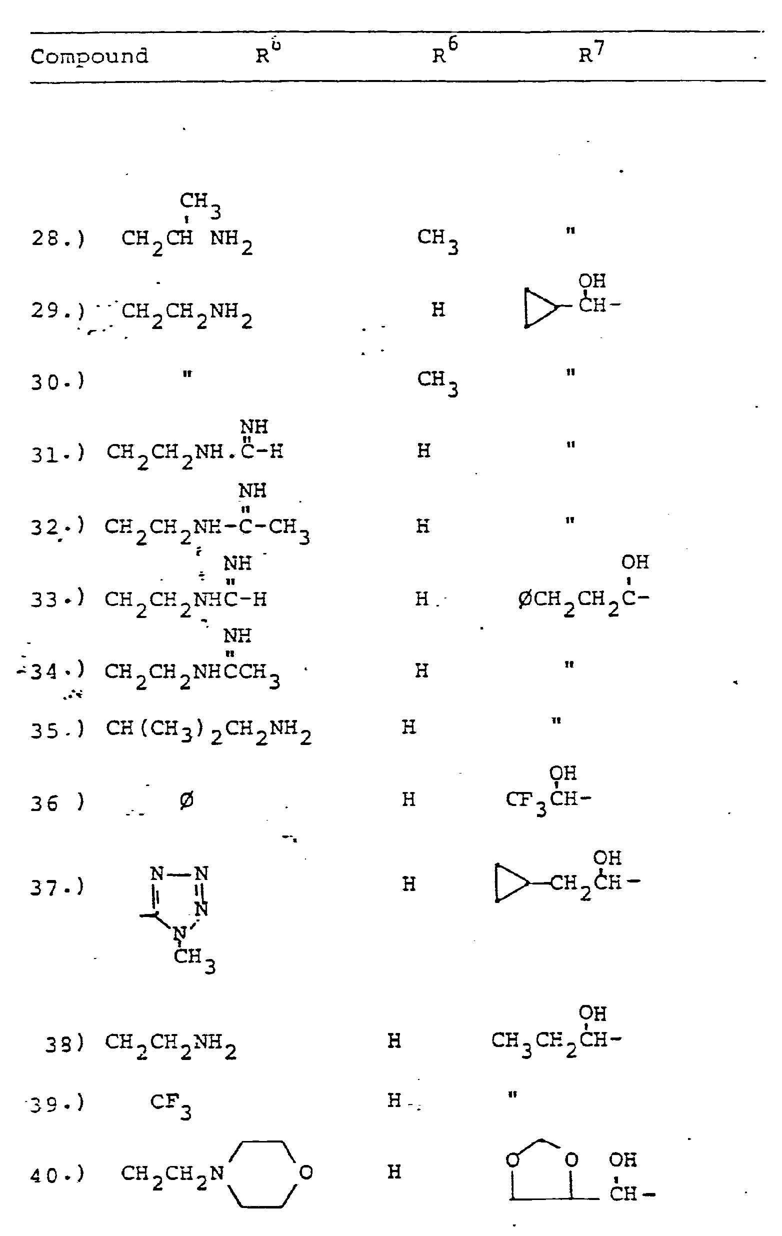

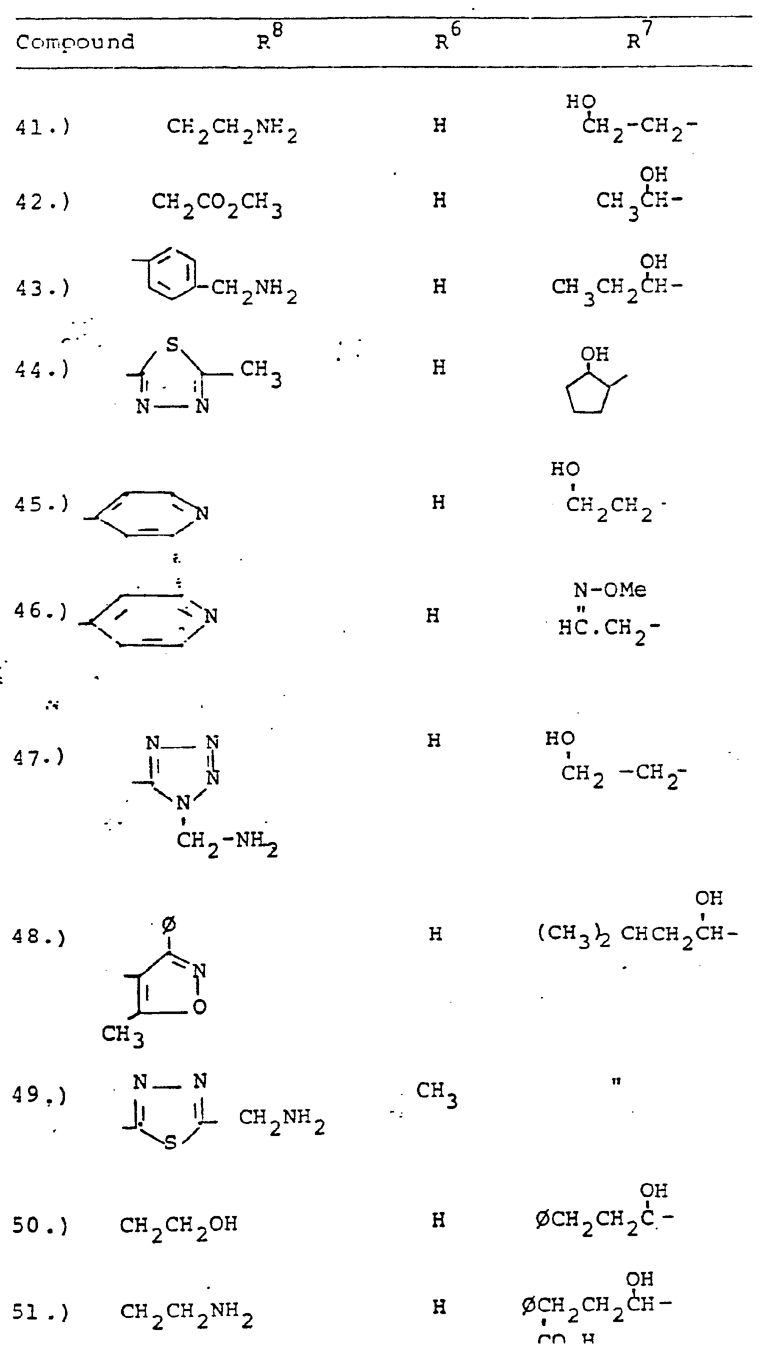

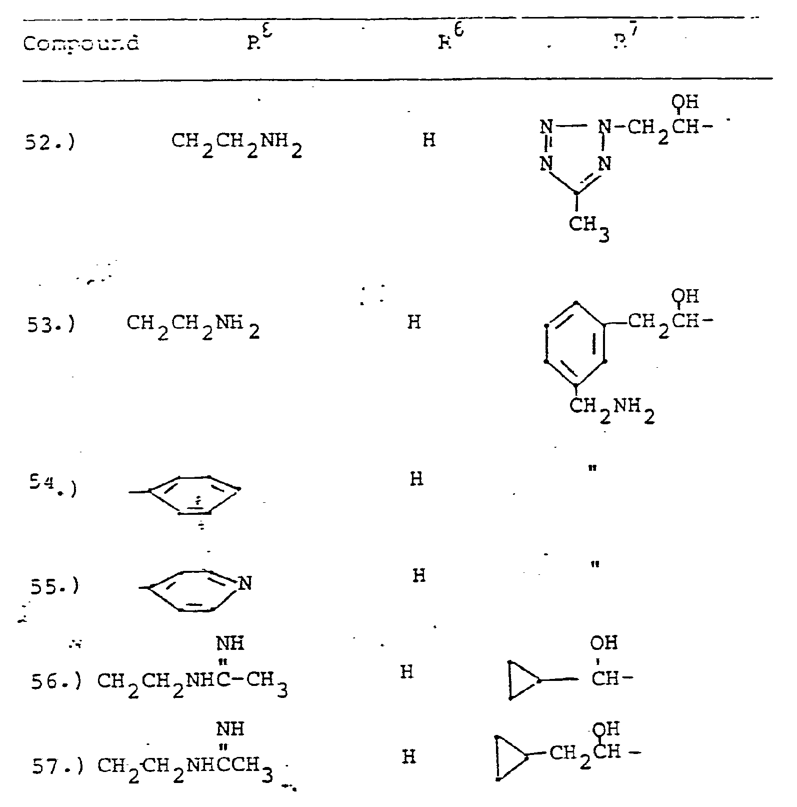

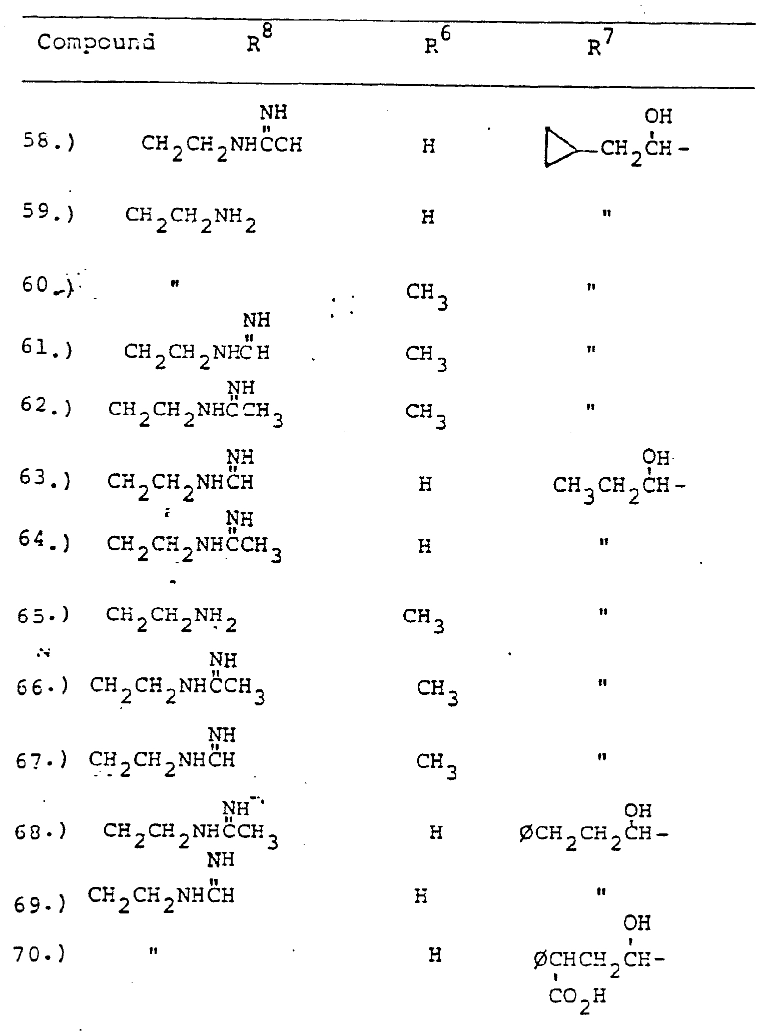

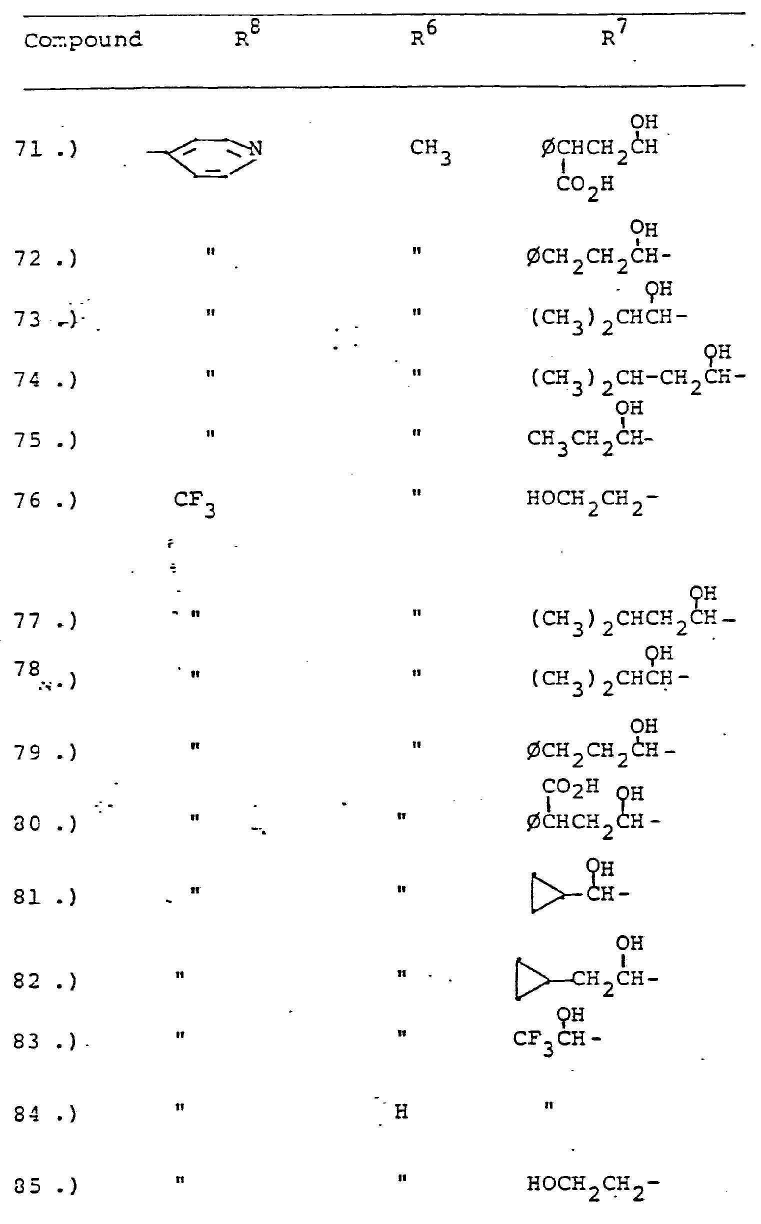

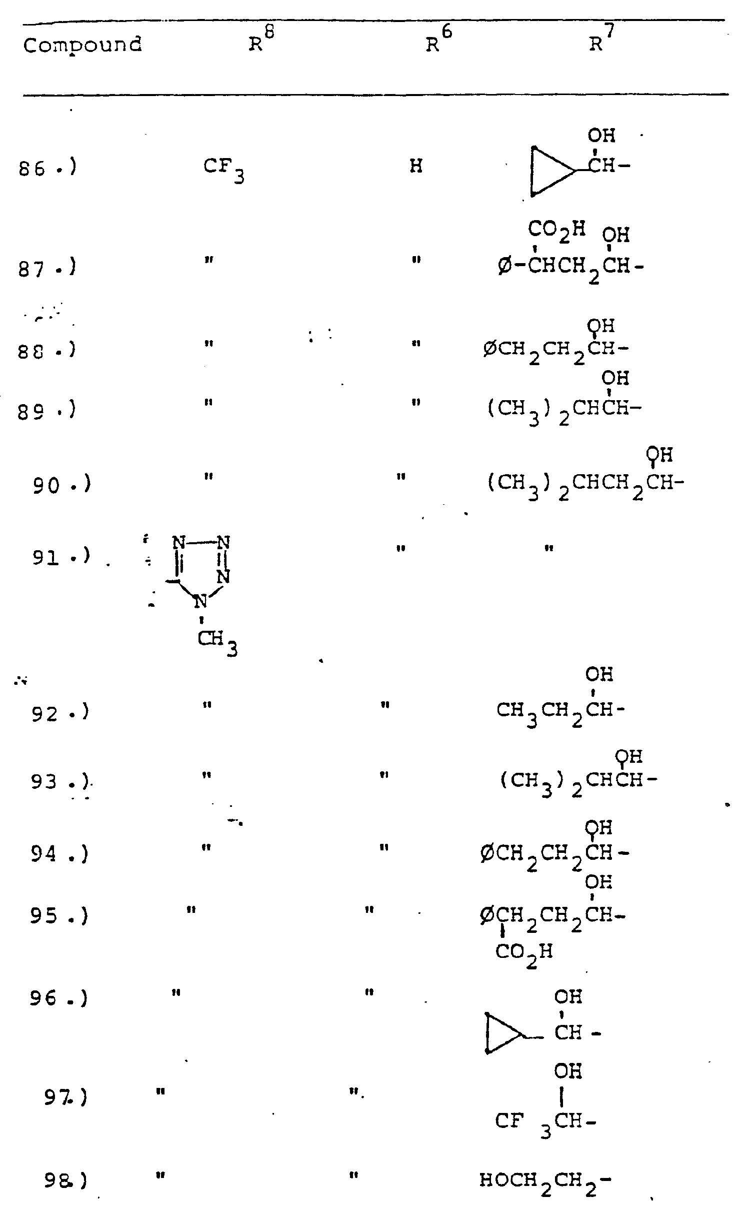

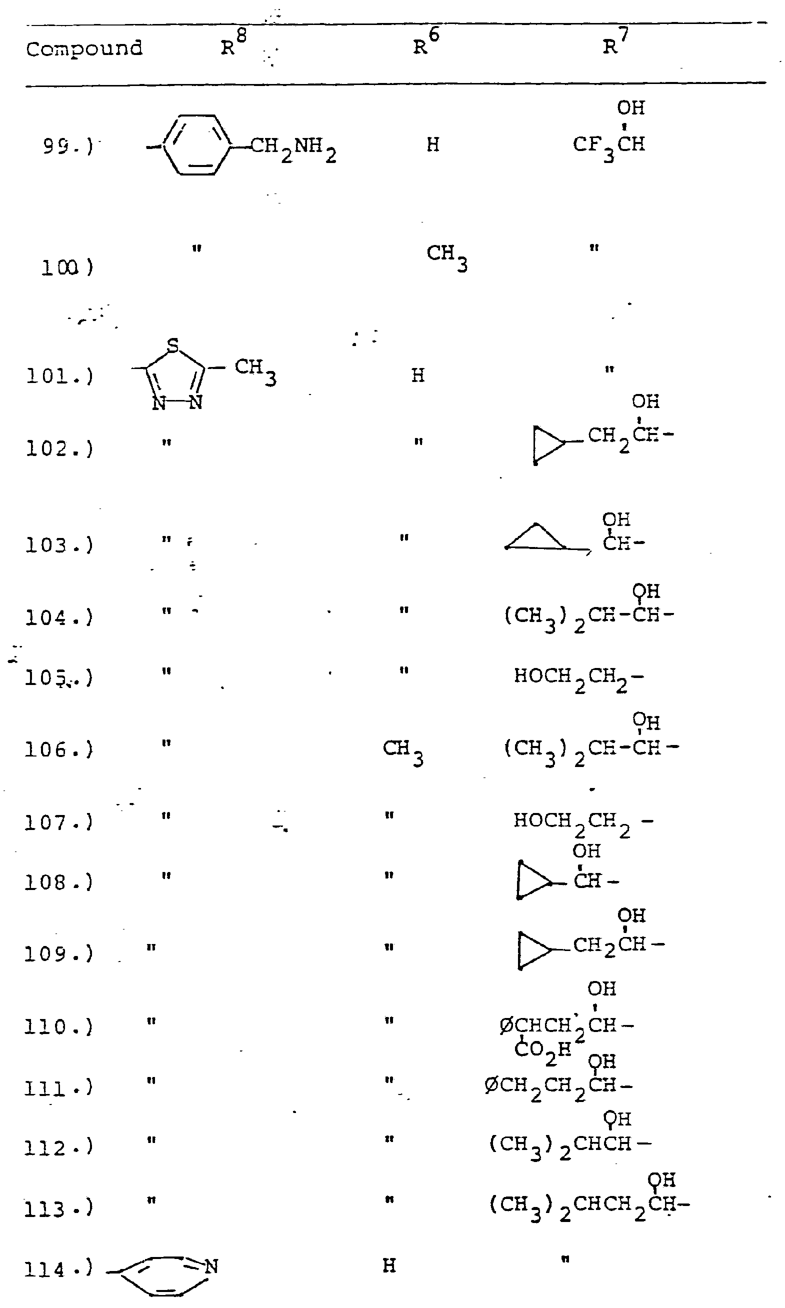

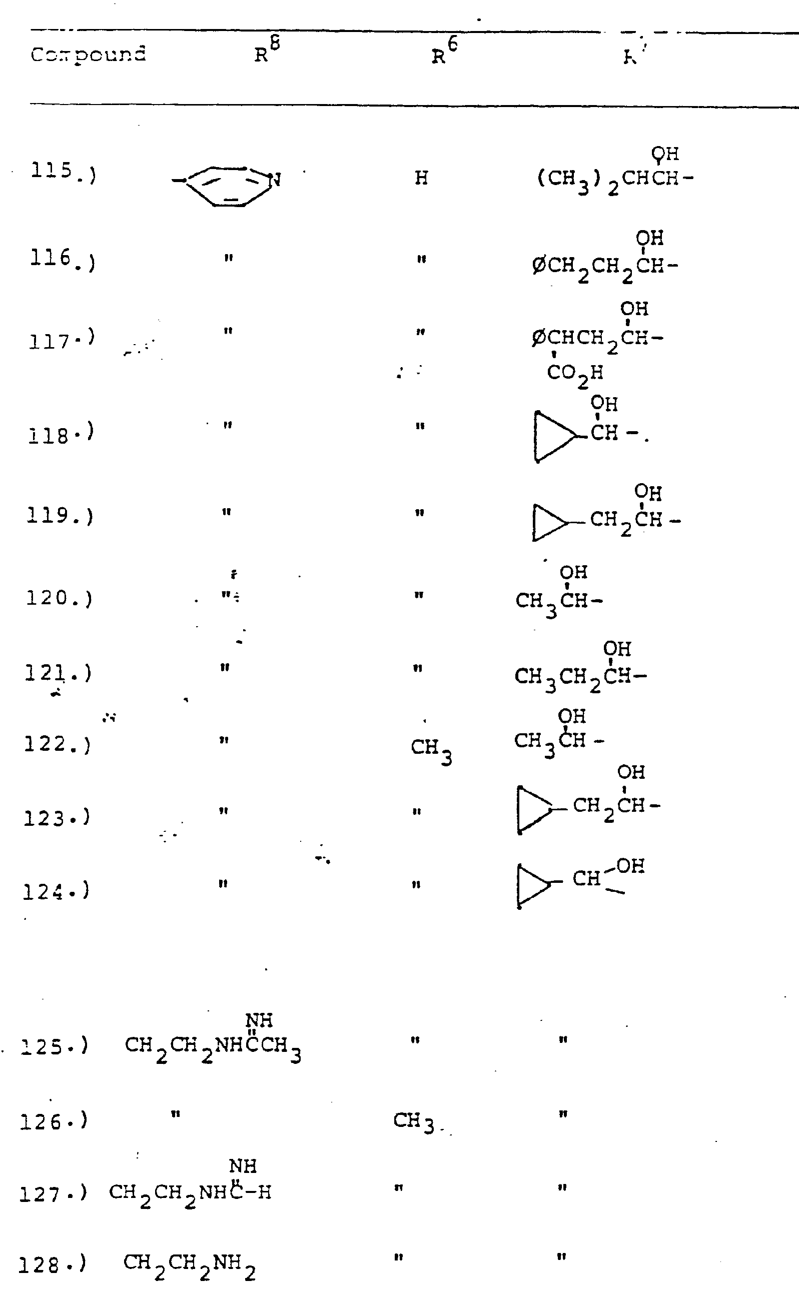

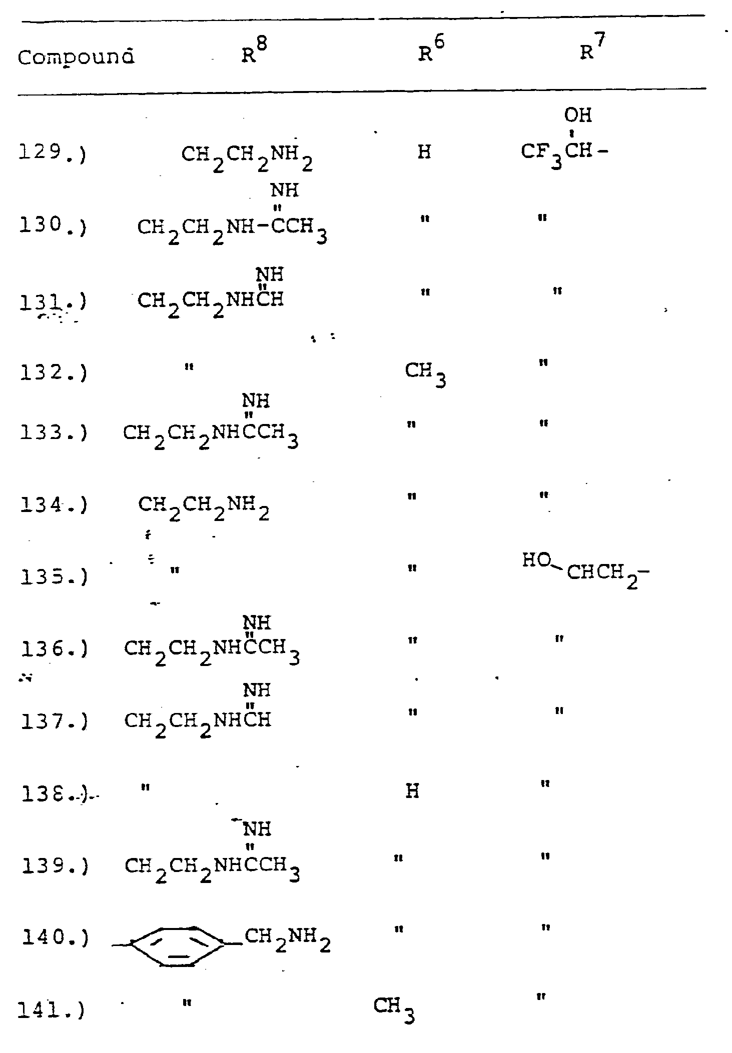

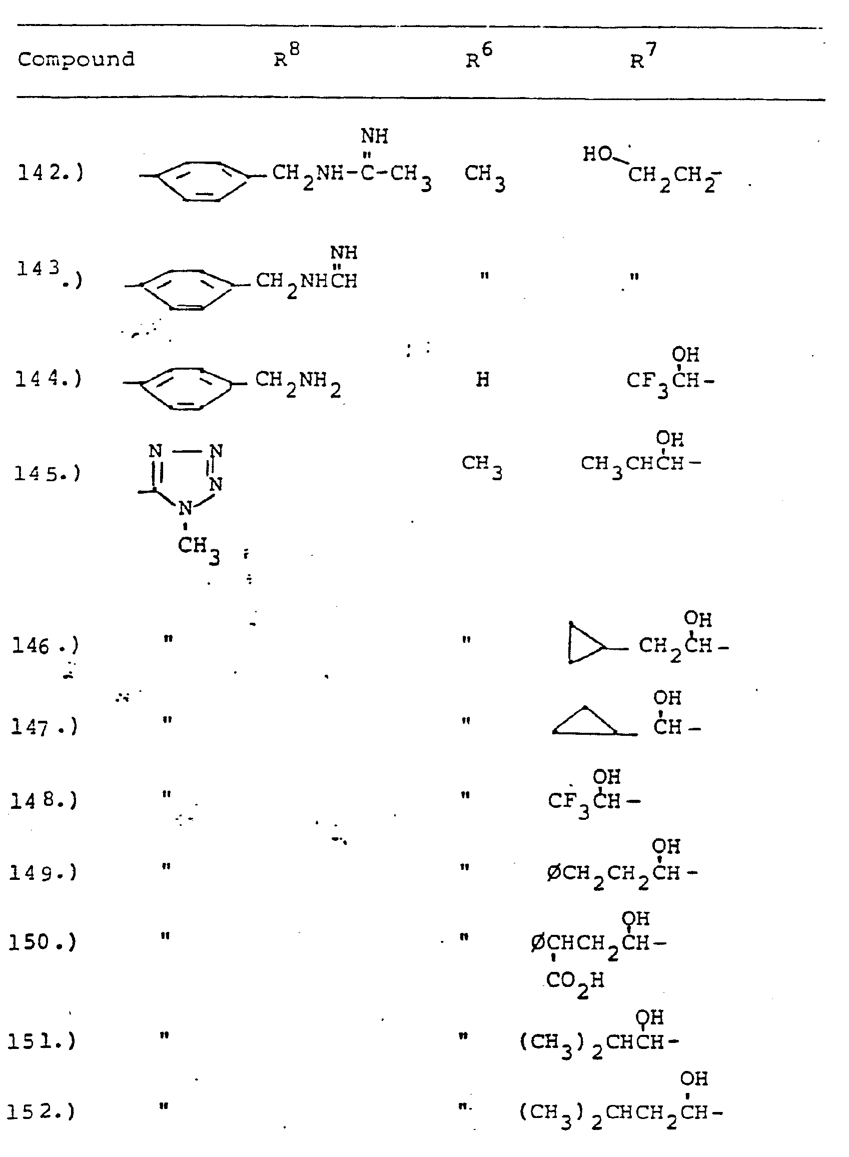

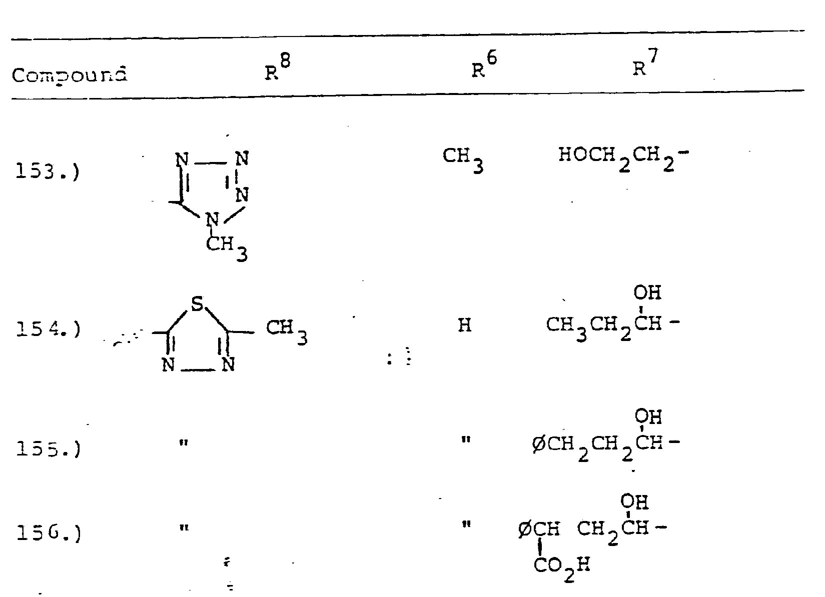

### EXAMPLE 7

### Preparation of 3-ethyl-l-(t-butyldimethylsilyl)-4-vinyl-2-azetidinone

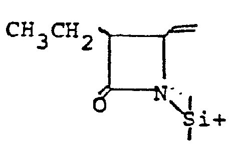

Following the procedure for the preparation of 8-oxo-2,2-dimethyl-7α-isopropyl-3-oxa-1-azabicyclo [4.2.0]octane (Example 7a), except that an equivalent amount of l-(t-butyl-dimethylsilyl)-4-vinyl-2-azetidinone is substituted for the 8-oxo-2,2-dimethyl-3-oxa-1-azabicyclo [4.2.0]octane of Example 4a and using ethyl iodide instead of isopropyl iodide, the title compound is obtained.

### EXAMPLE 7a

### Preparation of 8-oxo-2,2-dimethyl-7c-isogropyl-3-oxa- l-azabicyclo[4-2-0)octane

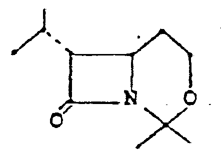

THF, 20 ml is placed under N₂, treated with 1.54 ml diisopropylamine and cooled to -78°C. A solution of n-butyl lithium 1.97M in hexane 5.6 ml is added dropwise over 5 min. The reaction mixture is stirred at -78°C for 10 min. and then treated with 8-oxo-2,2-dimethyl-3-oxa-1-azabicyclo [4·2·0]octane 1.55 g in 15 ml THF added dropwise over 5 min. After another 10 min. hexamethylphosphoramide 1.97 ml is added. The mixture is stirred another 10 min., then treated with 2 ml of isopropyl iodide. The reaction mixture,is stirred at -78°C for 15 min. and allowed to warm to 25°C and stirred for 15 min. The reaction mixture is diluted with EtOAc, washed once with pH 7 phosphate buffer then dried and evaporated. The residue is chromatographed on silica gel using 25% EtOAc/C6H6 as eluant to give 8-oxo-2,2-dimethyl-7α-isopropyl-3-oxa-1-azabicyclo [4.2.0]-octane. µ i.r. : 5.7 (β-lactam). n.m.r.δ: 0.96d, 1.06d (CH₃-C-H); 1.4S, 1.765 (^{g}em dimethyl); CH₃
1.9m (C-5 H); 2.59d of d (C-7 H);
3.33m (C-6 H); 3.83 d of d (C-4 H).

### EXAMPLE 8

### Preparation of 3-ethyl-4-(2-(2-p-nitrobenzyloxycarbonylamino)ethylthio)vinyl]-2-azetidinone

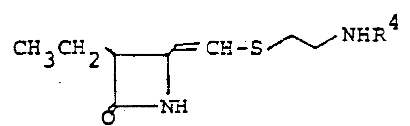

Following the procedures in Example 1 - Steps D-F except that an equivalent amount of 3-ethyl-1-(t-butyl-dimethylsilyl)-4-vinyl-2-azetidinone is used in place of Compound 26 of Example 1 - Step D, the title compound is obtained.

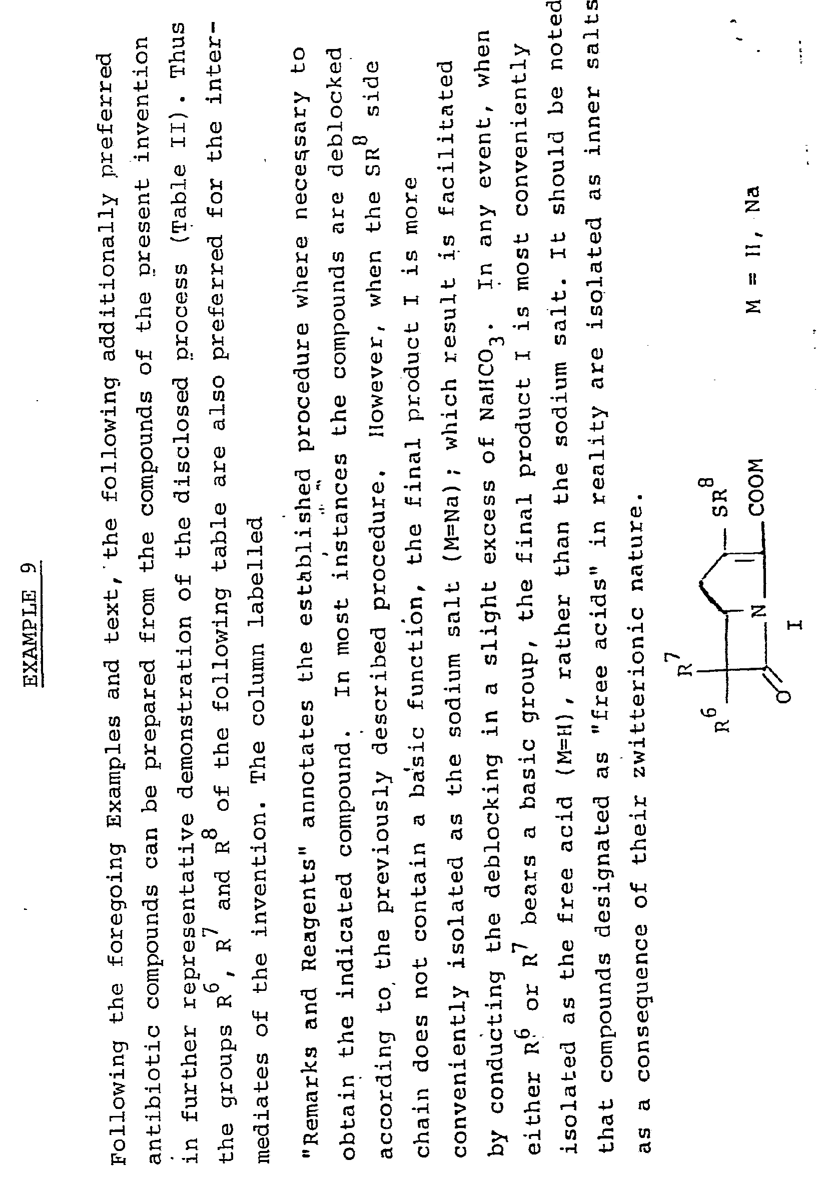

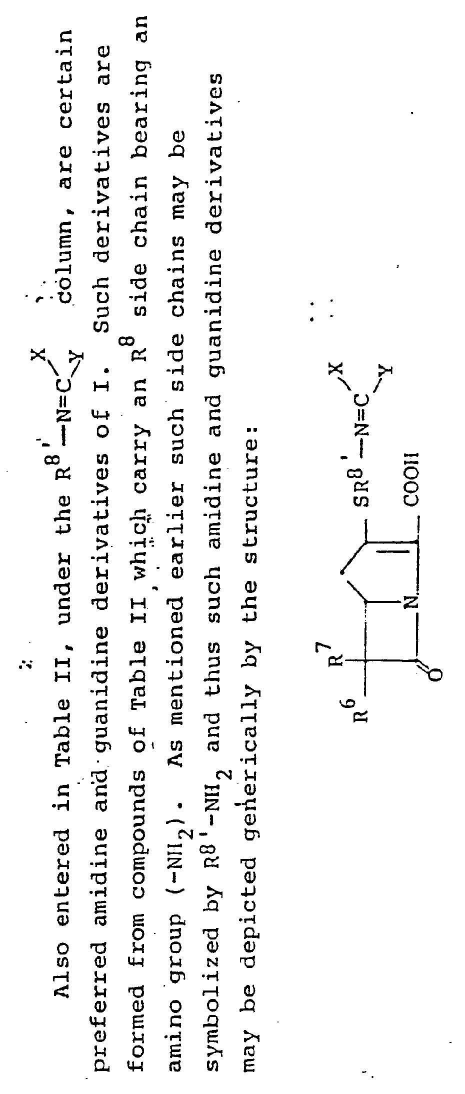

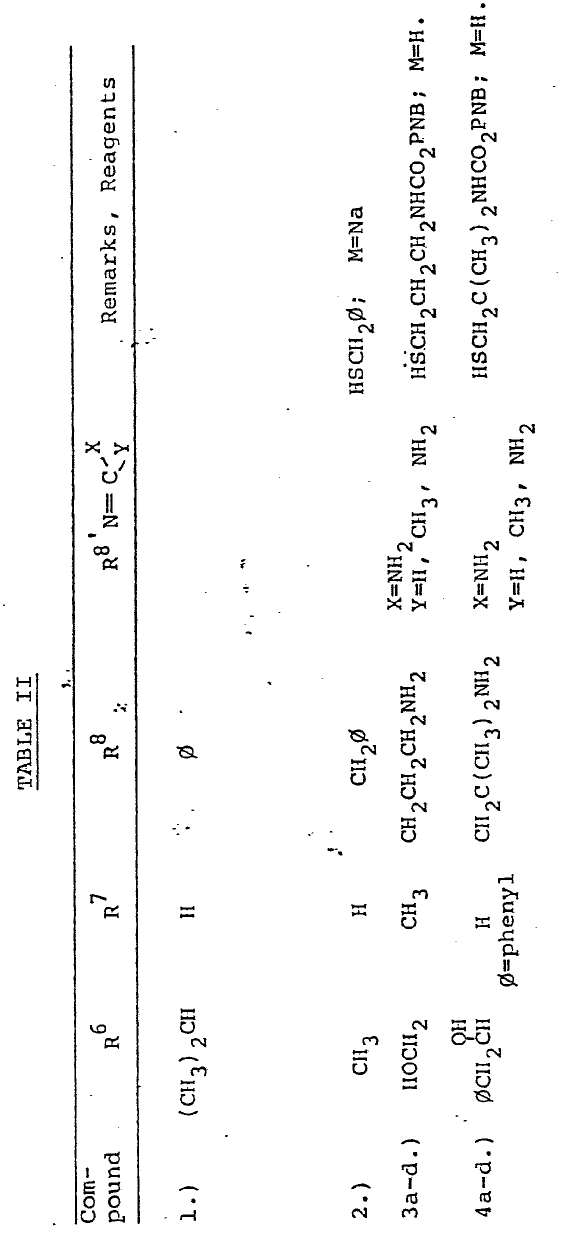

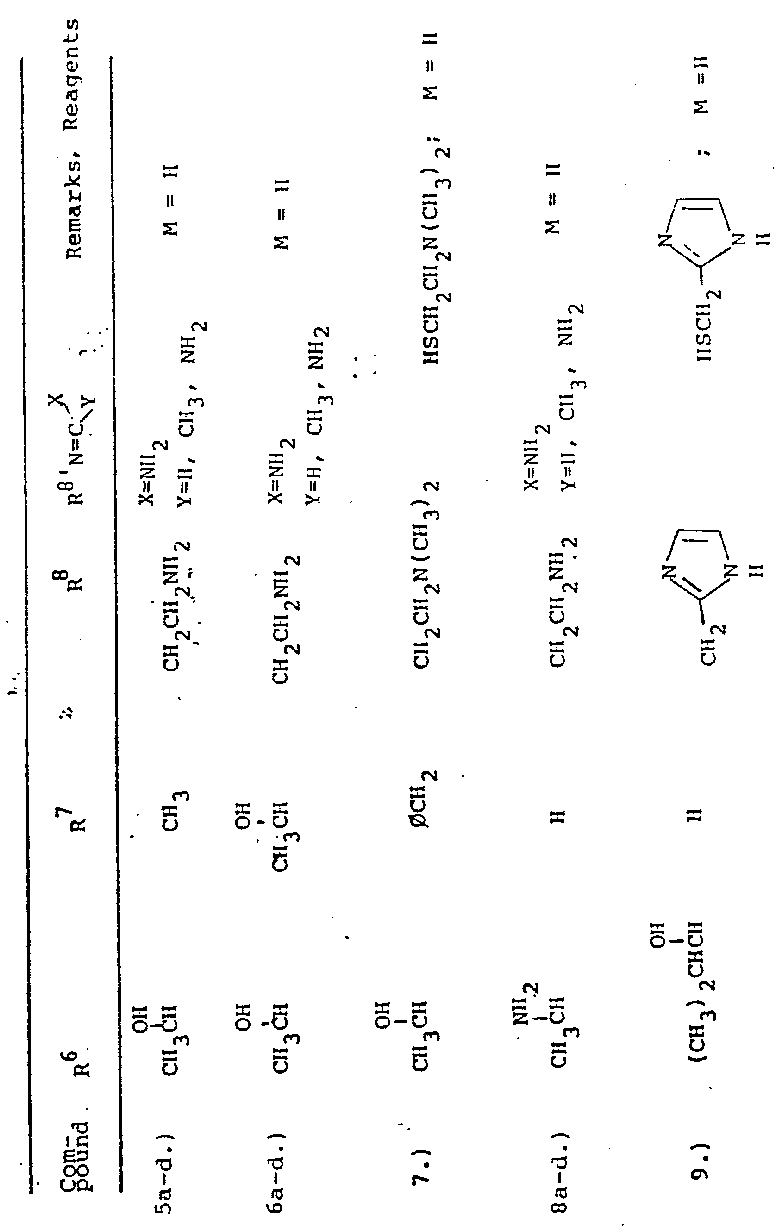

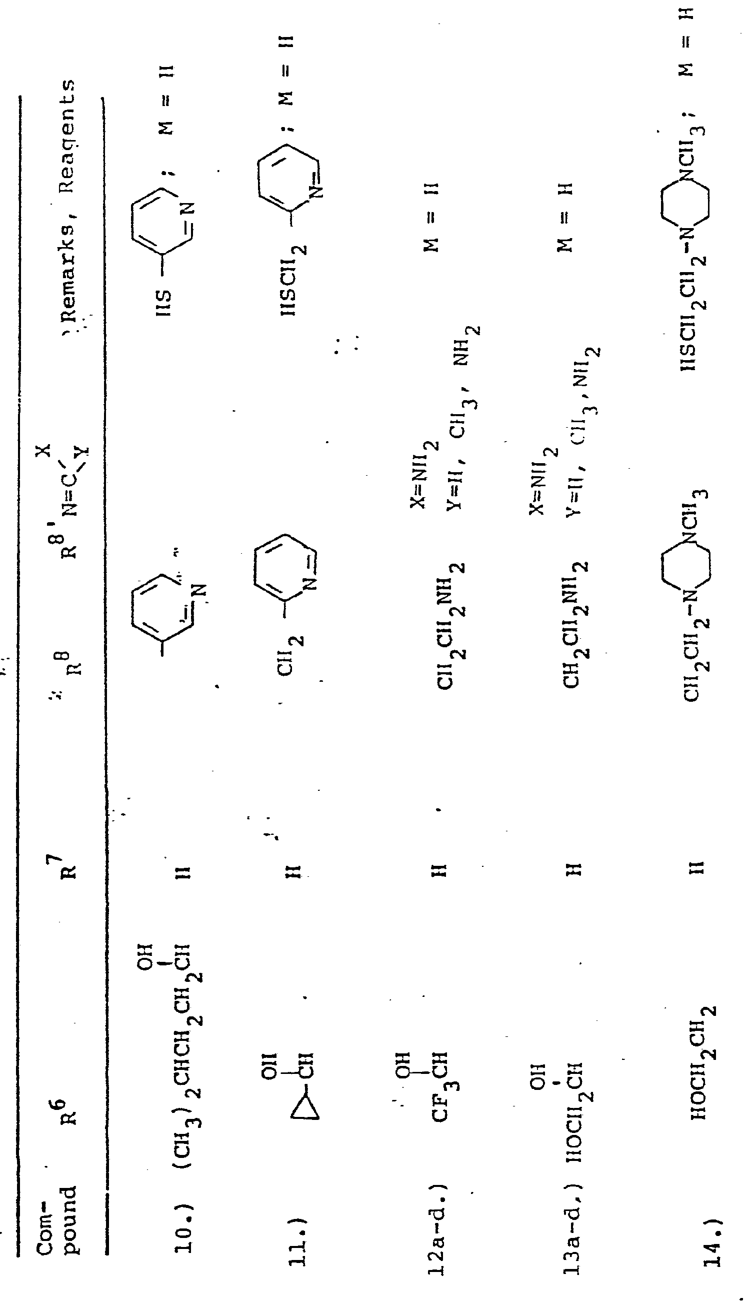

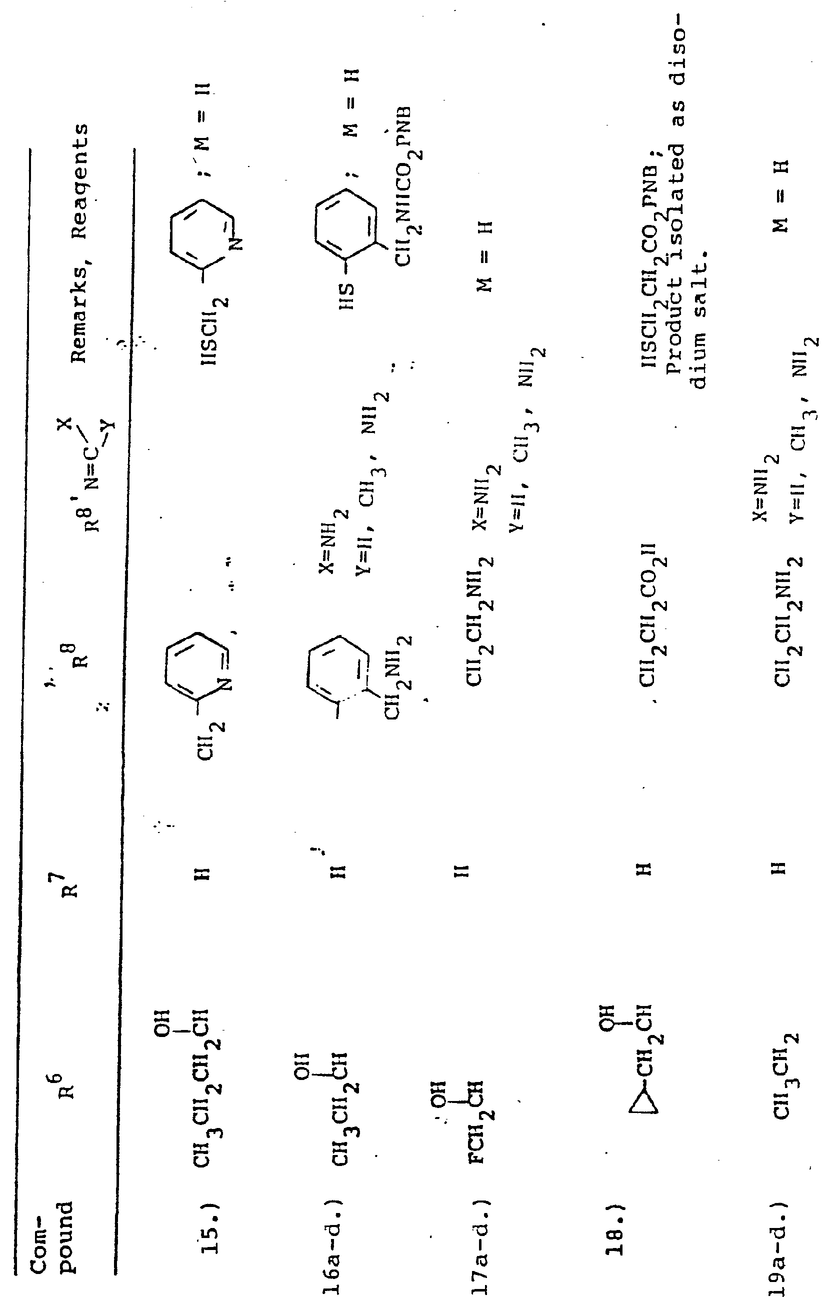

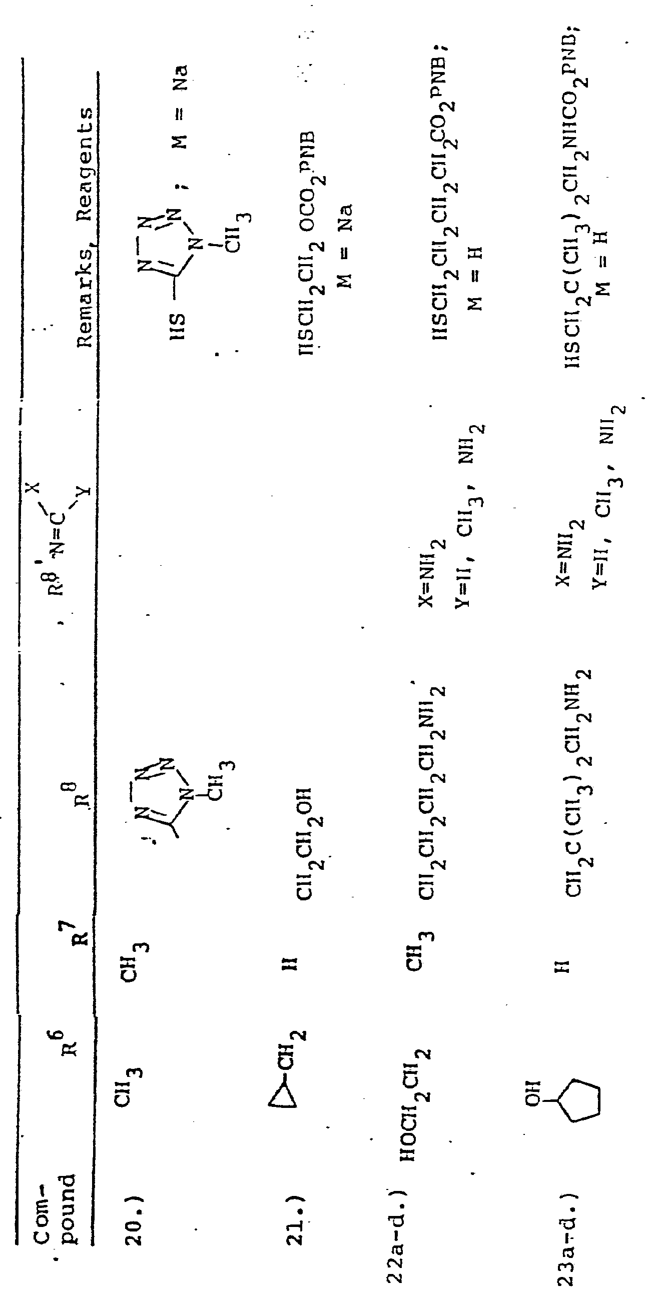

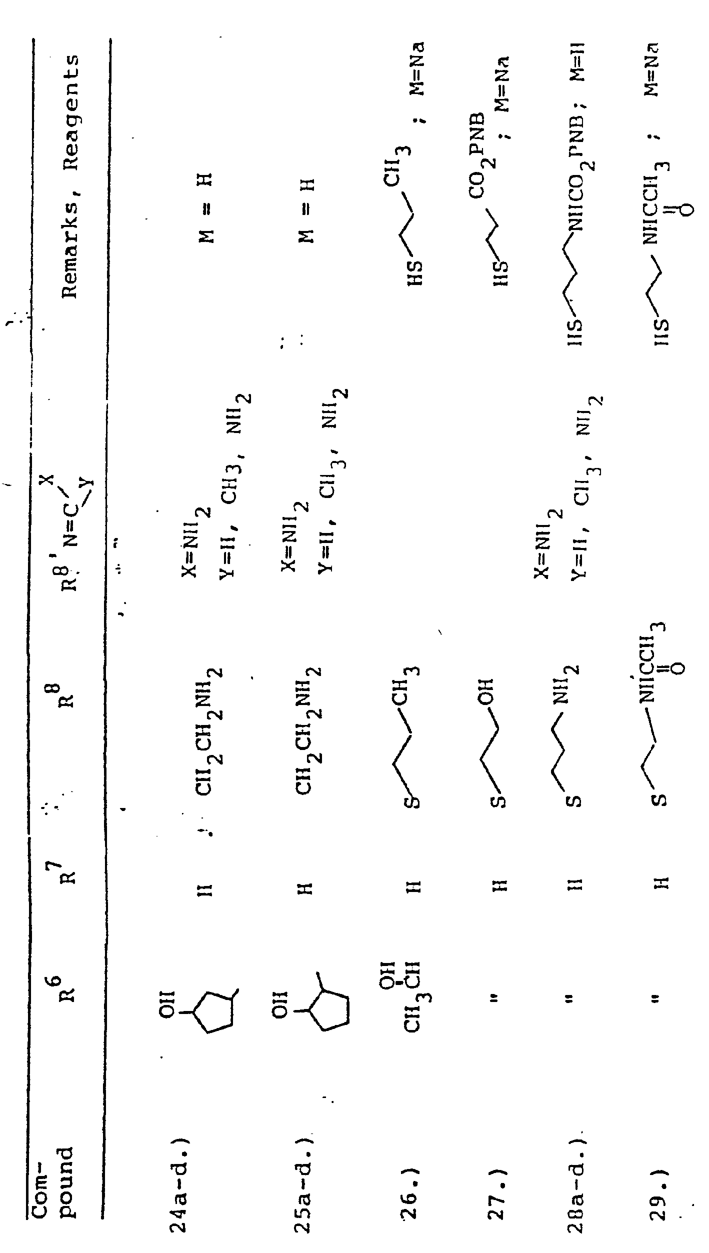

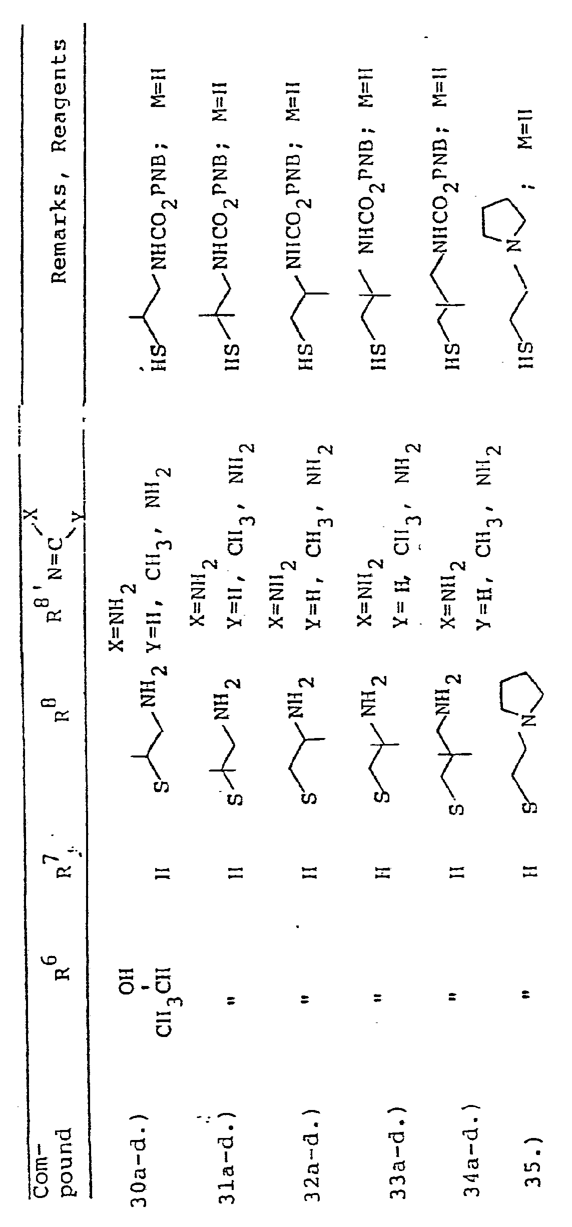

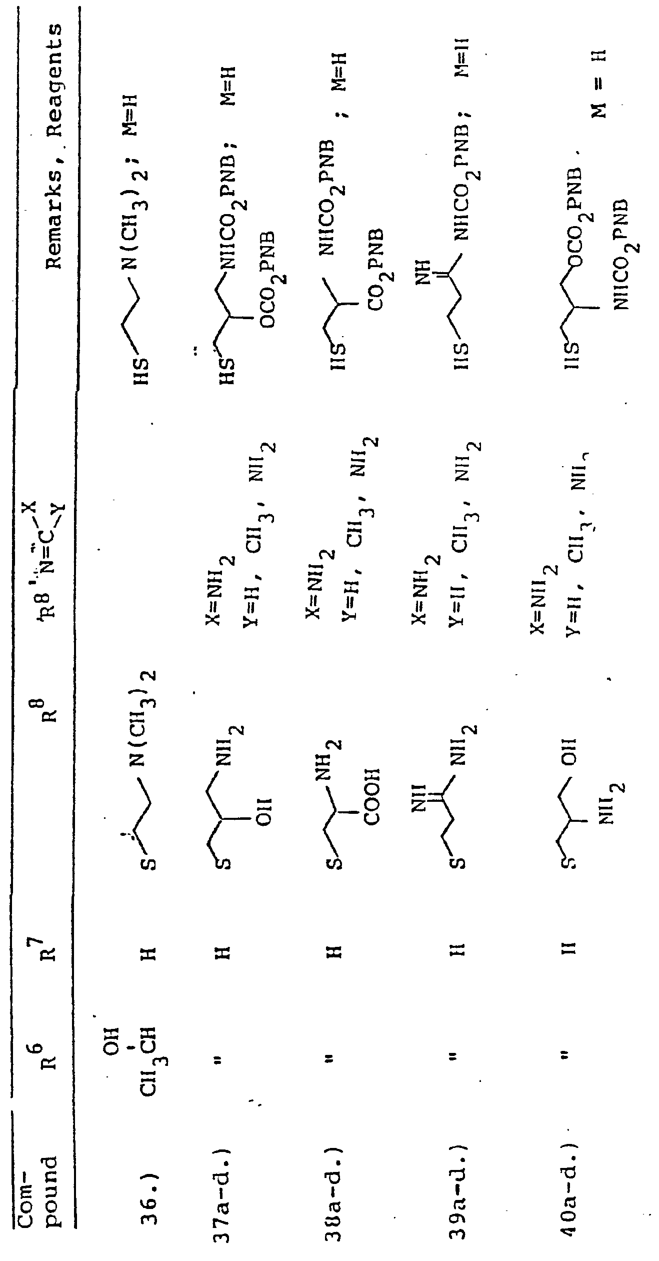

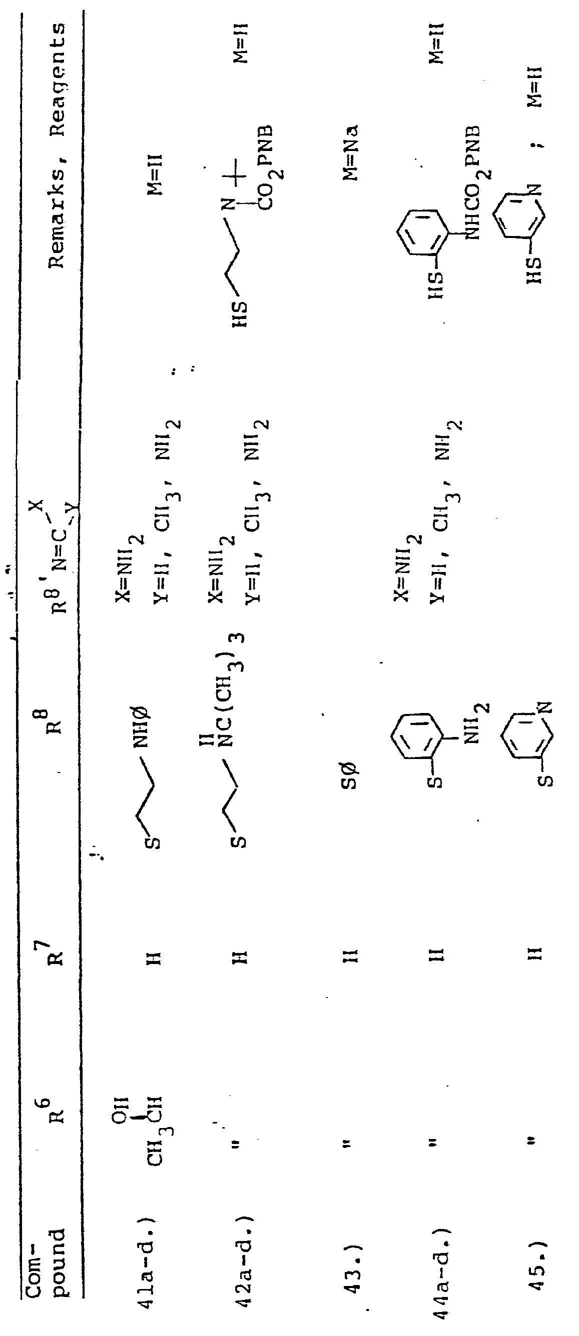

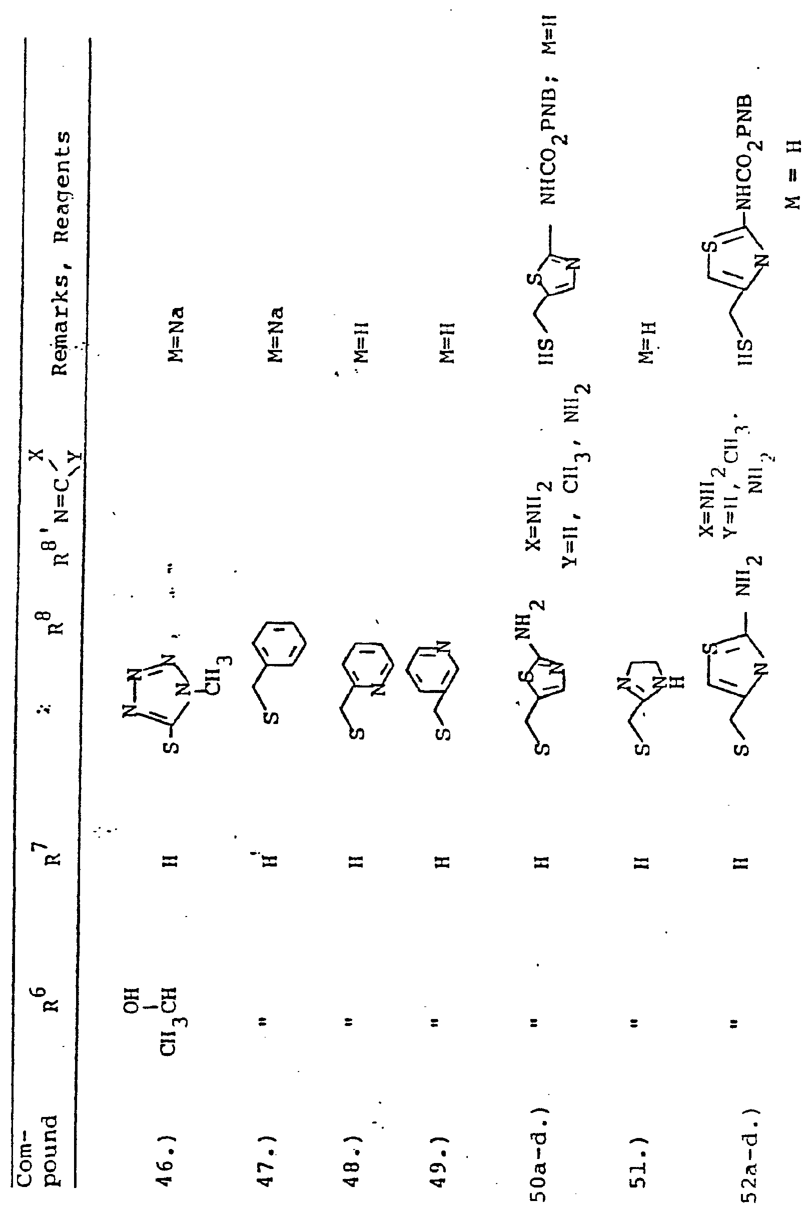

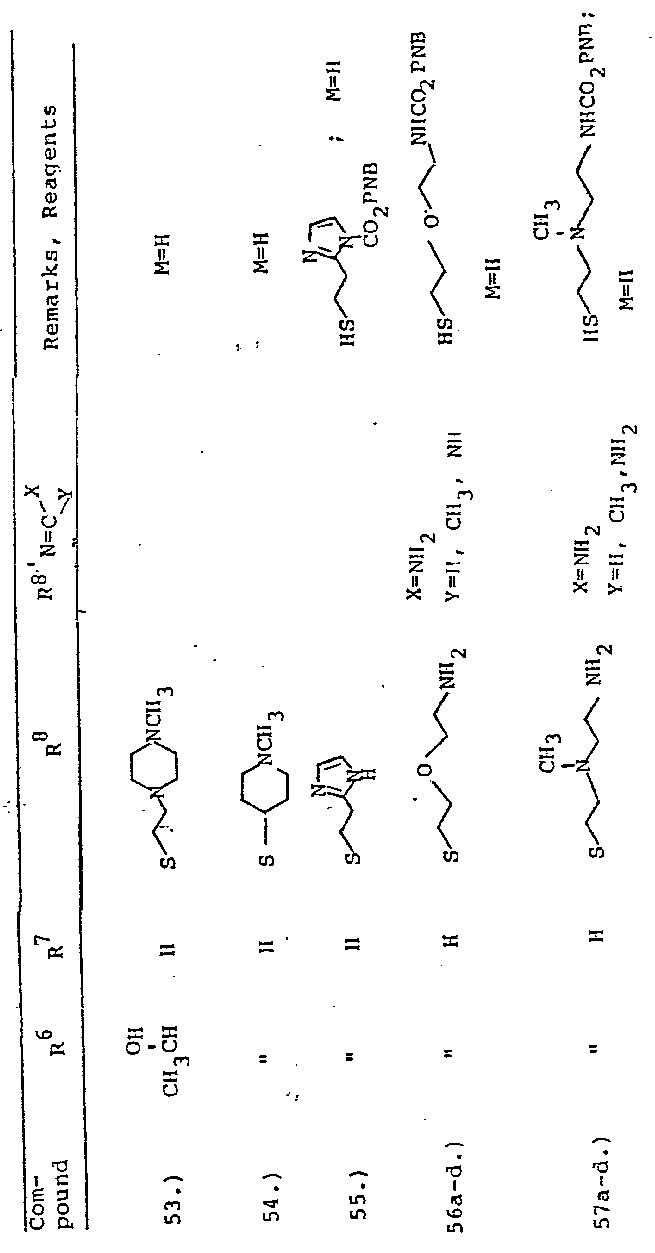

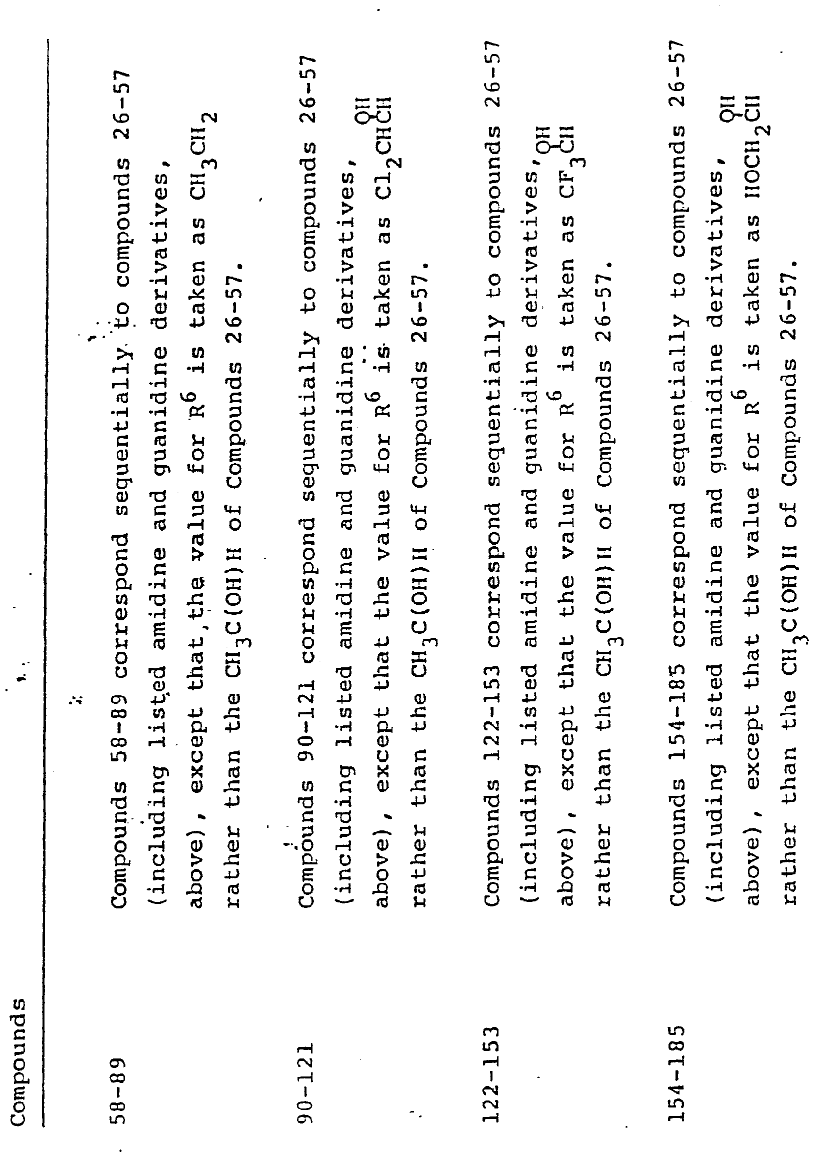

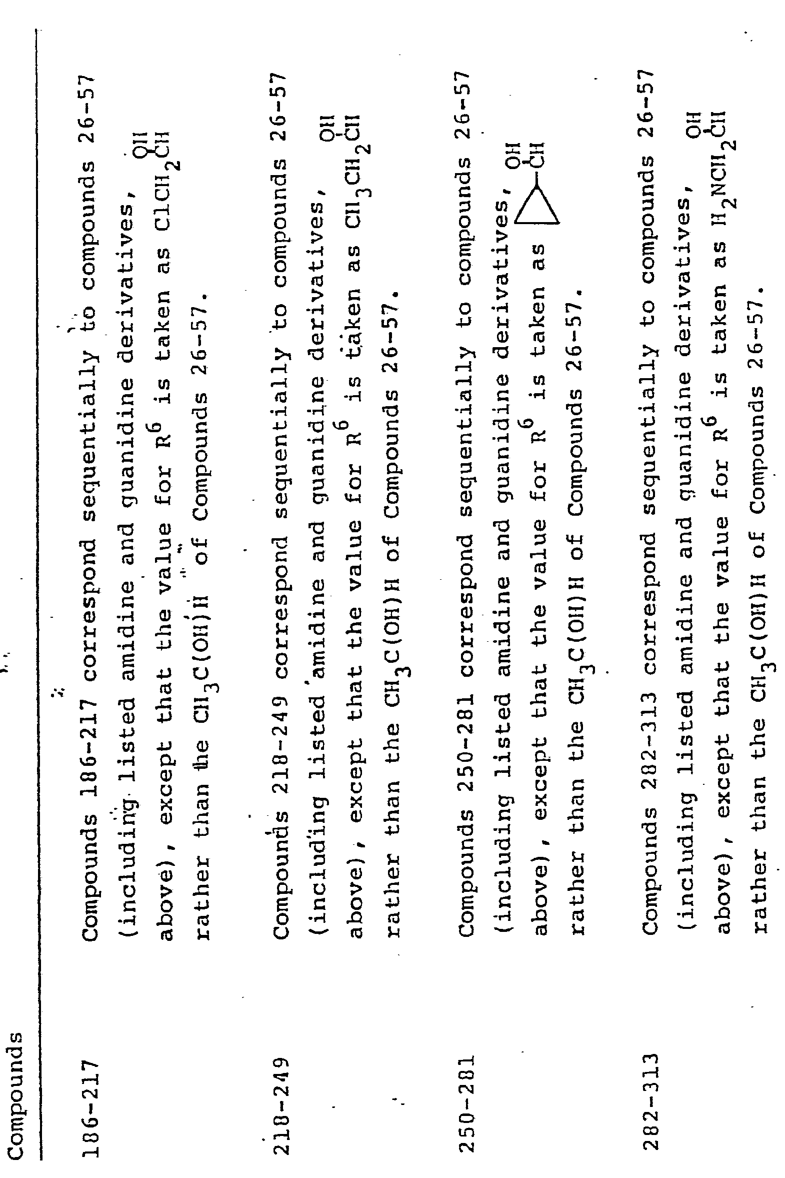

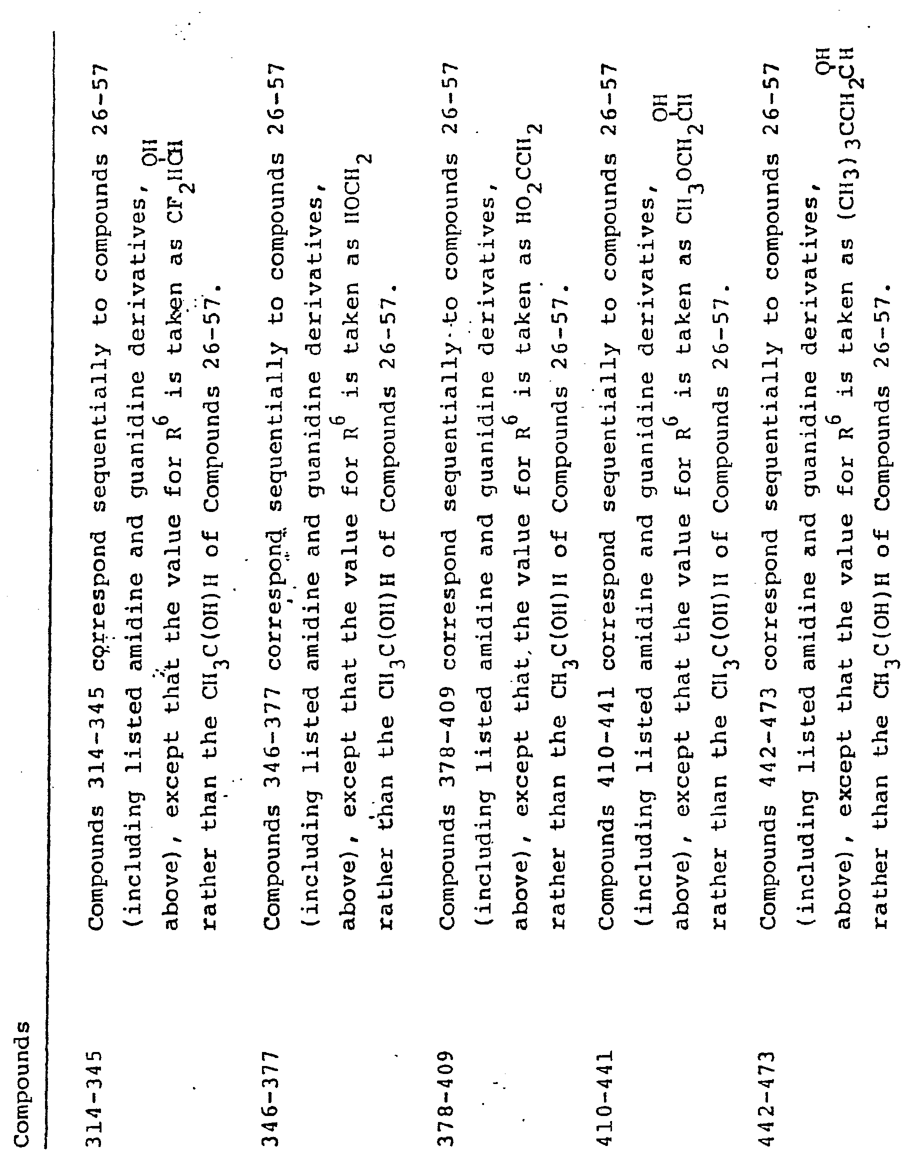

The following compounds are examples for endproducts which can be prepared from the compounds of the invention with corresponding substituents R⁶, R⁷ and R⁸. 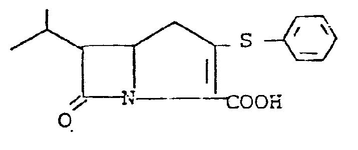 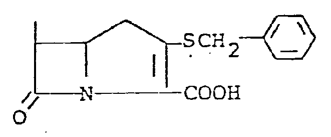 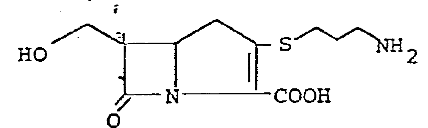 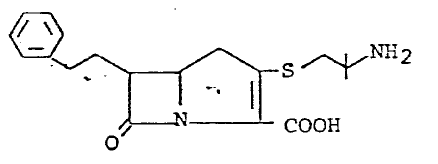 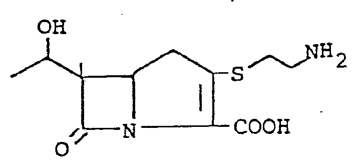 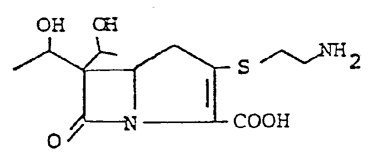 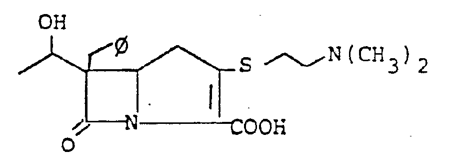 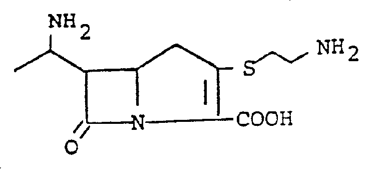 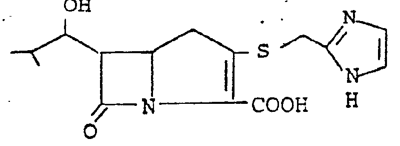 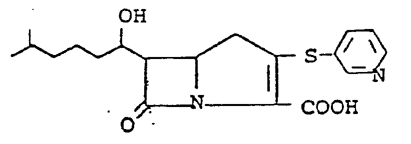 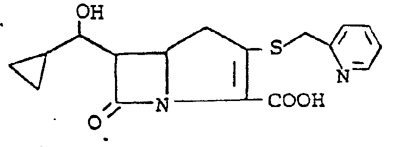 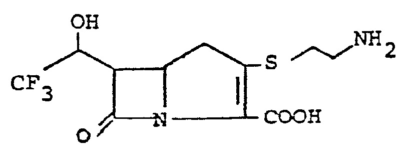 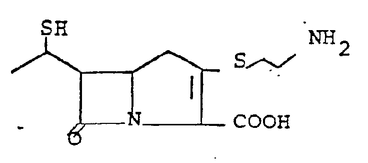 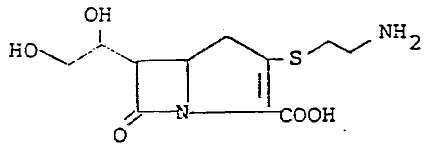 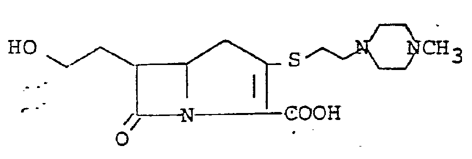 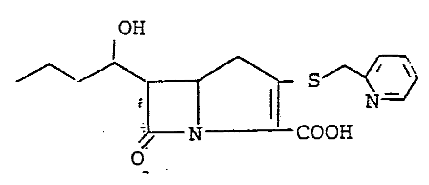 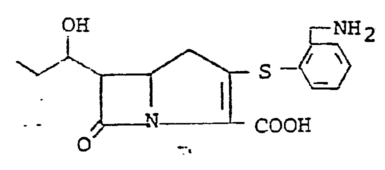 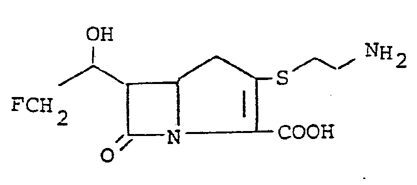 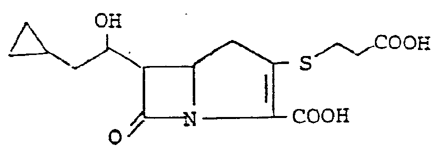 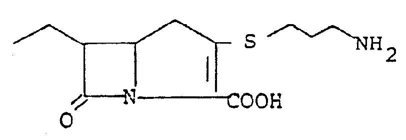 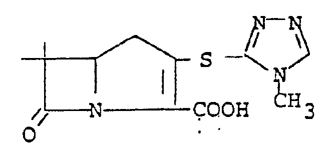 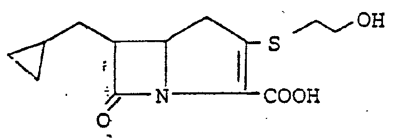 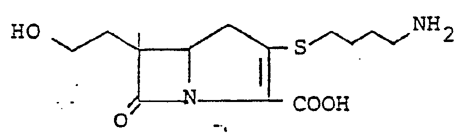 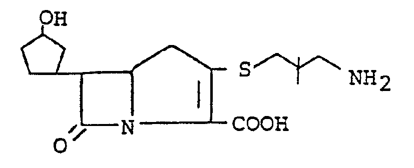 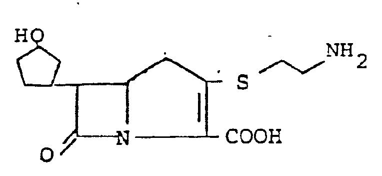 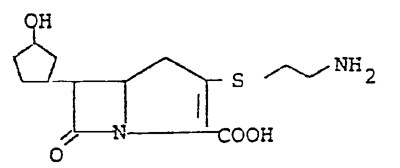 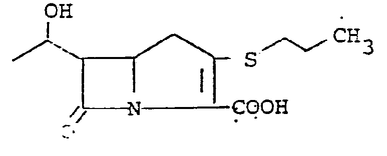 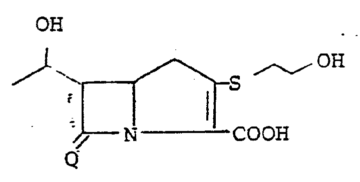 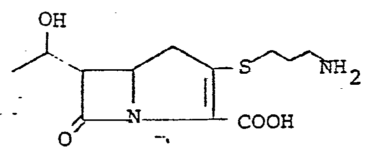 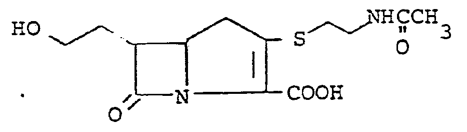 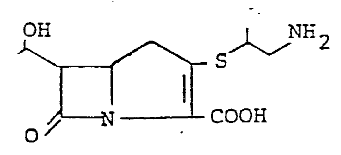 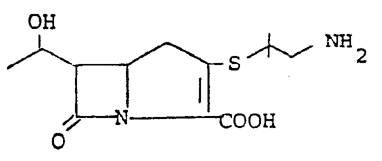 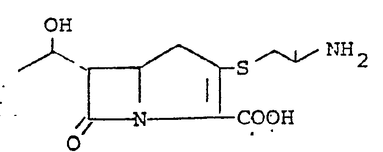 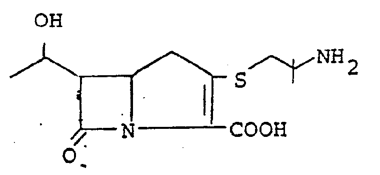 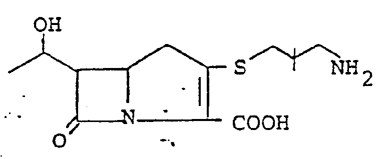 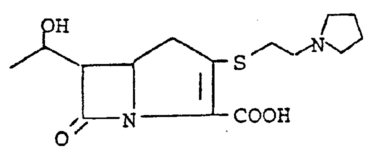 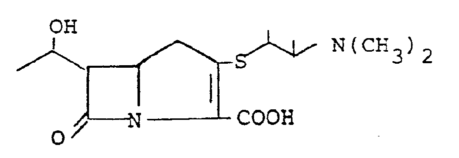 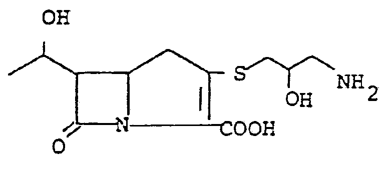 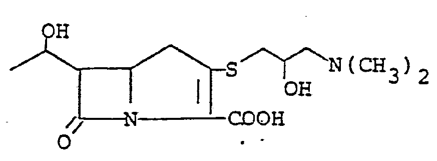 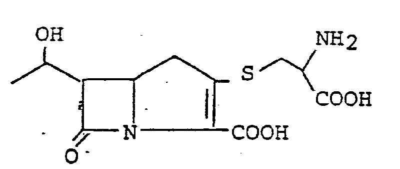 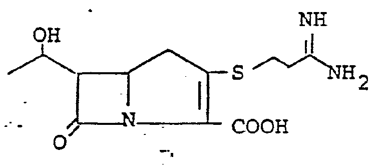 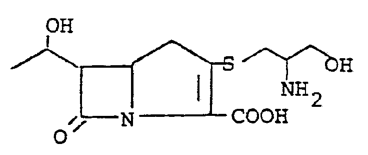 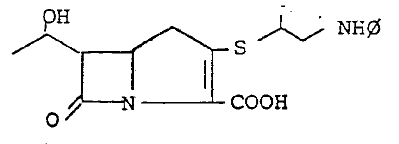 ⌀ = phenyl 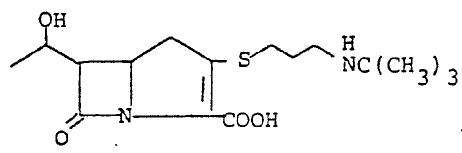 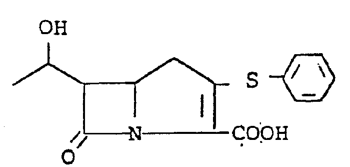 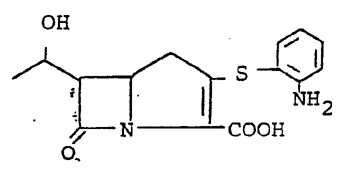 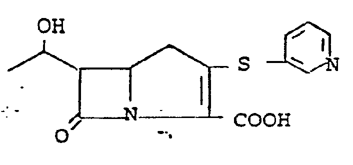 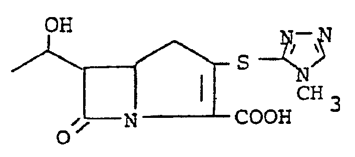 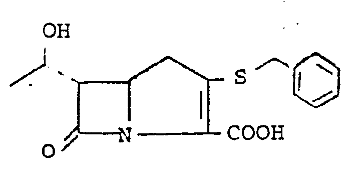 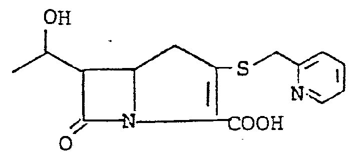 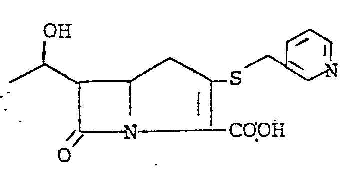 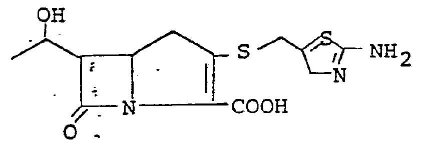 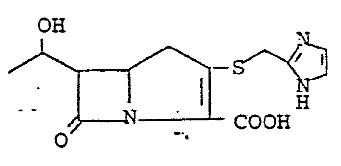 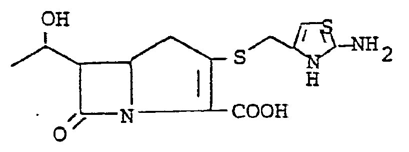 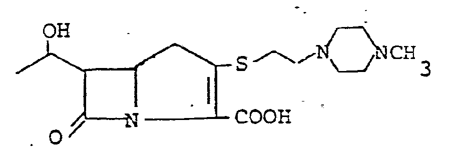 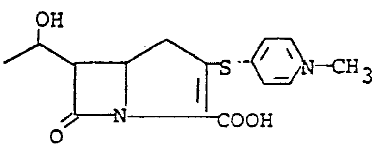 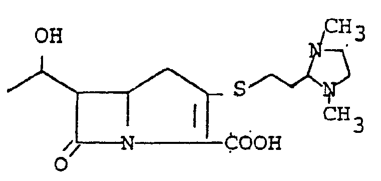 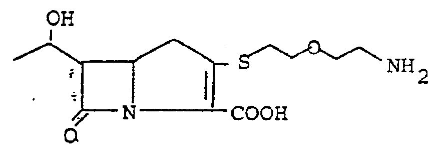 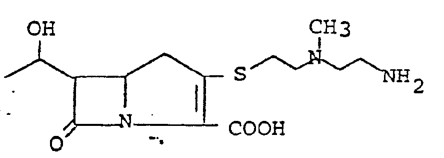 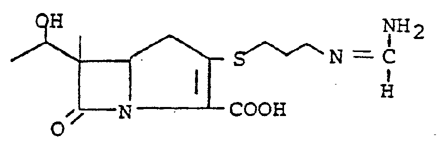 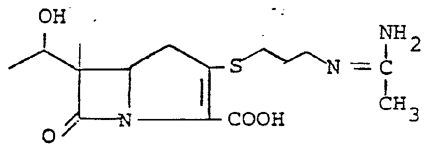 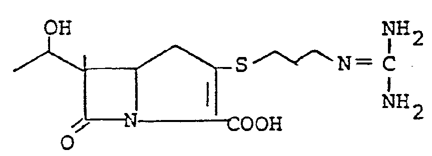 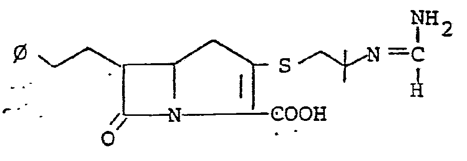 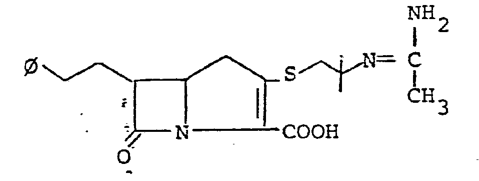 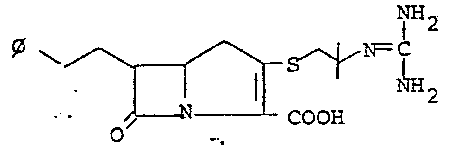 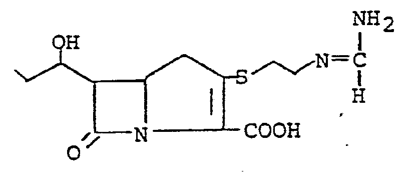 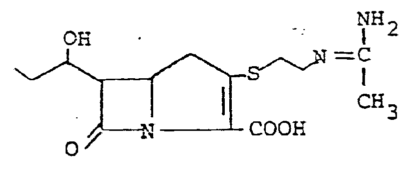 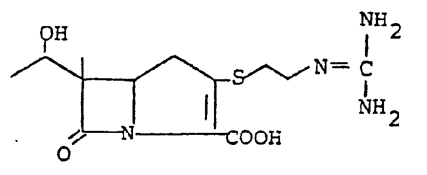 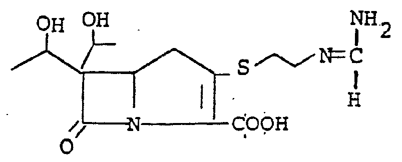 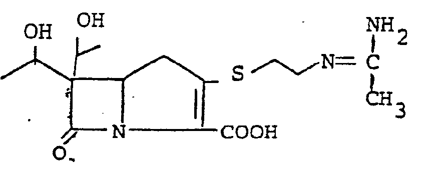 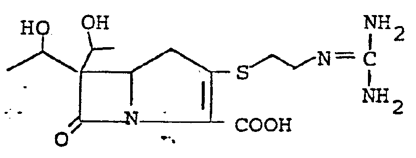 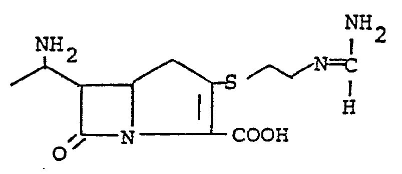 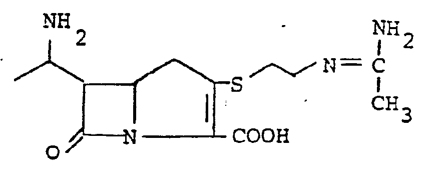 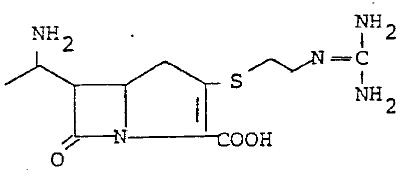 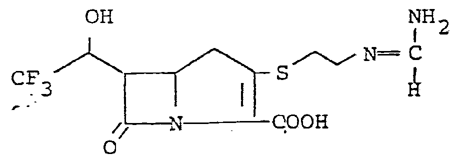 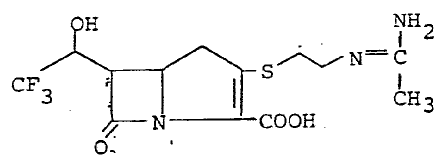 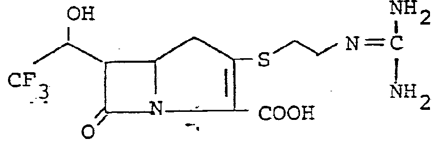 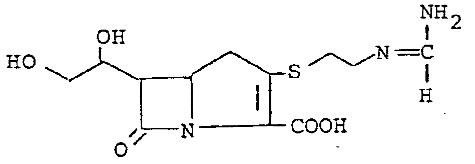 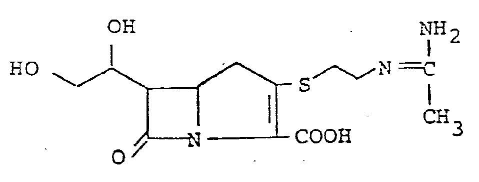 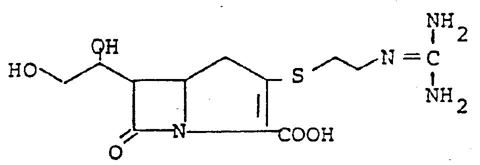 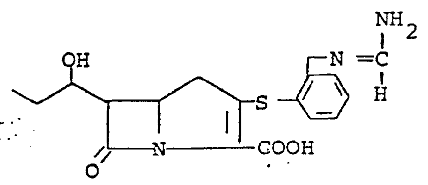 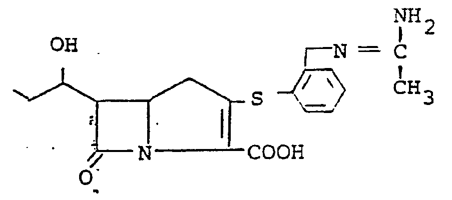 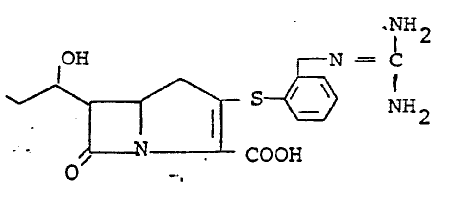 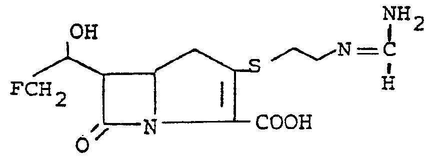 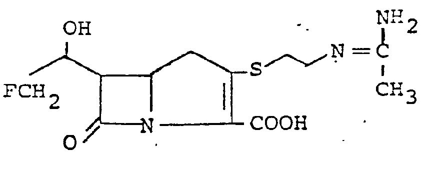 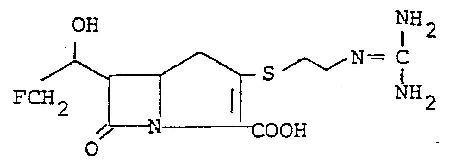 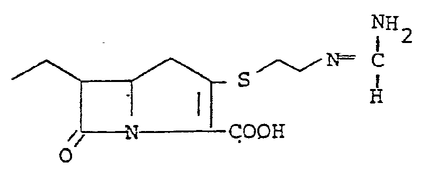 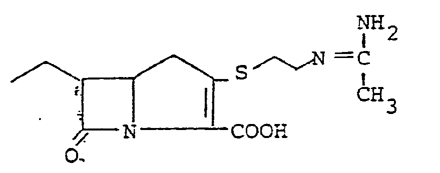 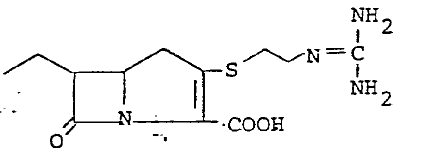 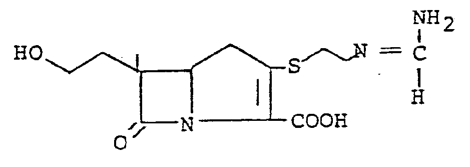 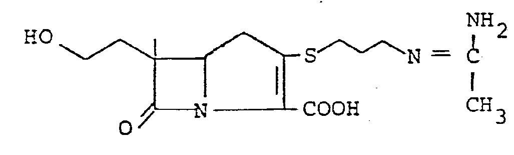 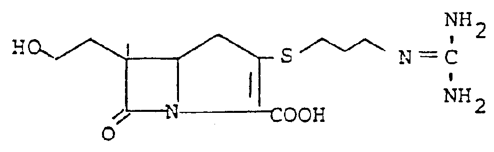 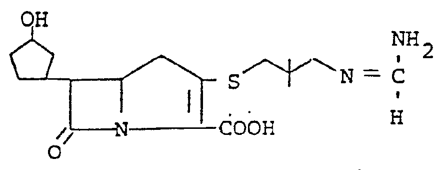 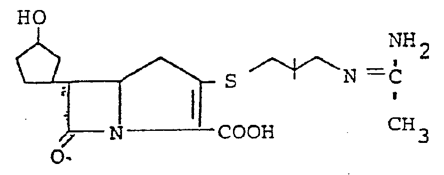 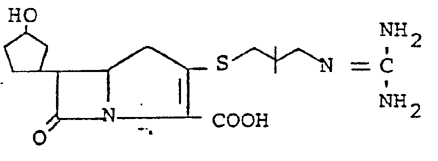 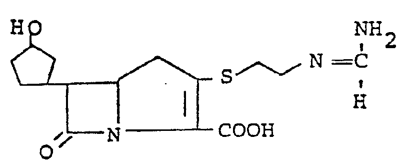 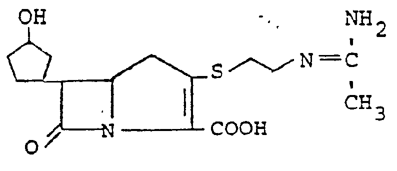 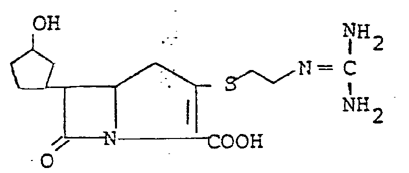 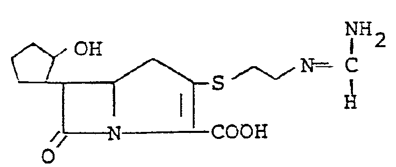 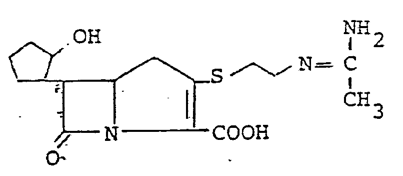 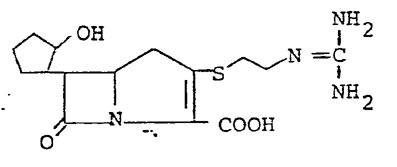 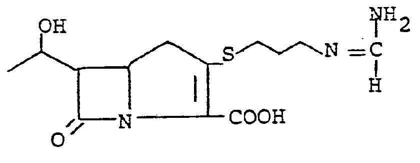 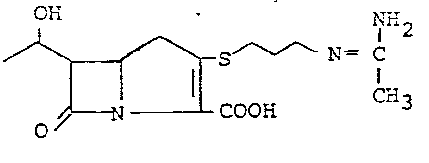 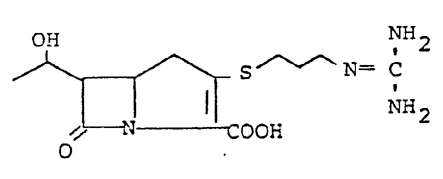 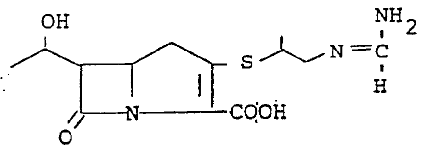 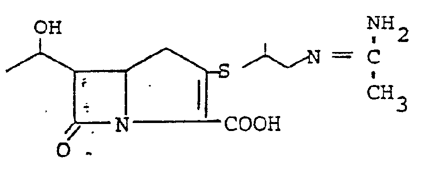 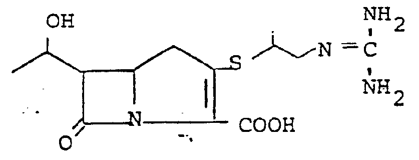 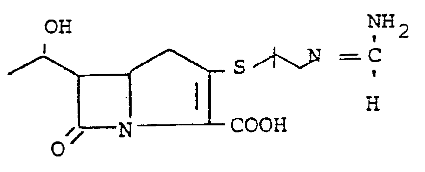 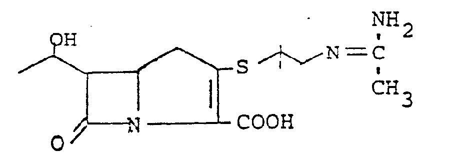 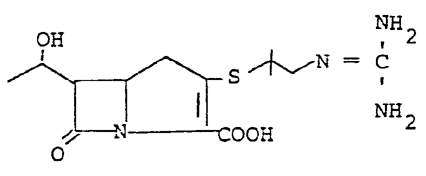 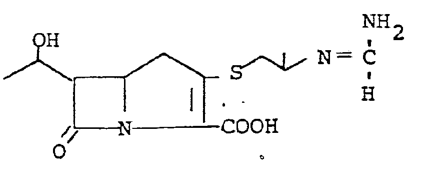 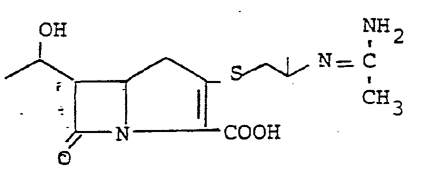 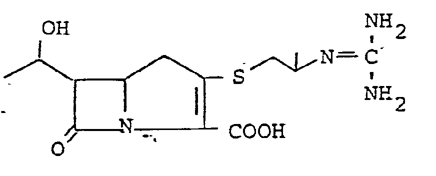 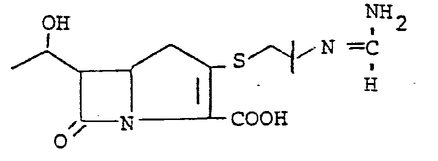 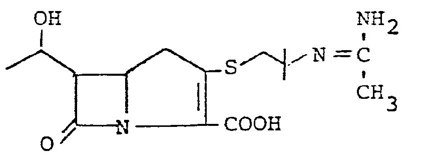 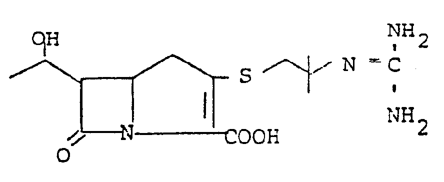 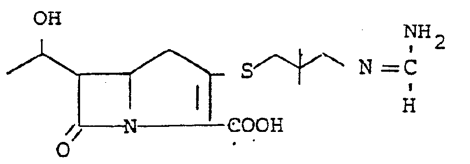 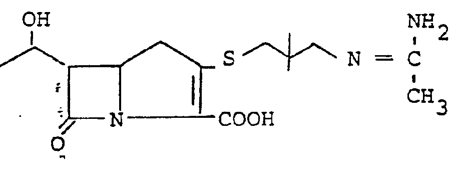 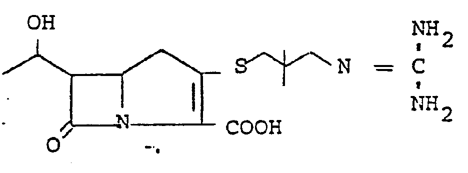 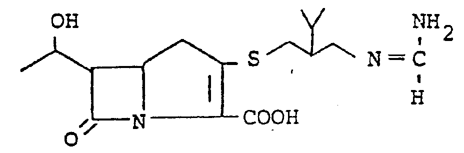 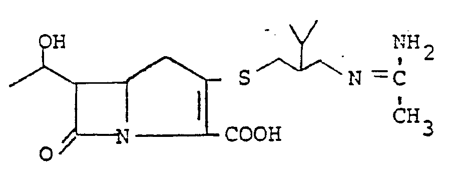 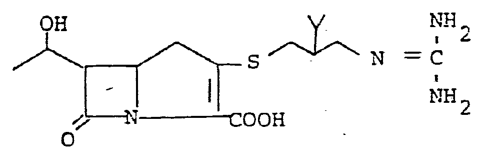 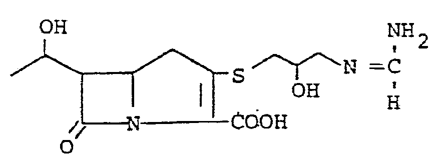 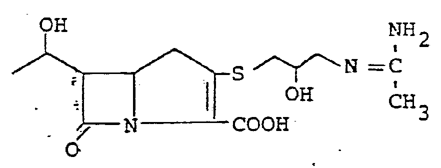 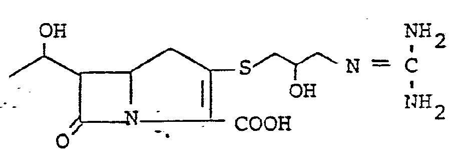 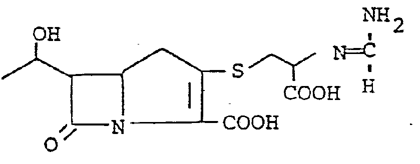 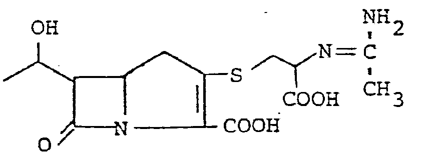 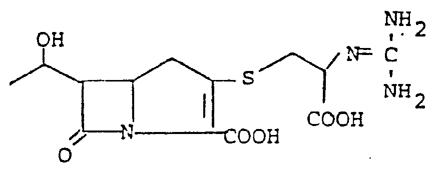 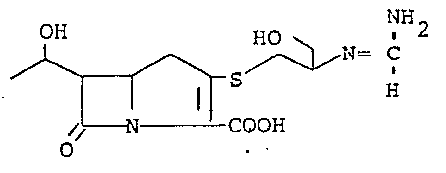 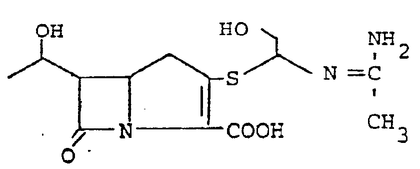 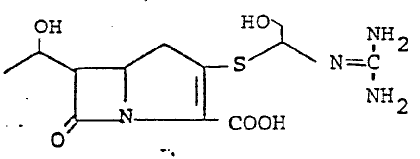 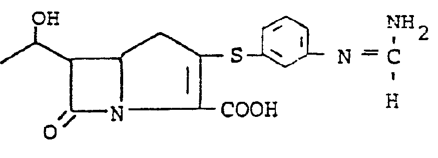 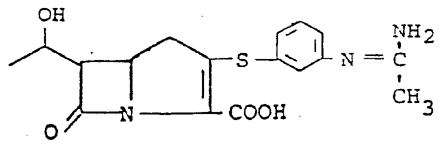 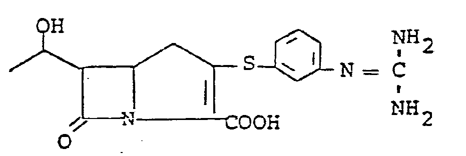 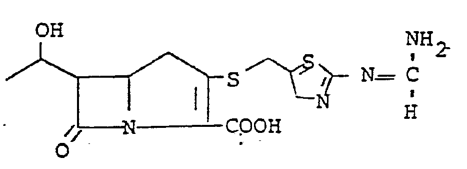 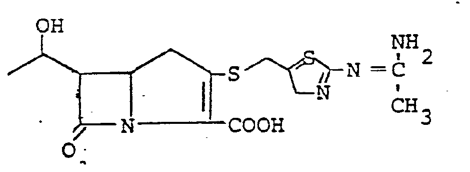 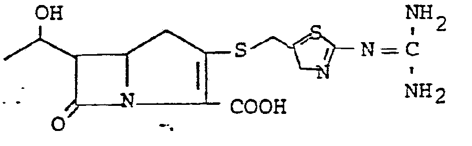 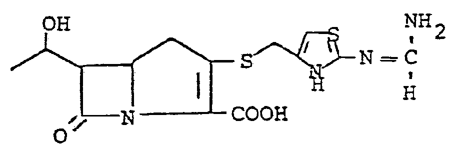 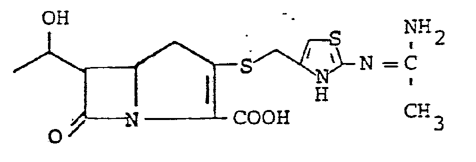 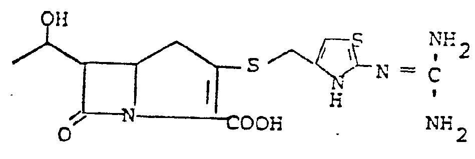 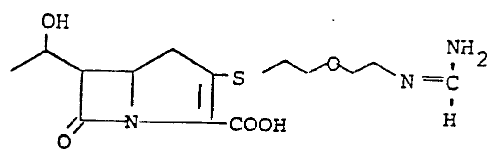 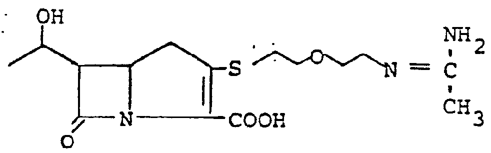 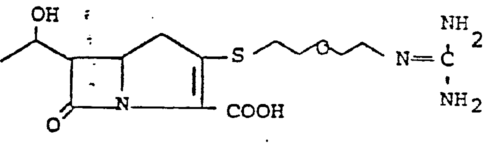 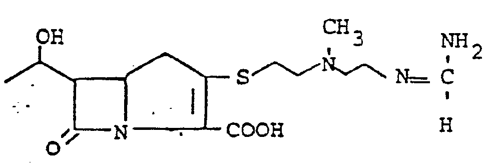 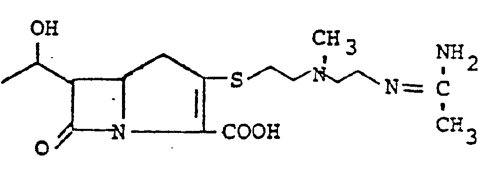 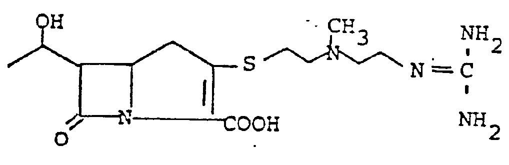 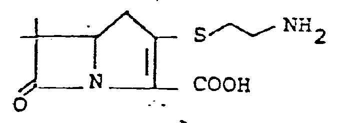 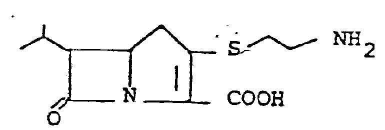 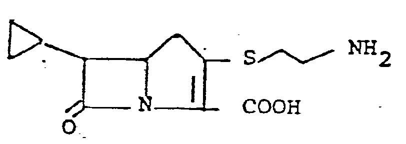 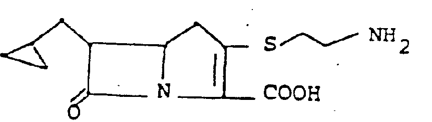 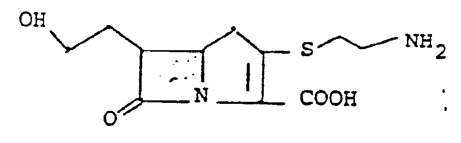 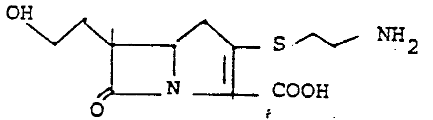 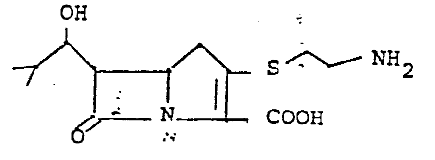 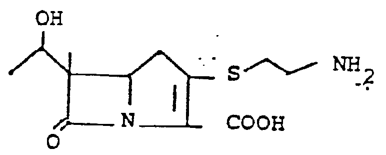 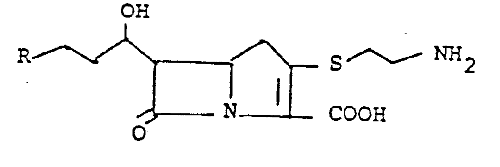
(R = phenyl, m-aminomethylphenyl,
o-, p-, m-hydroxyphenyl)

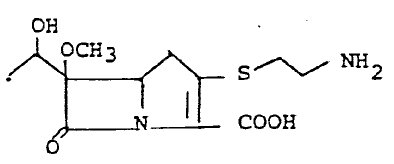

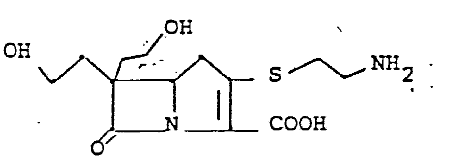

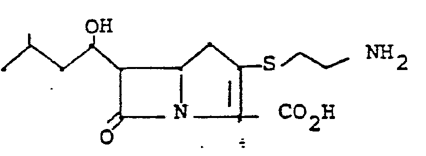

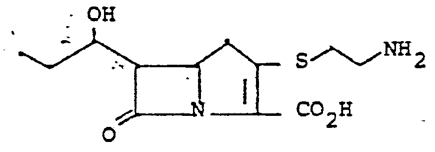

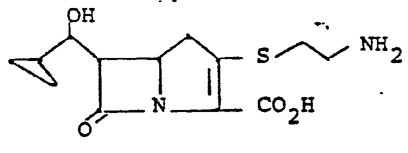

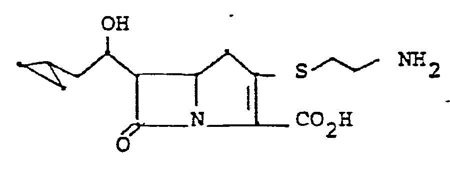

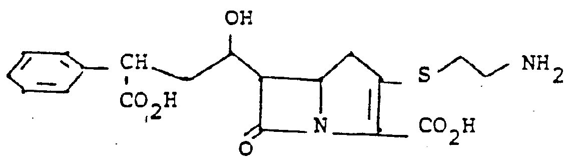

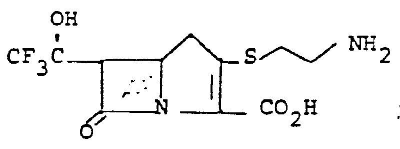

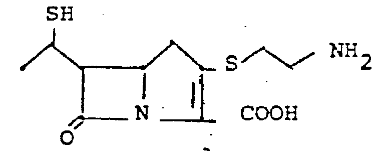

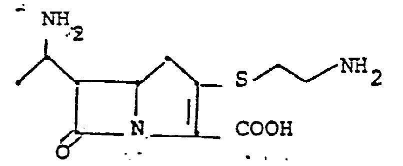

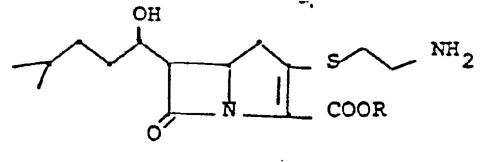

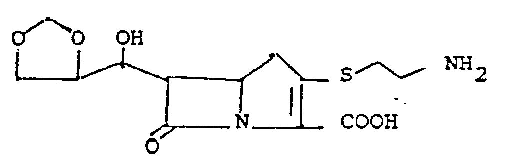

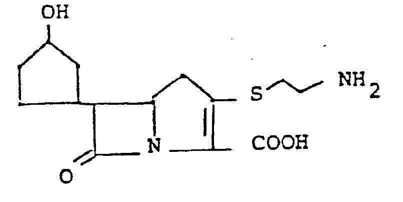

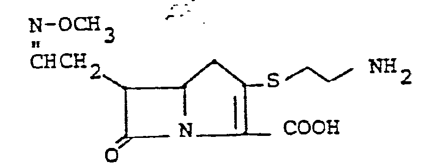

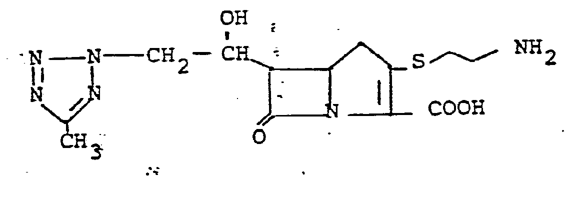

## Claims

1. A process for preparing a compound of the formula: 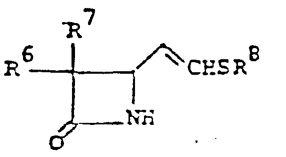 wherein R⁶, R⁷, and R⁸ are independently selected from the group consisting of: hydrogen; substituted and unsubstituted: alkyl, alkenyl, and alkynyl, having from 1-10 carbon atoms; cycloalkyl, cycloalkyl- alkyl, and alkylcycloalkyl, having 3-6 carbon atoms in the cycloalkyl ring and 1-6 carbon atoms in the alkyl moieties; phenyl, aralkyl, aralkenyl, and aralkynyl wherein the.aryl moiety is phenyl and the aliphatic portion has 1-6 carbon atoms; heteroaryl, heteroaralkyl, heterocyclyl and heterocyclylalkyl; wherein the substituent or substituents relative to the above-named radicals are selected from the group consisting of: chloro, bromo, fluoro, R¹,
-OH
-OR¹ 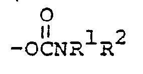 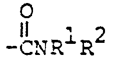
-NR¹R²
-N^{H}2
-NHR¹ 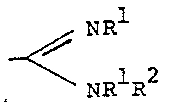
-SO₂NR¹R² 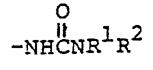 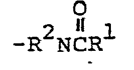
-CO₂H
-CO₂ R¹ 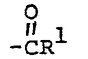 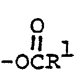
-SH 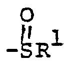 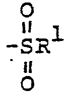
-CN
-N₃
-NO₂ 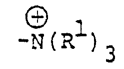 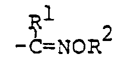
-SR¹ ;
wherein, relative to the above listed substituents on R⁶, Rand R⁸, the groups R¹ and R are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl; and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms; _{R}⁸ is selected from: 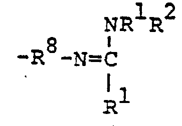 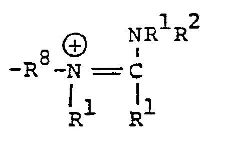 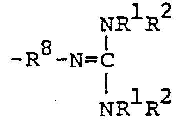 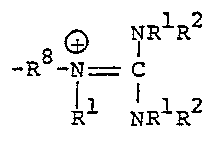 wherein: R⁸, R¹ and R² are as defined; when _{R}⁶/_{R}⁷ is hydrogen R⁷/R⁶ is not: hydrogen; CH₃CH₂; or 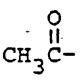 when R⁸ is -SCH₂CH₂NH₂; or when R⁶/R⁷ is hydrogen R⁷/R⁶ is not CH₃CH (OH), or O-derivative thereof, when R⁸ is -SCH₂CH₂NH₂ or N-derivative thereof, comprising reacting: 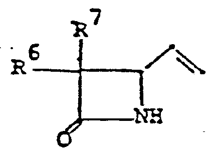 with XSR⁸ to yield 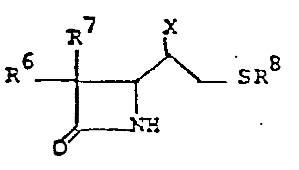 followed by elimination of HX; wherein X is halo.

2. A rrocess according to Claim 1, wherein R⁷ is selected from H, OCH₃ and CH₃.

3. A process according to Claim 2 wherein R⁶ is selected from the group consisting of: substituted and unsubstituted: alkyl, alkenyl and cycloalkylalkyl wherein the substituent or substituents are selected from hydroxyl, alkoxyl having from 1-6 carbon atoms, phenoxy, amino, and carboxy.

4. A process according to Claims 2 or 3 wherein R⁶ is selected from the group consisting of alkyl, cycloalkylalkyl, alkyl substituted by one or more hydroxyl groups, or cycloalkylalkyl substituted by one or more hydroxyl groups.

5. A process according to Claims 2, 3 or 4 wherein R is hydrogen.

6. A process according to Claims 1, 2, 3, 4, or 5 wherein R⁶ is selected from: 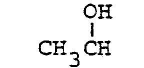 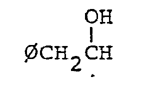 ⌀= phenyl 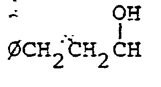 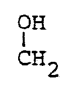
⌀CH₂ 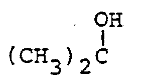
CH₂ =CHCH₂
(CH₃)₂ C=CHCH₂ 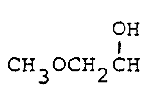
^{CH}3
CH₃^{CH}₂
(CH₃)₂CH 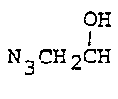 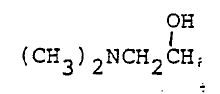
HO₂CCH₂ 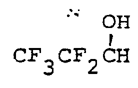 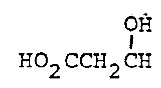 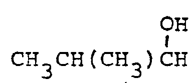 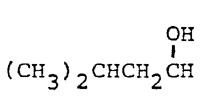 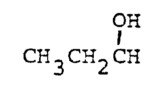
HOCH₂CH₂ 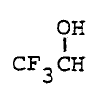 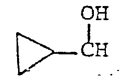 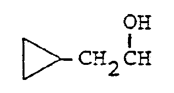 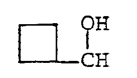 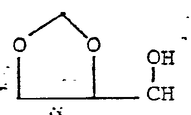
HOCH₂^{CH}₂^{CH}₂

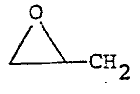

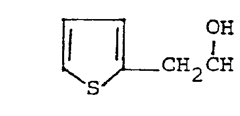

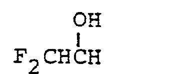

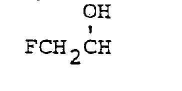

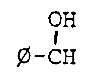

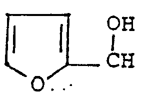

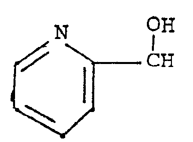

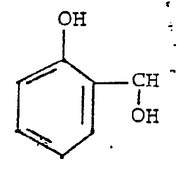

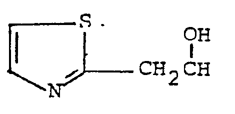

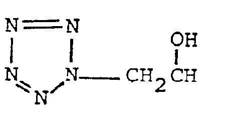

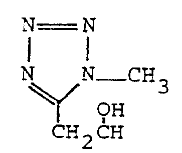

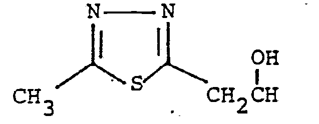

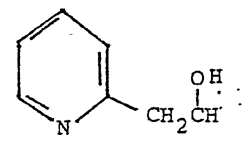

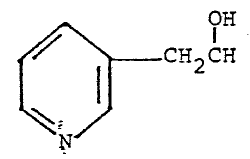

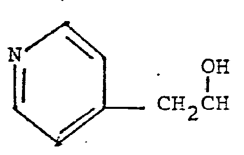

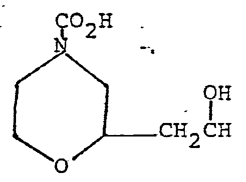

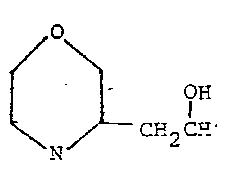

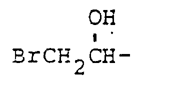

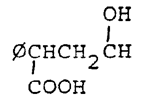

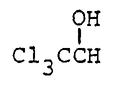

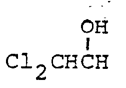

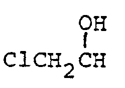

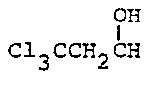

7. A process according to Claims 1, 2, 3, 4, 5, or 6 wherein R⁶ is:
CH₃CH₂ 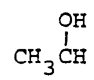 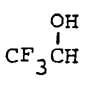 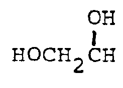 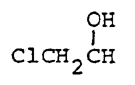 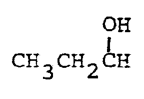 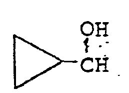 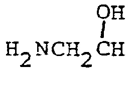 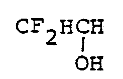
HOCH₂
HO₂ CCH₂

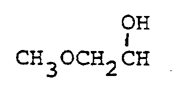

8. A process according to Claims 1, 2, 3, 4, 5, 6 or 7 wherein R⁸ is selected from: 1.) aliphatic (including carbocyclic) groups having 1-10 carbon atoms selected from: alkyl, cycloalkyl, alkenyl, cycloalkenyl and alkynyl; 2.) substituted aliphatic groups having 1-10 carbon atoms selected from: alkyl, cycloalkyl, alkenyl, cycloalkenyl and alkynyl; wherein the substituents are selected from: chloro, bromo, fluoro, R¹,
-OH
-OR¹ 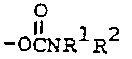 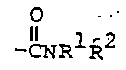
-NR¹R²
-^{NH}2
-NHR¹ 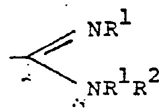
-SO₂NR¹R² 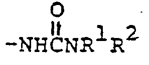 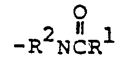
-CO₂H
-CO₂R¹ 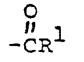 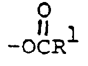
-SH 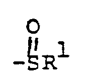 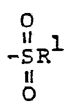
-CN
-N₃
-NO₂ 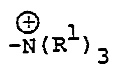 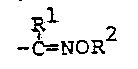
-SR¹ 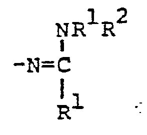 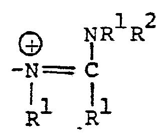 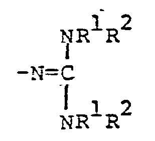 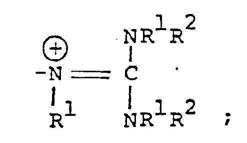
3.) aryl and substituted aryl; wherein the aryl is phenyl and wherein the substituents are defined above under 2.);
4.) heteroaryl and substituted heteroaryl having 1-4 O, N or S atoms; and wherein the substituents are defined above under 2.);
5.) arylaliphatic, wherein aryl is phenyl, which are selected from the aliphatic groups defined under 1.) which are substituted by phenyl or 'substituted phenyl; wherein such substituents on phenyl are defined under 2.), above;
6.) heteroarylaliphatic and heterocyclylaliphatic; wherein the aliphatic moiety is defined under 1.), above; the substituted and unsubstituted heteroaryl and heterocyclic moieties have 1-4 O, N or S atoms; wherein such substituents are defined under 2.),above;
7.) substituted and unsubstituted alkyl-heteroatom- alkyl having 4-12 carbon atoms; wherein the heteroatom is selected from: O, S, NR° (R° is H, substituted or unsubstituted alkyl); wherein such substituents are defined under 2.),above.

9. A process according to Claim 8, subparagraph 2.); wherein: 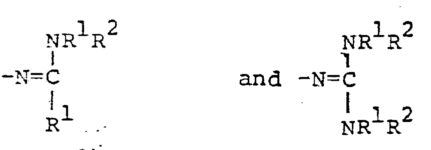 are selected from:

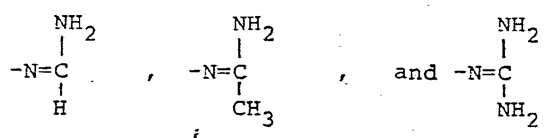

10. A process according to Claim 8, subparagraph 3.) ; wherein: 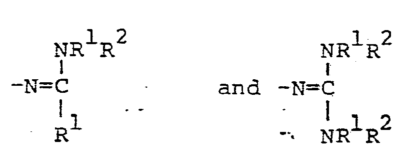 are selected from:

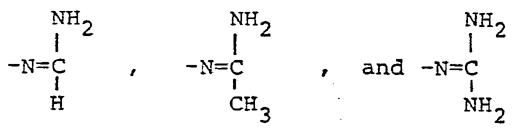

11. A process according to Claim 8, subparagraph 4.); wherein: 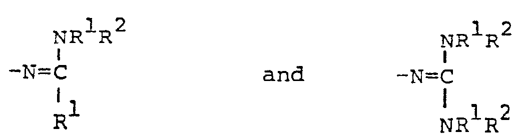 are selected from:

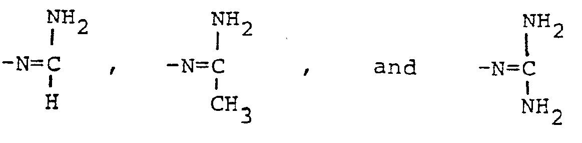

12. A process according to Claim 8, subparagraph 5.) ; wherein: 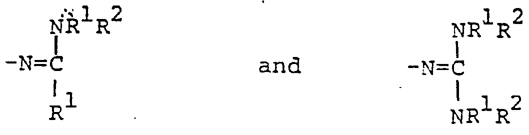 are selected from:

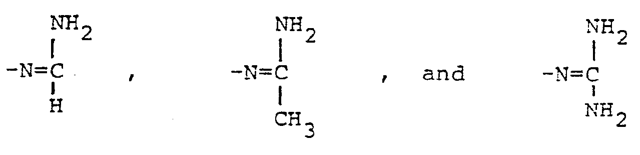

13. A process according to Claim 8, subparagraph 6.); wherein: 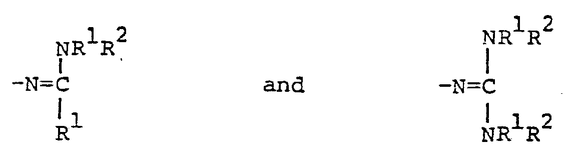 are selected from:

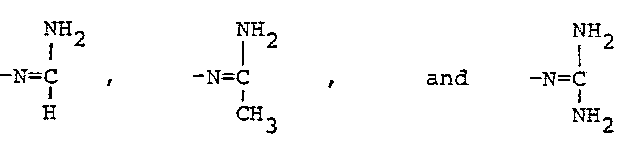

14. A process according to Claim 8, subparagraph 7.) ; wherein: 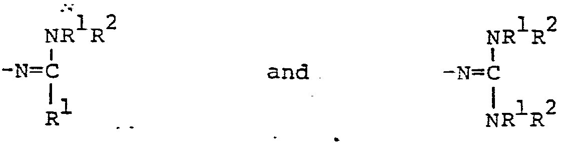 are selected from:

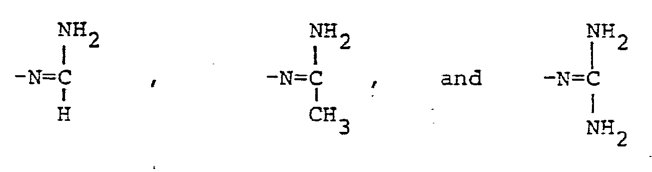

15. A process according to Claim 8, subparagraph 1.); wherein R⁸ is selected from:
-CH₃
-CH₂CH₃
- CH₂CH₂CH₃
-CH(CH₃)₂
- (CH₂)₃CH₃ 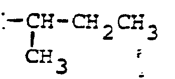
-CH₂CH(CH₃)₂ 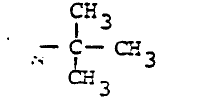 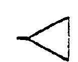 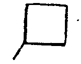 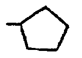 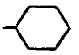 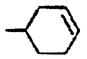 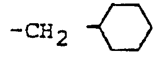
-CH₂-CH⁼CH₂
-CH₂-CH=C(CH₃)₂
-CH₂-C=CH
-CH₂-C-=C-CH₃

16. A process according to Claim 8, subparagraph 2.); wherein R⁸ is selected from: -(CH₂)ₙOR¹ ; n ⁼ 2-⁶; R¹ = H, 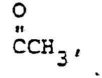
C^{H}3 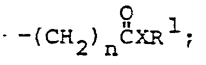 n=1-6 ; X=0, NH, NR¹, R¹=H , CH₃ -(CH₂)ₙNH₂ ; n=2-6
- (CH₂)ₙNHR¹ ; n=2-6; R¹CH₃, CH₂CH₃, CH₂CH₂CH₃ , 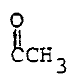
-(CH₂)ₙNR¹R² ; n=2-6; R¹/R²=CH₃, CH₂CH₃; CH₃, CH₃; CH₂CH₃, CH₂CH₃ 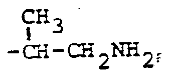 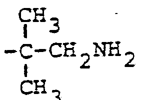 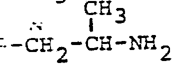 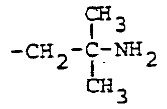
-CH₂CH₂^{SCH}₃
-CH₂CH₂NHC(CH₃)₃ 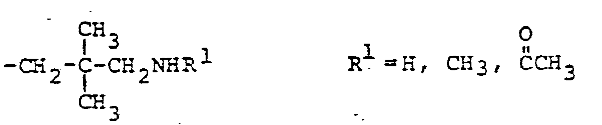 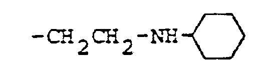 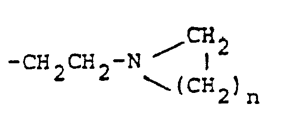
n=3-5 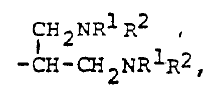
R¹ and R² are independently chosen from H and CH₃ 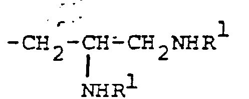
R¹=H , CH₃ 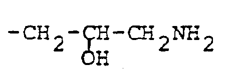 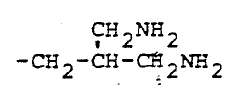 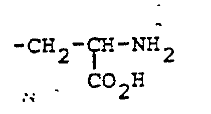 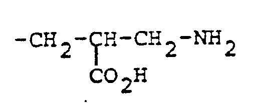 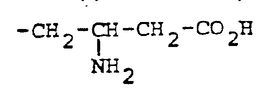 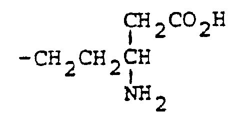 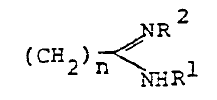
n=1, R²/R¹=H,H; CH₃,H 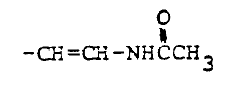 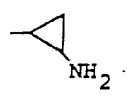 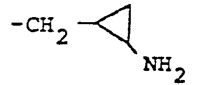 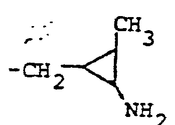 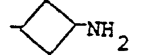 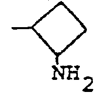 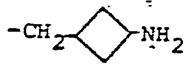 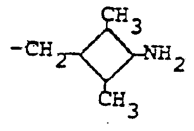 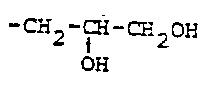 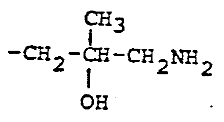 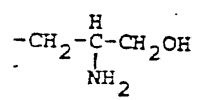 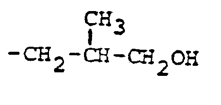 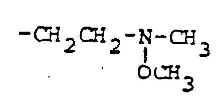 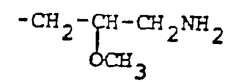 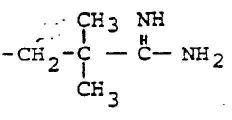 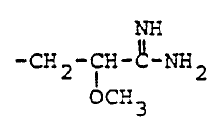
-CH₂-CH=CH-CH₂NH₂

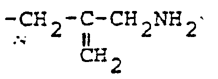

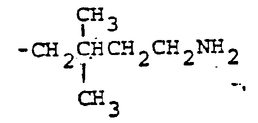

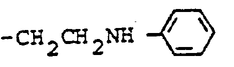

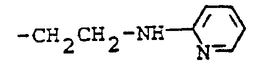

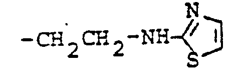

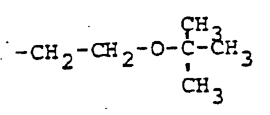

17. A process according to Claim 8, subparagraph 2.); wherein R⁸ is selected from:
-(^{CH}₂)ₙ-N⁼A n= 1-6 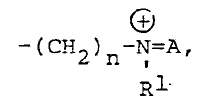
R¹=CH₃, CH₂CH₃, C(CH₃)₃, (CH₂)₂CH₃, 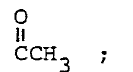
n= 1-6 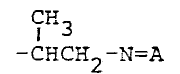 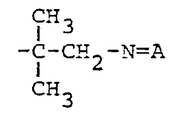 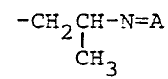 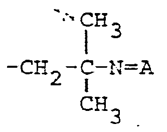 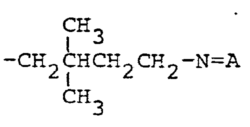 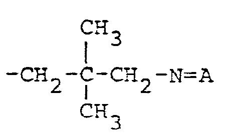 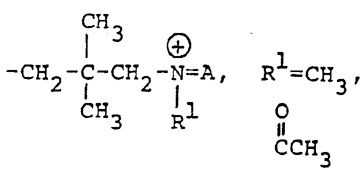 CH₂CH₃, C(CH₃)₃, (CH₂)₂CH₃, 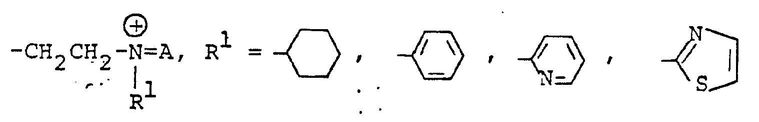 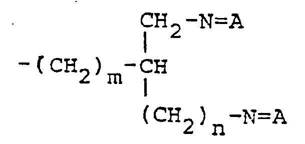
, n=0, 1, 2; m=0, 1, 2 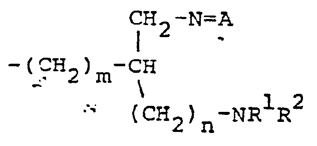
, n=0, 1, 2; m=0, 1, 2 R¹ and R² = _{H},H; H,CH₃ 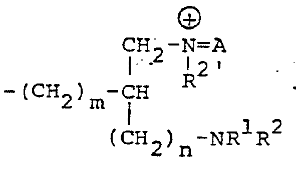
n=0, 1, 2; m=0, 1, 2 R¹ and R²=H,H; H, CH₃; R^{2'=CH}3 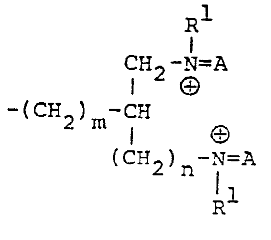
n=0, 1, 2; m=0, 1, 2 R¹=CH₃ 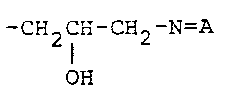 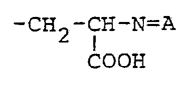 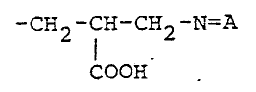 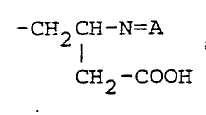 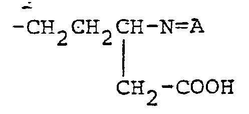
-CH=CH-N=A 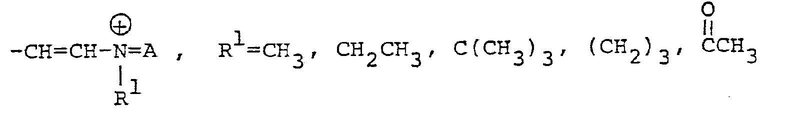 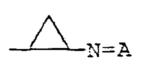 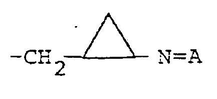 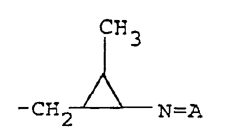 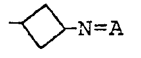 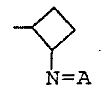 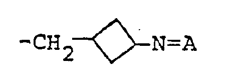 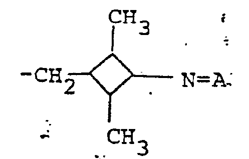 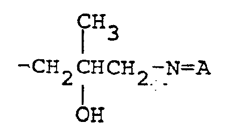 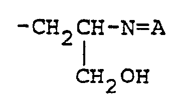 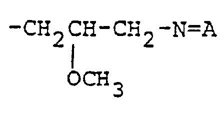
-CH₂-CH=CH-CH₂-N=A 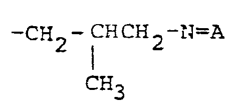 wherein A is selected from:

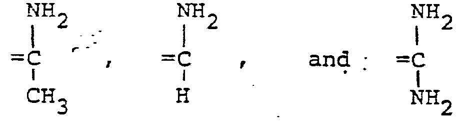

^{18.} A process according to Claim 8, subparagraph 3.); wherein R⁸ is selected from:

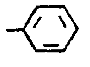

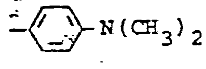

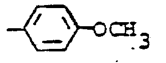

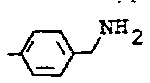

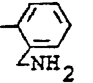

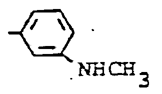

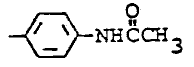

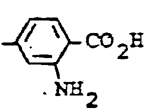

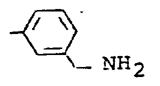

19. A process according to Claim 8, subparagraph 3.); wherein R⁸ is selected from: 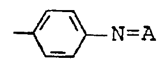 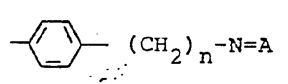
, n = 1-4 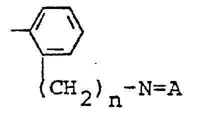
, n = 1-4 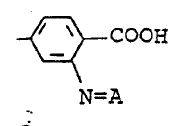 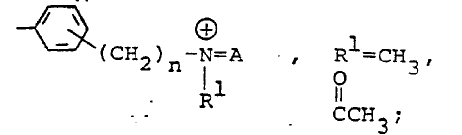 CH₂CH₃, CH₂CH₂CH₃;
n = 1-4 wherein A is selected from:

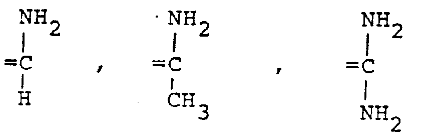

²0. A process according to Claim 8, subparagraph 4.), wherein R⁸ is : 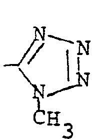 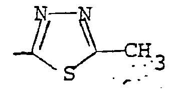 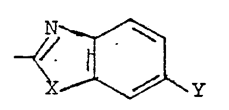
X=N,O Y=H
X=_{S} Y=H, Cl, OCH₂^{CH}₃ 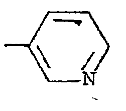 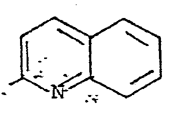 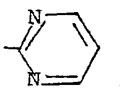 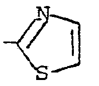 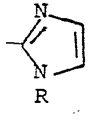
R=H, ^{CH}₃ 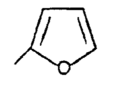 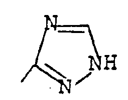 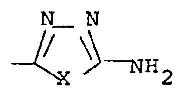
X=NH, S 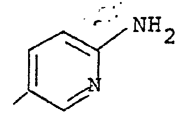 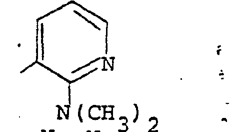 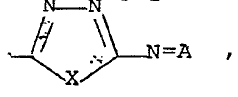
X=NH, S 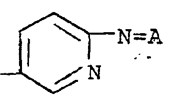
wherein A is selected from:

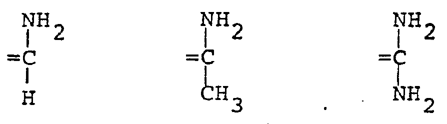

21. A process according to Claim 8, subparagraph 5.); wherein R⁸ is:. 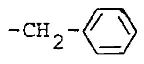 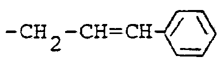 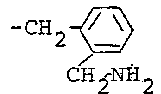 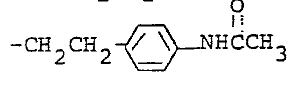 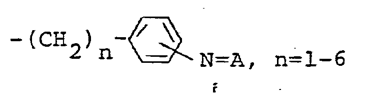 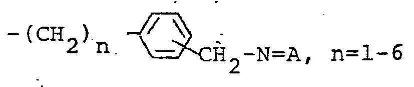 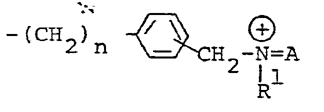 , R¹=CH₃, C^{H}₂^{CH}₃ CH₂CH₃CH₃, C(CH₃)₃, 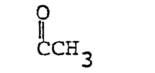 wherein A is selected from:

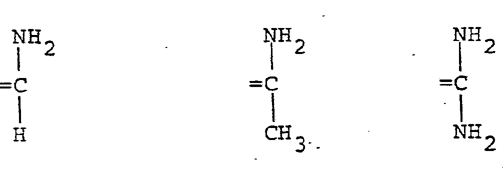

22. A process according to Claim 8, subparagraph 6.); wherein R⁸ is: 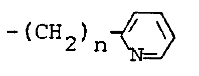 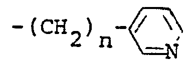 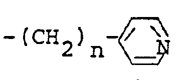 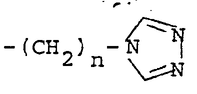 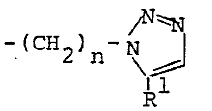
R¹ = OCH₂^{CH}₃ 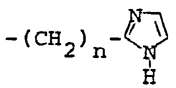 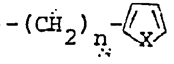 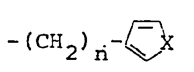
X = O, S, NH 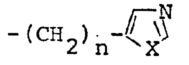
X = O, S, NH 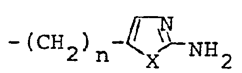
X = O, S, NH 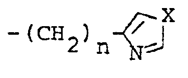
X = O , S, NH 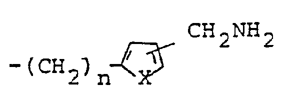
X = O, S, NH 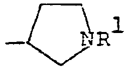
R¹=H, C^{H}3 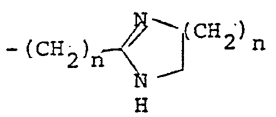
m = 1-3
n = 1-3 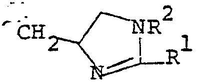
R²=H, CH₃, R¹=H, CH₃, ^{NH}₂ 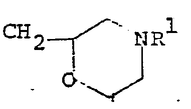
R¹= _{H}, C^{H}3 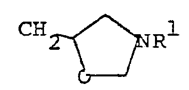
R¹=H, CH₃ 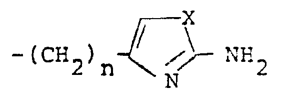
X = 0, S, NH 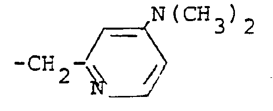 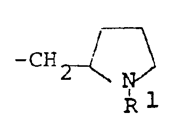
R¹ = H, ^{CH}₃ 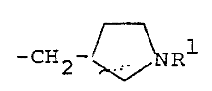
R¹ = _{H}, C^{H}3 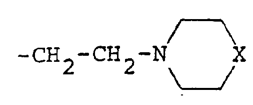
X = 0, NH, NC^{H}₃ 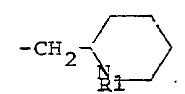
R¹ = H, C^{H}3 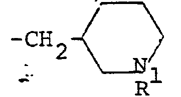
R¹ = _{H}, CH₃ 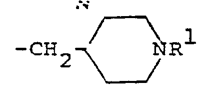
R¹ = _{H}, CH₃ 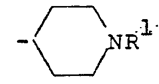
R¹ = H, ^{CH}₃ 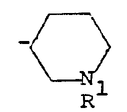
R¹ = H, ^{CH}₃ 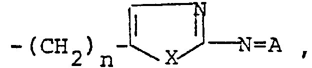
X = 0, S, NH 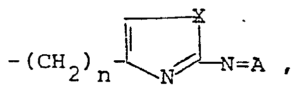
X = 0, S, NH wherein: n = 1,2,3; and A is selected from:

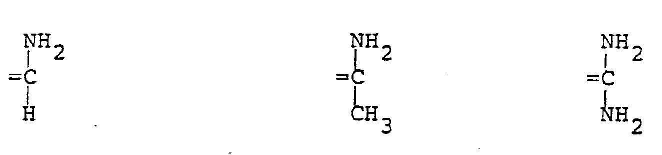

23. A process according to Claim 8, subparagraph 7.); wherein R⁸ is:

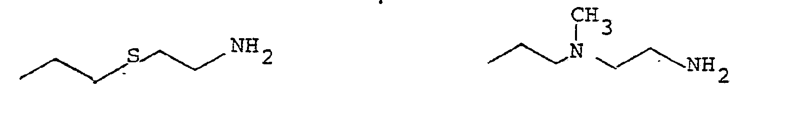

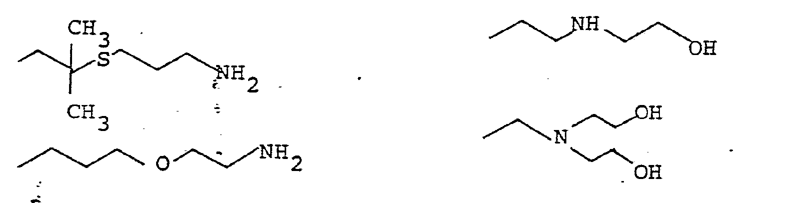

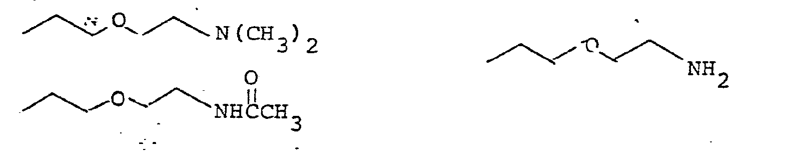

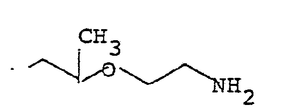

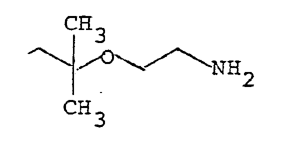

^{24.} A process according to Claim 8, subparagraph ^{7.})^{;} wherein R⁸ is:
CH₂CH₂SCH₂CH₂-N=A
CH₂C(CH₃)₂SCH₂CH₂-N=A
CH₂CH₂CH₂OCH₂CH₂-N=A 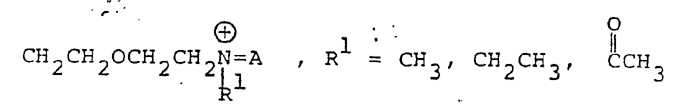
CH₂CH(CH₃)OCH₂CH₂N⁼A
CH₂C(CH₃)₂OCH₂CH₂N=A 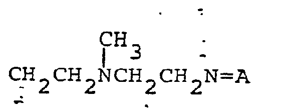 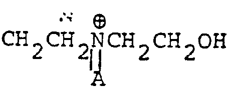
CH₂CH₂OCH₂CH₂N=A
wherein A is selected from:

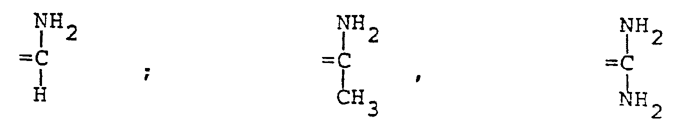

^{25.} A process according to Claims 15, 16, 17, 18, 19, 20, 21, 22, 23, or 24 wherein R⁷ is hydrogen and R⁶ is selected from the group consisting of CH3CH2, 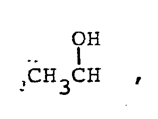 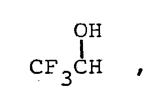 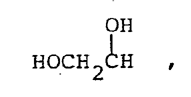 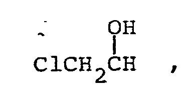
HOCH₂CH₂ ,
BrH2CCH(OH) , (CH₃)₃C-CH(OH) 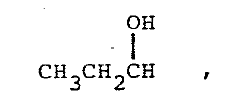 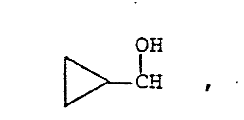 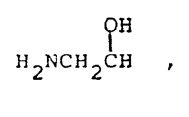 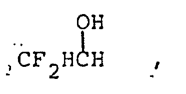
HOCH₂ ,
HO₂CCH₂
and

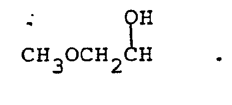

26. A process according to Claim 1, wherein R⁶ and R⁷ are independently selected from the group consisting of mercapto hydroxyl, chloro, bromo, fluoro, hydrogen; substituted and unsubstituted: alkyl, alkoxyl, alkylthio, alkenyl, and alkynyl having from 1-6 carbon atcms; cycloalkyl, cycloalkylalkyl, and alkylcycloalkyl having 3-6 carbon atoms in the cycloalkyl ring and 1-6 carbon atoms in the alkyl moiety; aryl, aralkyl, aralkenyl, and aralkynyl wherein the aryl moiety is phenyl and the aliphatic moiety-has 1-6 carbon atoms; heteroaryl, heteroaralkyl, heterocyclyl and heterocyclylalkyl wherein the alkyl moiety has 1-6 carbon atoms and wherein the heterocyclic structure comprises 4-6 atoms, the hetero atom or atoms being selected from the group consisting of 1-4 oxygen, nitrogen, or sulfur atoms; wherein the substituent or substituents on R⁶ and R⁷ are selected from the group consisting of: amino, mono- di- and trialkylamino, hydroxyl, alkoxyl, mercapto, alkylthio, chloro, bromo, fluoro, carboxy, oximino, ureido, alkoximino, and N-substituted ureido wherein the N-substituent or N-substituents are selected from alkyl, phenyl and phenylalkyl, and wherein the alkyl moieties of the aforementioned substituents have 1-6 carbon atoms; R is selected from the group consisting of: hydrogen; substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moiety; aryl, aralkyl, aralkenyl, and aralkynyl wherein the aryl moiety is phenyl and the aliphatic moiety has 1-6 carbon atoms; heteroaryl, heteroaralkyl, heterocyclyl and heterocyclylalkyl having 4-6 ring atoms; wherein the substituent or substituents relative to'R⁸ are selected from the group consisting of: amino, mono-, di- and trialkylamino, hydroxyl, alkoxyl, mercapto, alkylthio, phenylthio, sulfamoyl, amidino, guanidino, nitro, chloro, bromo, fluoro, cyano and carboxy; and wherein the hetero atom or atoms in the above-nampd heterocyclic moieties are selected from the group consisting of 1-4 oxygen, nitrogen or sulphur atoms; and wherein the alkyl moieties of the above-recited substituents have 1-6 carbon atoms.

27. A process according to Claim 26 wherein R⁸ is selected from the group consisting of:

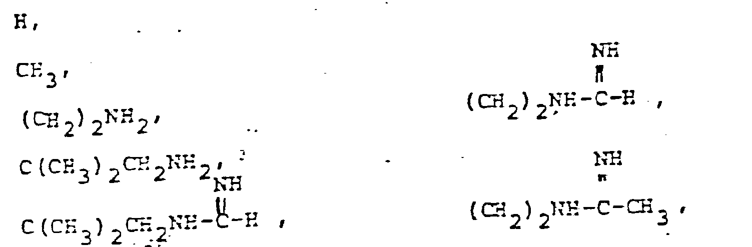

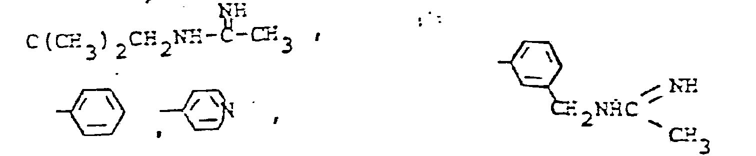

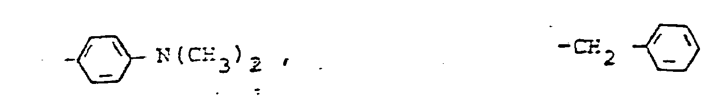

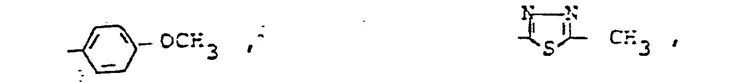

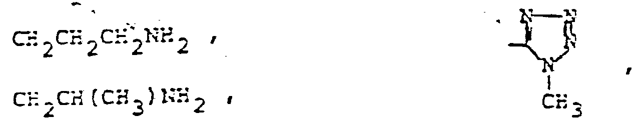

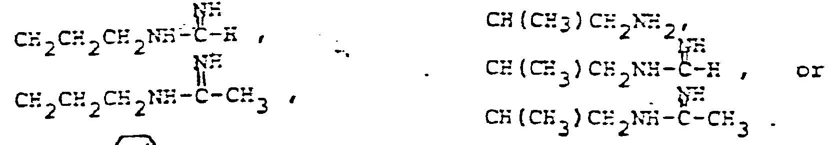

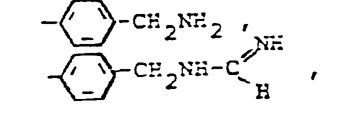

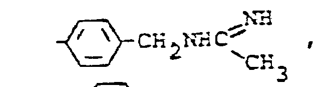

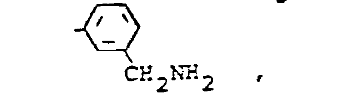

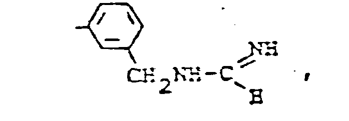

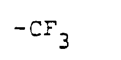

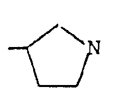

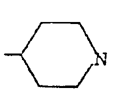

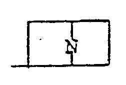

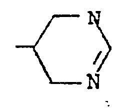

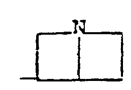

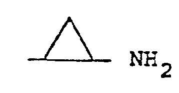

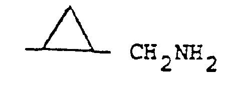

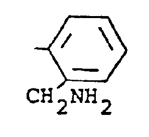

28. A process according to Claim ₂₇ wherein: R⁷ is hydrogen, hydroxyl, alkoxyl, alkylthio, mercapto, chloro, fluoro, bromo, alkyl and substituted alkyl. wherein the substituent or substituents are: alkoxyl or hydroxyl.

29. A process according to Claim ₂₈ wherein R is hydrogen, hydroxyl, OCH₃, CH₃, hydroxyl- and polyhydroxyl-substituted alkyl.

3₀. A process according to Claim₂₉ wherein- _{R}7 is hydrogen, CH₃ or OCH₃.

31. A process according to Claim 30 wherein R⁶ is: hydrogen; substituted and unsubstituted: alkyl, cycloalkyl, cycloalkylalkyl, phenylalkyl; wherein the substituent or substituents are selected from: hydroxyl, chloro, fluoro, bromo, carboxyl, oximino, alkoximino, ureido, amino, alkoxyl, or alkylthio.

32. A process according to Claim₃₁ wherein the substituent or substituents on R⁶ are hydroxyl.

33.. A process according to Claim 26 wherein R⁷ is hydrogen, hydroxyl, alkoxyl, alkylthio, chloro, fluoro, bromo, alkyl and substituted alkyl wherein the substituent or substituents are: alkoxyl, or hydroxyl;and R⁶ is: hydrogen; substituted and unsubstituted: alkyl, cycloalkyl, cycloalkylalkyl, phenylalkyl; wherein the substituent or substituents are selected from: hydroxyl, chloro, fluoro, bromo, oximino, alkoximino, ureido, amino, alkoxyl or alkylthio.

34 . A process according to Claim 27 wherein R⁷ is hydrogen, hydroxyl, alkoxyl, alkylthio, mercapto, chloro, fluoro, bromo, alkyl and substituted alkyl wherein the substituent or substituents are: alkoxyl, or hydroxyl; and R⁶ is hydrogen; substituted and unsubstituted: alkyl, cycloalkyl, cycloalkylalkyl, phenylalkyl; wherein the substituent or substituents are selected from: hydroxyl, chloro, fluoro, bromo, oximino, alkoximino, ureido, amino, alkcxyl or alkylthio.

35- A process according to Claim 34 wherein _{R}⁷ is hydrogen, CH₃ or OCH₃.

36. A process according to Claim 35 wherein R⁶ is selected from: H,' 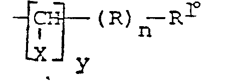
wherein: y = 0 or 1;
X = OH, NH₂ ; SH;
n = 0 or 1;
R = substituted or
unsubstituted: alkyl, alkenyl or alkynyl having 1-6 carbon atoms wherein the substituent is _{R}¹_{;} R^{1°} = alkoxyl, carboxyl, CF_{3'} OH, H, linear or branched alkyl bearing 1 or more hydroxyl groups, amino, aminoalkyl, Cl, F, Br, alkylthio, amidino, guanidino, oximino, phenyloxy, phenylthio,

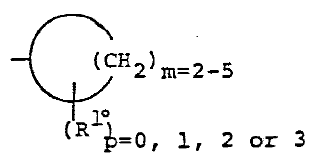

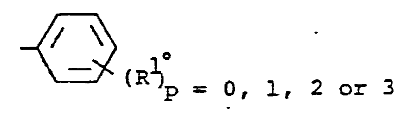

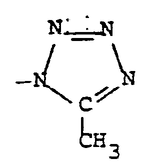

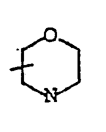

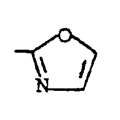

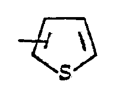

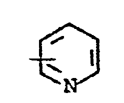

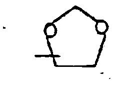

37. A process according to Claim 35 wherein R⁶ is selected from:

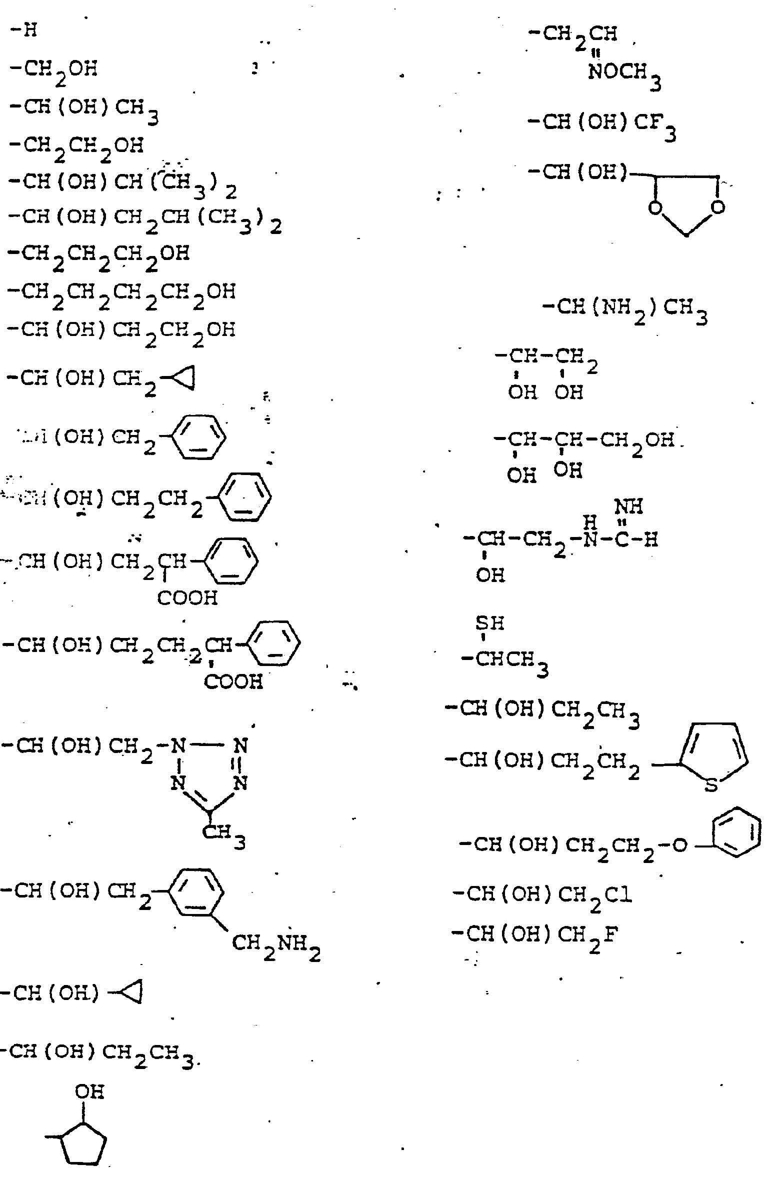

38 - A process according to Claim 37 wherein R⁸ is: -CH₂CH₂NH₂

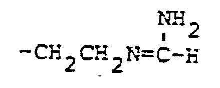

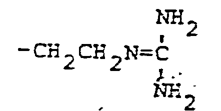

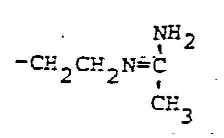

39. A process according to Claim 1 wherein R⁷ is hydrogen and _{R}⁶ is
